# EUROPÄISCHE PATENTSCHRIFT

(11) **EP 3 475 273 B1**
(45) Veröffentlichungstag und Bekanntmachung des Hinweises auf die Patenterteilung: **24.02.2021**
(21) Anmeldenummer: 17732071.0
(22) Anmeldetag: 19.06.2017
(51) Int. Cl.: C07D 405/12, C07D 249/14, A01N 43/707

(54) **3-AMINO-1,2,4-TRIAZINDERIVATE UND DEREN VERWENDUNG ZUR BEKÄMPFUNG UNERWÜNSCHTEN PFLANZENWACHSTUMS**
3-AMINO PYRIMIDINE DERIVATIVES AND USE THEREOF FOR COMBATING UNDESIRED PLANT GROWTH
DERIVES DE 3-AMINO-1, 2, 4 TRIAZINE ET LEUR UTILISATION POUR LUTTER CONTRE LA CROISSANCE INDESIRABLE DE PLANTES

(30) Priorität: 24.06.2016 EP 16176110
(43) Veröffentlichungstag der Anmeldung: 01.05.2019
(73) Patentinhaber: Bayer CropScience Aktiengesellschaft, 40789 Monheim am Rhein (DE)
(72) Erfinder: JAKOBI, Harald, 60598 Frankfurt (DE); MINN, Klemens, 65795 Hattersheim (DE); BUSCATÓ ARSEQUELL, Estella, 60486 Frankfurt am Main (DE); DIETRICH, Hansjörg, 65835 Liederbach (DE); GATZWEILER, Elmar, 61231 Bad Nauheim (DE); ROSINGER, Christopher Hugh, 65719 Hofheim (DE); MACHETTIRA, Anu Bheemaiah, 60326 Frankfurt am Main (DE)
(74) Vertreter: BIP Patents
(86) Internationale Anmeldenummer: PCT/EP2017/064889
(87) Internationale Veröffentlichungsnummer: WO 2017/220467

(56) Entgegenhaltungen:
- WO-A1-2004/056829
- WO-A1-2004/069814
- WO-A1-2013/144187
- ADAM F M ET AL: "Novel syntheses of the amino-1,2,4-triazine GW356194: identification of a synthesis amenable to scale up", TETRAHEDRON LETTERS, ELSEVIER LTD, AMSTERDAM, NL, vol. 44, no. 30, 21 July 2003 (2003-07-21) , pages 5657-5659, XP004433820, ISSN: 0040-4039, DOI: 10.1016/S0040-4039(03)01359-5
- Cherng-Chyi Tzeng ET AL: "Synthesis of Certain 5,6-Diamino-as -triazines: Precursors for Fused Heterocyclic Systems", J. Org. Chem., vol. 48, 1 January 1983 (1983-01-01), pages 1271-1275, XP055660245, DOI: org/10.1021/jo00156a025
- MARCH L C ET AL: "Antimalarials 3 1,2,4-Triazines", JOURNAL OF MEDICINAL CHEMISTRY, AMERICAN CHEMICAL SOCIETY, US, vol. 19, no. 6, 1 January 1976 (1976-01-01), pages 845-848, XP002470485, ISSN: 0022-2623, DOI: 10.1021/JM00228A024

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen sowie im Ziergartenbereich und zur generellen Bekämpfung von Unkräutern und Ungräsern in Umweltbereichen, in denen Pflanzenwuchs störend ist.

Insbesondere betrifft die Erfindung substituierte 3-Amino-1,2,4-Triazinderivate, Verfahren zu deren Herstellung sowie deren Verwendung zur Bekämpfung von Schadpflanzen.

Die erfindungsgemäßen Verbindungen der Formel (I) weisen in der 3-Positon des 1,2,4-Triazins einen in der alpha Position zum Aromaten über ein Amin gebundenen teilhydrierten bicyclischen Substituienten auf, wobei das Triazin in der 5-Position und 6-Position ebenfalls substituiert sein kann und der Aminosubstituent R¹ zusammen mit dem Substituenten in der benachbarten Position (R²) einen Ring bilden kann.

Aus dem Stand der Technik ist die herbizide Wirkung derartiger 1,2,4-Triazine nicht bekannt.

Die Anwendung der bekannten selektiven Herbizide zur Schadpflanzenbekämpfung oder als Pflanzenwachstumsregulatoren in verschiedenen Nutzpflanzenkulturen erfordert häufig eine hohe Kosten verursachende Aufwandmenge oder führt zu unerwünschten Schädigungen der Nutzpflanzen. Zudem ist die Anwendung der Wirkstoffe in vielen Fällen wegen verhältnismäßig hoher Herstellkosten unwirtschaftlich.

Es ist deshalb erstrebenswert, alternative chemische Wirkstoffe bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Die Aufgabe der vorliegenden Erfindung besteht in der Bereitstellung alternativer Wirkstoffe, welche als Herbizide oder Pflanzenwachstumsregulatoren, mit einer zufriedenstellenden herbiziden Wirkung und einem breiten Wirkspektrum gegenüber Schadpflanzen und/oder mit einer hohen Selektivität in Nutzpflanzenkulturen, eingesetzt werden können.

Gelöst wird die Aufgabe durch speziell substituierte 3-Amino-1,2,4-Triazinderivate der Formel (I) gemäß Anspruch 1, welche vorteilhaft als Herbizide und auch als Pflanzenwachstumsregulatoren eingesetzt werden können.

Gegenstand der vorliegenden Erfindung sind daher Verbindungen der Formel (I) 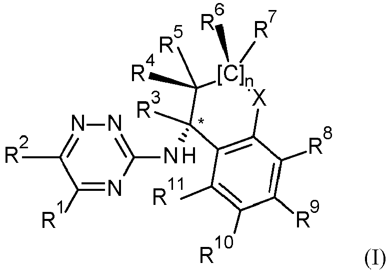 und deren agrochemisch verträglichen Salze, in welchen
- R¹: Wasserstoff, Amino, Hydrazino, Hydroxyamino, ((C₁-C₆)-alkyl)-amino, ((C₃-C₆)-cycloalkyl)-amino, Di-((C₁-C₆)-alkyl)-amino, (C₁-C₆)-Alkylcarbonylamino und Bis-((C₁-C₆)-alkyl)-carbonylamino bedeutet;
- R²: ausgewählt ist aus der Gruppe, bestehend aus Wasserstoff, Halogen, Hydroxy, Nitro, Amino, Cyano, C(O)OH, C(O)NH₂; (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl; (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl; (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)- Haloalkenyloxycarbonyl; (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl; Tri-(C₁-C₆)-alkylsilyl-(C₂-C₆)-alkinyl, Di-(C₁-C₆)-alkylsilyl-(C₂-C₆)-alkinyl, (C₁-C₆)-alkylsilyl-(C₂-C₆)-alkinyl; Phenylsilyl-(C₂-C₆)-alkinyl; (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können; (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy; Aminocarbonyl-(C₁-C₆)-alkyl, Di-(C₁-C₆)-Alkylaminocarbonyl-(C₁-C₆)-alkyl; N-((C₁-C₆)-Haloalkanoyl)-amino-carbonyl, ((C₆-C₁₄)-aryl)amino-carbonyl, Di-((C₆-C₁₄)-aryl)-amino-carbonyl, ((C₁-C₆)-alkyl)amino-carbonyl, Di-((C₁-C₆)-alkyl)-amino-carbonyl, N-((C₁-C₆)-Alkylsulfonyl)-amino-carbonyl, N-((C₁-C₆)-Haloalkylsulfonyl)-amino-carbonyl, N-((C₆-C₁₄)-Arylsulfonyl)-amino-carbonyl; (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy; (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy; (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyl-oxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy; Hydroxy-(C₁-C₆)-alkyl, Hydroxy-(C₁-C₆)-alkoxy, Cyano-(C₁-C₆)-alkoxy, Cyano-(C₁-C₆)-alkyl; und (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio, oder
- R¹ mit R²: über eine Bindung miteinander verbunden sein kann, so dass sich ein 5-bis 7-gliedriger teilhydrierter Carbocyclus oder Heterocyclus mit mindestens einem Heteroatom ausgewählt aus N, O, S und P ergibt, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert ist;
- R³: ausgewählt ist aus der Gruppe bestehend aus Wasserstoff, (C₁-C₆)-Alkyl, (C₁-C₆)-Alkoxy, (C₁-C₆)-Alkoxy-(C₁-C₆)-Alkyl, (C₁-C₆)-Alkoxycarbonyl und Aminocarbonyl;
- R⁴ und R⁵: jeweils unabhängig voneinander, ausgewählt sind aus der Gruppe, bestehend aus Wasserstoff, (C₁-C₆)-Alkyl, (C₁-C₆)-Haloalkyl, Hydroxy, (C₁-C₆)-Alkoxy und (C₁-C₆)-Halogenalkoxy; oder
- R⁴ und R⁵: zusammen mit dem Kohlenstoffatom, an welchem sie gebunden sind, einen gesättigten drei- bis siebengliedrigen Ring bilden, der ein oder mehrere Heteroatome ausgewählt aus Sauerstoff, Schwefel und Stickstoff enthalten kann;
- R⁶ und R⁷: jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁶ und R⁷ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
- n: die Laufzahl 0, 1 oder 2 ist;
- R⁸, R⁹, R¹⁰ und R¹¹: jeweils unabhängig voneinander, ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro, wobei die Reste R⁹ und R¹⁰ ringbildend durch eine -O-CH₂-O- Gruppe verbunden sein können;
- X: für eine Bindung, CH₂, O, S, Carbonyl, NH, CR¹²R¹³,NR¹⁴,CH₂O oder CH₂S steht, wobei bei den beiden letztgenannten Gruppen das Kohlenstoffatom an den aromatischen Teil und das Heteroatom O oder S an den teilhydrierten Teil des Amins gebunden ist;
- R¹² und R¹³: jeweils unabhängig voneinander, ausgewählt sind aus der Gruppe, bestehend aus Wasserstoff, (C₁-C₆)-Alkyl und (C₁-C₆)-Haloalkyl; und
- R¹⁴: ausgewählt ist aus der Gruppe, bestehend aus Wasserstoff, (C₁-C₆)-Alkyl und (C₁-C₆)-Haloalkyl.

Die Aminogruppe in der 3-Positon des 1,2,4-Triazins weist bei den erfindungsgemäßen Bis(amino)-triazinen der Formel (I) einen teilhydrierten bicyclischen Substituenten auf, wobei der teilhydrierte bicyclische Substituent in der alpha Position zum Aromaten an das Amin gebunden ist.

Die Verbindungen der Formel (I) zeichnen sich, neben einem guten Wirksamkeitsprofil und einer guten Kulturpflanzenverträglichkeit, durch ihre kostengünstige Herstellung aus, da die erfindungsgemäßen Substanzen aus preiswerten und gut zugänglichen Vorstufen durch kostengünstige Prozesse hergestellt werden können. Daher kann auf die Verwendung teurer und schwer zugänglicher Zwischenprodukte verzichtet werden.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf. Das gilt auch für die Laufzahl n, d.h. die Laufzahl n ist in den nachfolgenden Ausführungsformen 0, 1 oder 2.

Eine erste Ausführungsform der vorliegenden Erfindung umfasst Verbindungen der allgemeinen Formel (I), in welchen
- R¹: bevorzugt ausgewählt ist aus der Gruppe bestehend aus Wasserstoff, Amino, ((C₁-C₆)-alkyl)amino, ((C₃-C₆)-cycloalkyl)amino, und Di-((C₁-C₆)-alkyl)amino;
- R¹: besonders bevorzugt ausgewählt ist aus der Gruppe bestehend aus Wasserstoff, Amino, ((C₁-C₃)-alkyl)amino, ((C₃-C₆)-cycloalkyl)amino, und Di((C₁-C₃)-alkyl)amino;
- R¹: ganz besonders bevorzugt ausgewählt ist aus der Gruppe bestehend aus Wasserstoff, Amino, NH(CH₃), N(H)Cyclopropyl und N(CH₃)₂; und in welchem
- R¹: am meisten bevorzugt Wasserstoff und Amino ist, und ganz besonders bevorzugt Amino.

Eine zweite Ausführungsform der vorliegenden Erfindung umfasst Verbindungen der allgemeinen Formel (I), in welchen
- R²: bevorzugt ausgewählt ist aus der Gruppe bestehend aus Wasserstoff, Halogen, Hydroxy, Nitro, Amino, Cyano, C(O)OH, C(O)NH₂; (C₁-C₆)-Alkyl, (C₁-C₆)-Halogenalkyl; (C₁-C₆)-Alkylcarbonyl, (C₁-C₆)-Haloalkylcarbonyl, (C₁-C₆)-Alkoxycarbonyl, (C₁-C₆)-Haloalkoxycarbonyl; (C₂-C₆)-Alkenyl, (C₂-C₆)-Halogenalkenyl; (C₂-C₆)-Alkinyl, (C₂-C₆)-Halogenalkinyl; (C₆-C₁₄)-Aryl, welches am Arylteil mit Halogen, (C₁-C₆)-Alkyl und/oder (C₁-C₆)-Haloalkyl substituiert sein kann; (C₁-C₆)-Alkoxy-(C₁-C₆)-Alkyl, Hydroxy-(C₁-C₆)-alkyl; ((C₁-C₆)-Alkyl)amino-carbonyl, Di-((C₁-C₆)-alkyl)aminocarbonyl, N-((C₁-C₆)-Alkylsulfonyl)amino-carbonyl, N-((C₁-C₆)-Haloalkylsulfonyl)amino-carbonyl, (C₃-C₈)-Cycloalkylcarbonyl, (C₃-C₈)-Cycloalkoxycarbonyl; (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylsulfinyl, (C₄-C₁₄)-Arylsulfonyl, und (C₆-C₁₄)-Arylsulfinyl; (C₁-C₆)-Alkoxy-(C₁-C₆)-alkyl,
- R²: mehr bevorzugt ausgewählt ist aus der Gruppe bestehend aus Halogen, Cyano, C(O)OH, (C₁-C₆)-Alkyl, (C₁-C₆)-Halogenalkyl; (C₁-C₆)-Alkylcarbonyl, (C₁-C₆)-Haloalkylcarbonyl, (C₁-C₆)-Alkoxycarbonyl, (C₁-C₆)-Haloalkoxycarbonyl; (C₂-C₆)-Alkenyl, (C₂-C₆)-Halogenalkenyl; (C₂-C₆)-Alkinyl, (C₂-C₆)-Halogenalkinyl; Phenyl, welches am Arylteil mit Halogen, (C₁-C₆)-Alkyl und/oder (C₁-C₆)-Haloalkyl substituiert sein kann; (C₁-C₃)-Alkoxy-(C₁-C₆)-Alkyl, Hydroxy-(C₁-C₆)-alkyl; ((C₁-C₃)-alkyl)amino-carbonyl, Di-((C₁-C₃)-Alkyl)aminocarbonyl; N-((C₁-C₃)-Alkylsulfonyl)amino-carbonyl, N-((C₁-C₃)-Haloalkylsulfonyl)amino-carbonyl, (C₃-C₆)-Cycloalkylcarbonyl, (C₃-C₆)-Cycloalkoxycarbonyl; (C₁-C₃)-Alkylsulfonyl, (C₁-C₃)-Alkylsulfinyl, (C₁-C₃)-Haloalkylsulfonyl, (C₁-C₆)-Halogenalkylsulfinyl, (C₄-C₁₄)-Arylsulfonyl, und (C₆-C₁₄)-Arylsulfinyl;
- R²: besonders bevorzugt ausgewählt ist aus der Gruppe, bestehend aus Cl, Br, Cyano, C(O)OH, C(O)NH₂; (C₁-C₆)-Alkyl, (C₁-C₆)-Halogenalkyl, (C₁-C₆)-Alkylcarbonyl, (C₁-C₆)-Haloalkylcarbonyl; (C₁-C₆)-Alkoxycarbonyl, (C₁-C₆)-Haloalkoxycarbonyl; (C₂-C₆)-Alkenyl, (C₂-C₆)-Halogenalkenyl: (C₂-C₆)-Alkinyl, (C₂-C₆)-Halogenalkinyl; Phenyl, welches mit F, Cl, Br, Methyl und/oder Trifluormethyl substituiert sein kann; (C₁-C₃)-Alkoxy-(C₁-C₃)-Alkyl, Hydroxy-(C₁-C₃)-alkyl; ((C₁-C₂)-alkyl)amino-carbonyl, Di-((C₁-C₂)-alkyl)amino-carbonyl, N-((C₁-C₂)-Alkylsulfonyl)amino-carbonyl, N-((C₁-C₂)-Haloalkylsulfonyl)amino-carbonyl; (C₃-C₆)-Cycloalkylcarbonyl, (C₃-C₆)-Cycloalkoxycarbonyl; (C₁-C₃)-Alkylsulfonyl, (C₁-C₃)-Alkylsulfinyl, (C₁-C₃)-Haloalkylsulfonyl, (C₁-C₆)-Halogenalkylsulfinyl, (C₄-C₄)-Arylsulfonyl, (C₆-C₁₄)-Arylsulfinyl;
- R²: ganz besonders bevorzugt ausgewählt ist aus der Gruppe, bestehend aus Cl, Cyano, C(O)NH₂; (C₁-C₃)-Halogenalkyl, (C₁-C₃)-Alkylcarbonyl, (C₁-C₃)-Haloalkylcarbonyl; (C₁-C₆)-Alkoxycarbonyl, (C₁-C₆)-Haloalkoxycarbonyl; (C₂-C₆)-Alkenyl, (C₂-C₆)-Halogenalkenyl; Methoxy-(C₁-C₃)-Alkyl, Hydroxy-(C₁-C₃)-alkyl; ((C₁-C₂)-alkyl)amino-carbonyl, Di-((C₁-C₂)-alkyl)amino-carbonyl (C₁-C₃)-Alkylsulfonyl, (C₁-C₃)-Alkylsulfinyl; und in welchem
- R²: am meisten bevorzugt Cl, Cyano, C(O)NH₂, Trifluormethyl, Acetyl (C(O)CH₃), Trifluoracetyl (C(O)CF₃), Methoxy-(C₂-C₃)-Alkyl, Hydroxy-(C₂-C₃)-alkyl, (C₁-C₂)-Alkoxycarbonyl, (C₂-C₃)-Alkenyl, (C₂-C₃)-Halogenalkenyl, Methylamino-carbonyl, Dimethylamino-carbonyl, Methylsulfonyl, und Methylsulfinyl ist.

Eine dritte Ausführungsform der vorliegenden Erfindung umfasst Verbindungen der allgemeinen Formel (I), in welchen
- R³: bevorzugt Wasserstoff ist.

Eine vierte Ausführungsform der vorliegenden Erfindung umfasst Verbindungen der allgemeinen Formel (I), in welchen
- R⁴ und R⁵: jeweils unabhängig voneinander, bevorzugt ausgewählt sind aus der Gruppe, bestehend aus Wasserstoff, Hydroxy, (C₁-C₆)-Alkyl, (C₁-C₆)-Alkylphenyl und (C₁-C₆)-Alkoxy;
- R⁴ und R⁵: jeweils unabhängig voneinander, besonders bevorzugt ausgewählt sind aus der Gruppe, bestehend aus Wasserstoff, (C₁-C₆)-Alkyl und (C₁-C₆)-Alkoxy;
- R⁴ und R⁵: jeweils unabhängig voneinander, ganz besonders bevorzugt ausgewählt sind aus der Gruppe, bestehend aus Wasserstoff, (C₁-C₃)-Alkyl, (C₁-C₆)-Alkoxy; und in welchen
- R⁴ und R⁵: am meisten bevorzugt jeweils unabhängig voneinander, Wasserstoff oder Methyl sind.

In dieser vierten Ausführungsform ist speziell bevorzugt, wenn mindestens einer der Reste R⁴ bzw. R⁵ Wasserstoff ist. D.h. wenn jeweils mindestens einer der Reste R⁴, bzw. R⁵ Wasserstoff ist und der andere Rest R⁴, bzw. R⁵ ungleich Wasserstoff, insbesondere (C₁-C₆)-Alkyl, bevorzugt Methyl (CH₃), ist.

Eine fünfte Ausführungsform der vorliegenden Erfindung umfasst Verbindungen der allgemeinen Formel (I), in welchen
- R⁴ und R⁵: zusammen mit dem Kohlenstoffatom, an welchem sie gebunden sind, einen gesättigten drei- bis sechsgliedrigen Ring bilden, der ein Heteroatom ausgewählt aus Sauerstoff, Schwefel und Stickstoff enthalten kann;
- R⁴ und R⁵: bevorzugt zusammen mit dem Kohlenstoffatom, an welchem sie gebunden sind, einen gesättigten drei- bis sechsgliedrigen Ring bilden, der ein Sauerstoffatom enthalten kann;
- R⁴ und R⁵: besonders bevorzugt zusammen mit dem Kohlenstoffatom, an welchem sie gebunden sind, einen gesättigten drei- oder sechsgliedrigen Ring bilden, der ein Sauerstoffatom enthalten kann; und in welchem
- R⁴ und R⁵: am meisten bevorzugt zusammen mit dem Kohlenstoffatom, an welchem sie gebunden sind, einen gesättigten dreigliedrigen Ring bilden, der ein Sauerstoffatom enthalten kann oder einen 4-Pyranring bilden.

Eine sechste Ausführungsform der vorliegenden Erfindung umfasst Verbindungen der allgemeinen Formel (I), in welchen
- R⁶ und R⁷: unabhängig voneinander, bevorzugt ausgewählt sind aus der Gruppe, bestehend aus Wasserstoff, (C₁-C₆)-Alkyl und (C₆-C₁₄)-Aryl;
- R⁶ und R⁷: unabhängig voneinander, besonders bevorzugt ausgewählt sind aus der Gruppe, bestehend aus Wasserstoff, Methyl und Phenyl;
- R⁶ und R⁷: ganz besonders bevorzugt jeweils unabhängig voneinander Wasserstoff oder Methyl sind; und in welchen
- R⁶ und R⁷: am meisten bevorzugt Wasserstoff bedeuten.

Eine siebte Ausführungsform der vorliegenden Erfindung umfasst Verbindungen der allgemeinen Formel (I), in welchen
- R⁸: bevorzugt ausgewählt ist aus der Gruppe, bestehend aus Wasserstoff, Halogen, (C₁-C₆)-Alkyl, (C₁-C₆)Alkoxy, und (C₆-C₁₄)-Aryl;
- R⁸: besonders bevorzugt ausgewählt ist aus der Gruppe, bestehend aus Wasserstoff, Halogen, (C₁-C₃)-Alkyl und (C₆-C₈)-Aryl;
- R⁸: ganz besonders bevorzugt ausgewählt ist aus der Gruppe, bestehend aus Wasserstoff, Fluor, Chlor, CH₃, und Phenyl; und in welchen
- R⁸: am meisten bevorzugt Wasserstoff oder CH₃ ist.

Eine achte Ausführungsform der vorliegenden Erfindung umfasst Verbindungen der allgemeinen Formel (I), in welchen
- R⁹: bevorzugt ausgewählt ist aus der Gruppe, bestehend aus Wasserstoff, Halogen, (C₁-C₆)-Alkyl und (C₁-C₆)-Alkoxy;
- R⁹: besonders bevorzugt ausgewählt ist aus der Gruppe, bestehend aus Wasserstoff, Chlor, Fluor und (C₁-C₃)-Alkoxy;
- R⁹: ganz besonders bevorzugt ausgewählt ist aus der Gruppe, bestehend aus Wasserstoff, Fluor, Chlor und Methoxy; und in welchen
- R⁹: am meisten bevorzugt Wasserstoff ist.

Eine neunte Ausführungsform der vorliegenden Erfindung umfasst Verbindungen der allgemeinen Formel (I), in welchen
- R¹⁰: bevorzugt ausgewählt ist aus der Gruppe, bestehend aus Wasserstoff, Halogen, Cyano, Aminocarbonyl, Hydroxycarbonyl, (C₁-C₆)-Alkyl, (C₁-C₆)-Haloalkyl, (C₁-C₆)-Alkoxy, (C₂-C₆)-Alkenyl, (C₂-C₆)-Alkinyl, (C₁-C₆)-Alkyl-(C₂-C₆)-alkinyl, Hydroxy-(C₁-C₆)-alkyl-(C₂-C₆)-alkinyl, (C₁-C₆)-Alkoxy-(C₂-C₆)-alkinyl und Aryl-(C₂-C₆)-alkinyl;
- R¹⁰: besonders bevorzugt ausgewählt ist aus der Gruppe, bestehend aus Wasserstoff, Fluor, Chlor, Brom, Iod, Cyano, Methoxy (OCH₃), (C₁-C₃)-Alkyl, (C₁-C₃)-Haloalkyl, (C₁-C₃)-Alkoxy, (C₂-C₄)-Alkenyl, (C₂-C₄)-Alkinyl, (C₁-C₃)-Alkyl-(C₂-C₄)-alkinyl, Hydroxy-(C₁-C₃)-alkyl-(C₂-C₄)-alkinyl, (C₁-C₃)-Alkoxy-(C₂-C₄)-alkinyl und Phenyl-(C₂-C₄)-alkinyl;
- R¹⁰: ganz besonders bevorzugt ausgewählt ist aus der Gruppe, bestehend aus Wasserstoff, Fluor, Chlor, Brom, Iod, Cyano, Methyl, C≡CH, und C≡CCH3; und in welchen
- R¹⁰: am meisten bevorzugt Wasserstoff oder CH₃ ist.

Eine zehnte Ausführungsform der vorliegenden Erfindung umfasst Verbindungen der allgemeinen Formel (I), in welchen
- R¹¹: bevorzugt ausgewählt ist aus der Gruppe, bestehend aus Wasserstoff und (C₁-C₆)-Alkyl;
- R¹¹: besonders bevorzugt ausgewählt ist aus der Gruppe, bestehend aus Wasserstoff und (C₁-C₄)-Alkyl;
- R¹¹: ganz besonders bevorzugt Wasserstoff oder Methyl ist; und in welchen
- R¹¹: am meisten bevorzugt Wasserstoff ist.

Eine elfte Ausführungsform der vorliegenden Erfindung umfasst Verbindungen der allgemeinen Formel (I), in welchen
- X: bevorzugt ausgewählt ist aus der Gruppe, bestehend aus einer chemischen Bindung, CH₂, O, S, Carbonyl, NH, CH(C₁-C₆)Alkyl, N(C₁-C₆)Alkyl, OCH₂, und SCH₂ wobei bei den beiden letztgenannten Gruppen das Kohlenstoffatom an den aromatischen Teil und das Heteroatom O oder S an den teilhydrierten Teil des Amins gebunden ist;
- X: besonders bevorzugt ausgewählt ist aus der Gruppe bestehend aus einer chemischen Bindung, CH₂, O, S, CHCH₃, NCH₃, OCH₂, und SCH₂ wobei bei den beiden letztgenannten Gruppen das Kohlenstoffatom an den aromatischen Teil und das Heteroatom O oder S an den teilhydrierten Teil des Amins gebunden ist; und in welchen
- X: am meisten bevorzugt für eine chemische Bindung oder CH₂ steht.

Eine zwölfte Ausführungsform der vorliegenden Erfindung umfasst Verbindungen der allgemeinen Formel (I), in welchen
- n: bevorzugt 0 oder 1 ist.

Im Rahmen der vorliegenden Erfindung ist es möglich, die einzelnen bevorzugten, besonders bevorzugten, ganz besonders bevorzugten und am meisten bevorzugten Bedeutungen für die Substituenten R¹ bis R¹¹ und X beliebig miteinander zu kombinieren, wobei die Laufzahl n 0, 1 oder 2 ist, bevorzugt 0 oder 1 ist.

Das heißt, dass Verbindungen der allgemeinen Formel (I) von der vorliegenden Erfindung umfasst sind, in welchen beispielsweise der Substituent R¹ eine bevorzugte Bedeutung aufweist und die Substituenten R² bis R¹⁴ die allgemeine Bedeutung aufweisen oder aber der Substituent R² eine bevorzugte Bedeutung aufweist, der Substituent R³ eine besonders bevorzugte, bzw. eine ganz besonders bevorzugte, Bedeutung aufweist und die übrigen Substituenten eine allgemeine Bedeutung aufweisen.

Vier dieser Kombinationen der oben für die Substituenten R¹ bis R¹¹ und X gegebenen Definitionen werden nachfolgend beispielhaft erläutert und jeweils als weitere Ausführungsformen offenbart:
Kombination der oben für die Substituenten R¹ bis R¹¹ und X jeweils als besonders bevorzugt bezeichneten Definitionen (zwölfte Ausführungsform), Kombination der oben für die Substituenten R¹ bis R¹¹ und X jeweils als ganz besonders bevorzugt bezeichneten Definitionen (dreizehnten Ausführungsform), und Kombination der oben für den Substituenten R¹ als ganz besonders bevorzugt bezeichneten Definition mit den oben für die Substituenten R¹ bis R¹¹ und X jeweils als besonders bevorzugt bezeichneten Definitionen (vierzehnte Ausführungsform).

Die vorgenannten auf den Kombinationen der Substituenten basierenden weiteren Ausführungsformen werden nachfolgend aus Gründen der Klarheit explizit offenbart:
Eine dreizehnte Ausführungsform der vorliegenden Erfindung umfasst Verbindungen der allgemeinen Formel (I), in welchen
- R¹: ausgewählt ist aus der Gruppe, bestehend aus Wasserstoff, Amino, ((C₁-C₃)-alkyl)amino, ((C₃-C₆)-cycloalkyl)amino, Di((C₁-C₃)-alkyl)amino;
- R²: ausgewählt ist aus der Gruppe, bestehend aus Cl, Br, Cyano, C(O)OH, C(O)NH₂; (C₁-C₆)-Alkyl, (C₁-C₆)-Halogenalkyl, (C₁-C₆)-Alkylcarbonyl, (C₁-C₆)-Haloalkylcarbonyl; (C₁-C₆)-Alkoxycarbonyl, (C₁-C₆)-Haloalkoxycarbonyl; (C₂-C₆)-Alkenyl, (C₂-C₆)-Halogenalkenyl: (C₂-C₆)-Alkinyl, (C₂-C₆)-Halogenalkinyl; Phenyl, welches mit F, Cl, Br, Methyl und/oder Trifluormethyl substituiert sein kann; (C₁-C₆)-Alkoxy-(C₁-C₆)-alkyl, Hydroxy-(C₁-C₆)-alkyl; ((C₁-C₂)-alkyl)amino-carbonyl, Di-((C₁-C₂)-alkyl)amino-carbonyl, N-((C₁-C₂)-Alkylsulfonyl)amino-carbonyl, N-((C₁-C₂)-Haloalkylsulfonyl)amino-carbonyl; (C₃-C₆)-Cycloalkylcarbonyl, (C₃-C₆)-Cycloalkoxycarbonyl; (C₁-C₃)-Alkylsulfonyl, (C₁-C₃)-Alkylsulfinyl, (C₁-C₃)-Haloalkylsulfonyl, (C₁-C₆)-Halogenalkylsulfinyl, (C₄-C₁₄)-Arylsulfonyl, (C₆-C₁₄)-Arylsulfinyl;
- R³: Wasserstoff ist;
- R⁴ und R⁵: jeweils unabhängig voneinander ausgewählt sind aus der Gruppe, bestehend aus Wasserstoff, (C₁-C₆)-Alkyl und (C₁-C₆)-Alkoxy;
- R⁶ und R⁷: unabhängig voneinander ausgewählt sind aus der Gruppe, bestehend aus Wasserstoff, Methyl und Phenyl;
- R⁸: ausgewählt ist aus der Gruppe, bestehend aus Wasserstoff, Halogen, (C₁-C₃)-Alkyl und (C₆-C₈)-Aryl;
- R⁹: ausgewählt ist aus der Gruppe, bestehend aus Wasserstoff, Chlor, Fluor und (C₁-C₃)-Alkoxy;
- R¹⁰: ausgewählt ist aus der Gruppe, bestehend aus Wasserstoff, Fluor, Chlor, Brom, Iod, Cyano, Methoxy (OCH₃), (C₁-C₃)-Alkyl, (C₁-C₃)-Haloalkyl, (C₁-C₃)-Alkoxy, (C₂-C₄)-Alkenyl, (C₂-C₄)-Alkinyl, (C₁-C₃)-Alkyl-(C₂-C₄)-alkinyl, Hydroxy-(C₁-C₃)-Alkyl-(C₂-C₄)-alkinyl, (C₁-C₃)-Alkoxy-(C₂-C₄)-Alkinyl und Phenyl-(C₂-C₄)-Alkinyl;
- R¹¹: ausgewählt ist aus der Gruppe, bestehend aus Wasserstoff und (C₁-C₄)-Alkyl; und
- X: ausgewählt ist aus der Gruppe bestehend aus einer chemischen Bindung, CH₂, O, S, CHCH₃, NCH₃, OCH₂, und SCH₂ wobei bei den beiden letztgenannten Gruppen das Kohlenstoffatom an den aromatischen Teil und das Heteroatom O oder S an den teilhydrierten Teil des Amins gebunden ist.

Eine vierzehnte Ausführungsform der vorliegenden Erfindung umfasst Verbindungen der allgemeinen Formel (I), in welchen
- R¹: ausgewählt ist aus der Gruppe bestehend aus Wasserstoff, Amino, NH(CH₃), N(H)Cyclopropyl, und N(CH₃)₂;
- R²: ausgewählt ist aus der Gruppe, bestehend aus Cl, Cyano, C(O)NH₂; (C₁-C₃)-Halogenalkyl, (C₁-C₃)-Alkylcarbonyl, (C₁-C₃)-Haloalkylcarbonyl; (C₁-C₆)-Alkoxycarbonyl, (C₁-C₆)-Haloalkoxycarbonyl; (C₂-C₆)-Alkenyl, (C₂-C₆)-Halogenalkenyl; (C₁-C₃)-Alkoxy-(C₁-C₆)-alkyl, Hydroxy-(C₁-C₆)-alkyl; ((C₁-C₂)-alkyl)amino-carbonyl, Di-((C₁-C₂)-alkyl)amino-carbonyl (C₁-C₃)-Alkylsulfonyl, (C₁-C₃)-Alkylsulfinyl;
- R³: Wasserstoff ist;
- R⁴ und R⁵: jeweils unabhängig voneinander, ausgewählt sind aus der Gruppe, bestehend aus Wasserstoff, (C₁-C₃)-Alkyl, (C₁-C₆)-Alkoxy;
- R⁶ und R⁷: jeweils unabhängig voneinander Wasserstoff oder Methyl sind;
- R⁸: ausgewählt ist aus der Gruppe, bestehend aus Wasserstoff, Fluor, Chlor, CH₃, und Phenyl;
- R⁹: ausgewählt ist aus der Gruppe, bestehend aus Wasserstoff, Fluor, Chlor und Methoxy;
- R¹⁰: ausgewählt ist aus der Gruppe, bestehend aus Wasserstoff, Fluor, Chlor, Brom, Iod, Cyano, Methyl, C≡CH, und C≡CCH3;
- R¹¹: Wasserstoff oder Methyl ist; und
- X: ausgewählt ist aus der Gruppe bestehend aus einer chemischen Bindung, CH₂, O, S, CHCH₃, NCH₃, OCH₂, und SCH₂ wobei bei den beiden letztgenannten Gruppen das Kohlenstoffatom an den aromatischen Teil und das Heteroatom O oder S an den teilhydrierten Teil des Amins gebunden ist.

Eine fünfzehnte Ausführungsform der vorliegenden Erfindung umfasst Verbindungen der allgemeinen Formel (I), in welchen
- R¹: Wasserstoff und Amino ist;
- R²: ausgewählt ist aus der Gruppe, bestehend aus Cl, Cyano, C(O)NH₂, Trifluormethyl, Acetyl (C(O)CH₃), Trifluoracetyl (C(O)CF₃), (C₁-C₂)-Alkoxycarbonyl, (C₁-C₂)-Haloalkoxycarbonyl, (C₂-C₃)-Alkenyl, (C₂-C₃)-Halogenalkenyl, (C₁-C₃)-Alkoxy-(C₁-C₃)-alkyl, Hydroxy-(C₁-C₃)-alkyl, Methylamino-carbonyl, Dimethylamino-carbonyl, Methylsulfonyl und Methylsulfinyl;
- R³: Wasserstoff ist;
- R⁴ und R⁵: jeweils unabhängig voneinander Wasserstoff oder Methyl sind;
- R⁶ und R⁷: Wasserstoff sind;
- R⁸: Wasserstoff oder CH₃ ist;
- R⁹: Wasserstoff ist;
- R¹⁰: Wasserstoff oder CH₃ ist;
- R¹¹: Wasserstoff ist; und
- X: für eine chemische Bindung oder CH₂ steht.

Eine sechzehnte Ausführungsform der vorliegenden Erfindung umfasst Verbindungen der allgemeinen Formel (I), in welchen
- R¹: ausgewählt ist aus der Gruppe bestehend aus Wasserstoff, Amino, NH(CH₃), N(H)Cyclopropyl, und N(CH₃)₂;
- R²: ausgewählt ist aus der Gruppe, bestehend aus Cl, Cyano, C(O)NH₂; (C₁-C₃)-Halogenalkyl, (C₁-C₃)-Alkylcarbonyl, (C₁-C₃)-Haloalkylcarbonyl; (C₁-C₆)-Alkoxycarbonyl, (C₁-C₆)-Haloalkoxycarbonyl; (C₂-C₆)-Alkenyl, (C₂-C₆)-Halogenalkenyl; Methoxy-(C₂-C₃)-alkyl, Hydroxy-(C₂-C₃)-alkyl; ((C₁-C₂)-alkyl)amino-carbonyl, Di-((C₁-C₂)-alkyl)amino-carbonyl (C₁-C₃)-Alkylsulfonyl, (C₁-C₃)-Alkylsulfinyl;
- R³: Wasserstoff ist;
- R⁴ und R⁵: zusammen mit dem Kohlenstoffatom, an welchem sie gebunden sind, einen gesättigten drei- bis sechsgliedrigen Ring bilden, der ein Sauerstoffatom enthalten kann;
- R⁶ und R⁷: jeweils unabhängig voneinander Wasserstoff oder Methyl sind;
- R⁸: ausgewählt ist aus der Gruppe, bestehend aus Wasserstoff, Fluor, Chlor, CH₃, und Phenyl;
- R⁹: ausgewählt ist aus der Gruppe, bestehend aus Wasserstoff, Fluor, Chlor und Methoxy;
- R¹⁰: ausgewählt ist aus der Gruppe, bestehend aus Wasserstoff, Fluor, Chlor, Brom, Iod, Cyano, Methyl, C=CH, und C≡CCH3;
- R¹¹: Wasserstoff oder Methyl ist; und
- X: für eine chemische Bindung oder CH₂ steht.

Im Rahmen der vorliegenden Erfindung sind von der Verbindung der allgemeinen Formel (I) auch Verbindungen umfasst, die durch a) Protonierung, b) Alkylierung oder c) Oxidation an einem Stickstoffatom quaterniert sind. Insbesondere sind diesebzüglich die entsprechenden N-Oxide zu nennnen.

Die Verbindungen der Formel (I) können Salze bilden. Salzbildung kann durch Einwirkung einer Base auf solche Verbindungen der Formel (I) erfolgen, die ein acides Wasserstoffatom tragen. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R"' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 Kohlenstoffatomen, insbesondere 1 bis 4 Kohlenstoffatomen, bzw. bei ungesättigten Gruppen mit 2 bis 6 Kohlenstoffatomen, insbesondere 2 bis 4 Kohlenstoffatomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexy und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen, sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder lod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CF₂Cl, CHF₂, CH₂F, CF₃CF₂, CH₂FCHCI, CCI₃, CHCI₂, CH₂CH₂CI; Haloalkoxy ist z.B. OCF₃, OCHF₂, OCH₂F, CF₃CF₂O, OCH₂CF₃ und OCH₂CH₂CI; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Vor allem aus den Gründen der höheren herbiziden Wirkung, besseren Selektivität und/oder besseren Herstellbarkeit sind erfindungsgemäße Verbindungen der allgemeinen Formel (I) oder deren agrochemischen Salze oder quartären N-Derivate von besonderem Interesse, worin einzelne Reste eine der bereits genannten oder im Folgenden genannten bevorzugten Bedeutungen haben, oder insbesondere solche, worin eine oder mehrere der bereits genannten oder im folgenden genannten bevorzugten Bedeutungen kombiniert auftreten.

Die oben angeführten allgemeinen oder in Vorzugsbereichen angeführten Restedefinitionen gelten sowohl für die Endprodukte der allgemeinen Formel (I) als auch entsprechend für die die jeweils zur Herstellung benötigten Ausgangs- und Zwischenprodukte. Diese Restedefinitionen können untereinander, als auch zwischen den angegebenen bevorzugten Bereichen vertauscht werden.

Die vorliegenden Verbindungen der allgemeinen Formel (I) weisen an der Bindestelle zum Amino-1,2,4-triazin ein chirales Kohlenstoffatom auf, welches in der unten dargestellten Struktur durch die Kennzeichnung (*) verdeutlicht ist:

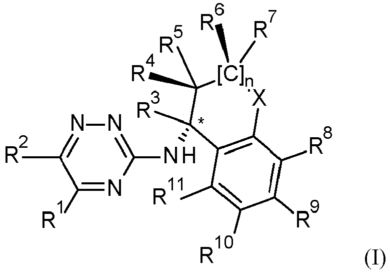

Gemäß den Regeln nach Cahn, Ingold und Prelog (CIP-Regeln) kann dieses Kohlenstoffatom sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration erfasst, d.h., dass die vorliegende Erfindung die Verbindungen der allgemeinen Formel (I) erfasst, in welchen das betreffende Kohlenstoffatom
(1) eine (R)-Konfiguration; oder
(2) eine (S)-Konfiguration
   aufweist.
   Darüber hinaus werden im Rahmen der vorliegenden Erfindung auch
(3) beliebige Mischungen von Verbindungen der allgemeinen Formel (I), welche eine (R)-Konfiguration (Verbindungen der allgemeinen Formel (I-(R)) aufweisen, mit Verbindungen der allgemeinen Formel (I), welche eine (S)-Konfiguration (Verbindungen der allgemeinen Formel (I-S)) aufweisen, erfasst, wobei eine racemische Mischung der Verbindungen der allgemeinen Formel (I) mit (R)- und (S)-Konfiguration von der vorliegenden Erfindung ebenfalls umfasst ist.

Allerdings sind im Rahmen der vorliegenden Erfindung insbesondere Verbindungen der allgemeinen Formel (I) mit (R)-Konfiguration mit einer Selektivität von 60 bis 100%, vorzugsweise 80 bis 100%, insbesondere 90 bis 100%, ganz besonders 95 bis 100%, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (R)-Verbindung vorliegt.

Daher betrifft die vorliegende Erfindung insbesondere Verbindungen der allgemeinen Formel (I*), in welchen die stereochemische Konfiguration an dem mit (*) gekennzeichnet Kohlenstoffatom mit einer stereochemischen Reinheit von 60 bis 100 % (R), vorzugsweise 80 bis 100 % (R), insbesondere 90 bis 100 % (R), ganz besonders 95 bis 100 % (R), vorliegt.

Unter Berücksichtigung der Regel nach Cahn, Ingold und Prelog kann es an dem mit (*) gekennzeichneten Kohlenstoffatom auch zu einer Situation kommen, in welcher aufgrund der Priorität der jeweiligen Substituenten die (S)-Konfiguration am mit (*) gekennzeichneten Kohlenstoffatom bevorzugt ist. Dieses ist beispielweise dann der Fall, wenn die Reste R⁴ und/oder R⁵ einem C₁-C₆-Alkoxyrest entsprechen.

Daher sind im Rahmen der vorliegenden Erfindung insbesondere Verbindungen der allgemeinen Formel (I) bevorzugt, die in ihrer räumlichen Anordnung den jenigen Verbindungen der allgemeinen Formel (I) mit R⁴ und R⁵ =Wasserstoff mit (R)-Konfiguration entsprechen, mit einer Selektivität von 60 bis 100 %, vorzugsweise 80 bis 100 %, insbesondere 90 bis 100 %, ganz besonders 95 bis 100 %, bevorzugt, wobei die jeweilige (R)-analoge-Verbindung mit einer Enantioselektivität von jeweils mehr als 50 % ee, vorzugsweise 60 bis 100 % ee, insbesondere 80 bis 100 % ee, ganz besonders 90 bis 100 % ee, meist bevorzugt 95 bis 100 % ee, bezogen auf den Gesamtgehalt an betreffender (R)-analogen-Verbindung, vorliegt. Daher betrifft die vorliegende Erfindung insbesondere Verbindungen der allgemeinen Formel (I), in welchen die stereochemische Konfiguration an dem mit (*) gekennzeichnet Kohlenstoffatom mit einer stereochemischen Reinheit von 60 bis 100 % (R, bzw. analog-R), vorzugsweise 80 bis 100 % (R, bzw. analog-R), insbesondere 90 bis 100 % (R, bzw. analog-R), ganz besonders 95 bis 100 % (R, bzw. analog-R), vorliegt.

Insbesondere können die erfindungsgemäßen Verbindungen der allgemeinen Formel (I) noch weitere Chiralitätszentren an den mit (**) und (***) gekennzeichneten Kohlenstoffatomen aufweisen:

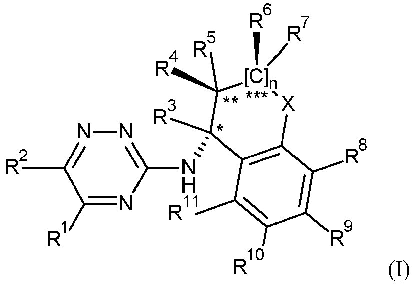

Im Rahmen der vorliegenden Erfindung sind beliebige stereochemische Konfigurationen an den mit (*), (**) und (***) gekennzeichneten Kohlenstoffatomen möglich:

| **Konfiguration Kohlenstoffatom (*)** | **Konfiguration Kohlenstoffatom (**)** | **Konfiguration Kohlenstoffatom (***)** |
|---|---|---|
| R | R | R |
| R | R | S |
| R | S | R |
| S | R | R |
| R | S | S |
| S | R | S |
| S | S | R |
| S | S | S |

Darüber hinaus können, je nach Wahl der jeweiligen Reste, weitere Stereoelemente in den erfindungsgemäßen Verbindungen der allgemeinen Formel (I) vorliegen.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Besonders bevorzugt sind Verbindungen der allgemeinen Formel (I) 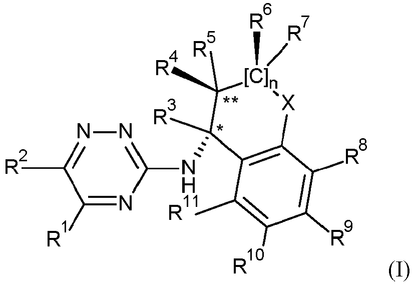 in denen das chirale Kohlenstoffatom mit der Kennzeichnung (*) eine (R)-Konfiguration und das chirale Kohlenstoffatom mit der Kennzeichnung (**) eine (S)-Konfiguration aufweist.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Im Folgenden werden in tabellarischer Form Beispiele der Verbindungen der allgemeinen Formel (I) wiedergegeben.

In der nachfolgenden Tabelle 1 werden die in Formel (I) allgemein definierten Substituenten spezifiziert. Dabei steht:
- "StNR³" für stereochemische Anordnung am Kohlenstoffatom, an das NH und R³ gebunden sind, "StR⁴R⁵" und "StR⁶R⁷" steht analog für die Kohlenstoffatome an die die jeweiligen Substituenten gebunden sind,
- die Bindung der Substituenten ist jeweils links,
- sofern für X zwei Bindestellen angegeben sind, bindet die linke Bindung an den Aromaten und die rechte Bindung an den hydrierten Teil des bicyclischen Amins,
- ein Bindestrich "-" für eine direkte Bindung, und
- falls n=0 ist, enthält die Tabelle in dem entsprechenden Feld für R⁶ und R⁷ keinen Eintrag.

**Tabelle 1: Beispiele der Verbindungen der allgemeinen Formel (I)**

| **Nr.** | **R¹** | **R²** | **R³** | **St N R³** | **R⁴** | **R⁵** | **St R⁴ R⁵** | **R⁶** | **R⁷** | **n** | **St R⁶ R⁷** | **R⁸** | **R⁹** | **R¹⁰** | **R¹¹** | **X** |
|---|---|---|---|---|---|---|---|---|---|---|---|---|---|---|---|---|
| 1.1 | N(H)Cyclopropyl | Cl | H | R | H | H | | H | H | 2 | | H | H | H | H | - |
| 1.2 | N(H)Cyclopropyl | Cl | H | R | CH₃ | H | **S** | H | H | 1 | | H | H | CH₃ | H | - |
| 1.3 | NH₂ | Cl | H | R | CH₃ | H | S | H | H | 1 | | H | H | CH₃ | H | - |
| 1.4 | NH₂ | Cl | H | R | H | H | | H | H | 2 | | H | H | H | H | - |
| 1.5 | NH₂ | *t*-Butyl | H | R | H | H | | H | H | 2 | | H | H | H | H | - |
| 1.6 | NH₂ | *t*-Butyl | H | R | CH₃ | H | S | H | H | 1 | | H | H | CH₃ | H | - |
| 1.7 | NH₂ | *t*-Butyl | H | R | H | H | | H | H | 1 | | H | H | H | H | - |
| 1.8 | NH₂ | *t*-Butyl | H | rac | H | H | | H | H | 1 | | H | H | H | H | O |
| 1.9 | N(H)CH₃ | Cl | H | rac | H | H | | H | H | 2 | | H | H | H | H | - |
| 1.10 | N(H)Cyclopropyl | Cl | H | rac | H | H | | H | H | 2 | | H | H | H | H | - |
| 1.11 | N(H)Cyclopropyl | Cl | H | rac | H | H | | H | H | 1 | | H | H | H | H | - |
| 1.12 | N(CH₃)₂ | Cl | H | rac | H | H | | H | H | 1 | | H | H | H | H | - |
| 1.13 | N(H)CH₃ | Cl | H | rac | H | H | | H | H | 1 | | H | H | H | H | - |
| 1.14 | N(H)Cyclopropyl | Cl | H | rac | H | H | | H | H | 1 | | H | H | H | H | O |
| 1.15 | N(CH₃)₂ | Cl | H | rac | H | H | | H | H | 2 | | H | H | H | H | - |
| 1.16 | N(H)Cyclopropyl | Cl | H | R | H | H | | H | H | 1 | | H | H | H | H | O |
| 1.17 | N(CH₃)₂ | Cl | H | R | H | H | | H | H | 1 | | H | H | H | H | O |
| 1.18 | N(H)Cyclopropyl | Cl | H | rac | H | H | | H | H | 1 | | CH₃ | H | F | H | O |
| 1.19 | N(H)CH₃ | Cl | H | rac | H | H | | H | H | 1 | | CH₃ | H | F | H | O |
| 1.20 | N(H)CH₃ | Cl | H | rac | H | H | | H | H | 1 | | H | H | H | H | O |
| 1.21 | NH₂ | C(O)OCH₂CH₃ | H | R | CH₃ | H | S | H | H | 1 | | H | H | CH₃ | H | - |
| 1.22 | NH₂ | CF₃ | H | R | H | H | | H | H | 2 | | H | H | H | H | - |
| 1.23 | NH₂ | CF₃ | H | R | CH₃ | H | S | H | H | 1 | | H | H | CH₃ | H | - |
| 1.24 | NH₂ | CF₃ | H | R | H | H | | H | H | 1 | | H | H | H | H | - |
| 1.25 | NH₂ | CN | H | R | CH₃ | H | S | H | H | 1 | | H | H | CH₃ | H | - |
| 1.26 | NH₂ | C(O)CH₃ | H | R | CH₃ | H | S | H | H | 1 | | H | H | CH₃ | H | - |
| 1.27 | NH₂ | C(O)OH | H | R | H | H | | H | H | 1 | | H | H | H | H | - |
| 1.28 | NH₂ | C(O)OH | H | R | H | H | | H | H | 2 | | H | H | H | H | - |
| 1.29 | NH₂ | C(O)CH₃ | H | R | H | H | | H | H | 1 | | H | H | H | H | - |
| 1.30 | NH₂ | C(O)CH₃ | H | R | H | H | | H | H | 2 | | H | H | H | H | - |
| 1.31 | NH₂ | CF₃ | H | R | H | H | | H | H | 1 | | H | H | CH₃ | H | - |
| 1.32 | NH₂ | C(O)OCH₂CH₃ | H | R | H | H | | H | H | 2 | | H | H | H | H | - |
| 1.33 | NH₂ | C(O)OCH₂CH₃ | H | R | H | H | | H | H | 1 | | H | H | H | H | - |
| 1.34 | NH₂ | C(O)N(H)CH₃ | H | R | H | H | | H | H | 2 | | H | H | H | H | - |
| 1.35 | NH₂ | C(O)N(H)CH₃ | H | rac | H | H | | H | H | 1 | | H | H | H | H | - |
| 1.36 | NH₂ | C(O)OH | H | R | CH₃ | H | S | H | H | 1 | | H | H | CH₃ | H | - |
| 1.37 | NH₂ | CN | H | R | H | H | | H | H | 2 | | H | H | H | H | - |
| 1.38 | NH₂ | CN | H | R | H | H | | H | H | 1 | | H | H | H | H | - |
| 1.39 | NH₂ | C(O)N(H)CH(CH₃)₂ | H | R | CH₃ | H | S | H | H | 1 | | H | H | CH₃ | H | - |
| 1.40 | NH₂ | C(O)N(CH₃)₂ | H | R | CH₃ | H | S | H | H | 1 | | H | H | CH₃ | H | - |
| 1.41 | NH₂ | C(O)N(H)CH₃ | H | R | CH₃ | H | S | H | H | 1 | | H | H | CH₃ | H | - |
| 1.42 | NH₂ | C(O)N(H)(CH₂)₂C(O)OEt | H | R | CH₃ | H | S | H | H | 1 | | H | H | CH₃ | H | - |
| 1.43 | NH₂ | C(O)N(H)CH₂C(O)OEt | H | R | CH₃ | H | S | H | H | 1 | | H | H | CH₃ | H | - |
| 1.44 | NH₂ | S(O)CH₃ | H | R | CH₃ | H | S | H | H | 1 | | H | H | CH₃ | H | - |
| 1.45 | NH₂ | S(O)₂CH₃ | H | R | CH₃ | H | S | H | H | 1 | | H | H | CH₃ | H | - |
| 1.46 | NH₂ | CF₃ | H | rac | H | H | | H | H | 2 | | H | F | H | H | - |
| 1.47 | NH₂ | CF₃ | H | rac | H | H | | H | H | 1 | | H | H | H | H | O |
| 1.48 | NH₂ | CF₃ | H | rac | H | H | | | | 0 | | H | H | H | H | O |
| 1.49 | NH₂ | CF₃ | H | rac | H | H | | H | H | 1 | | H | H | F | H | O |
| 1.50 | NH₂ | CF₃ | H | S | H | H | | H | H | 1 | | H | H | CH₃ | H | - |
| 1.51 | NH₂ | C(O)N(H)S(O)₂CH₃ | H | R | CH₃ | H | S | H | H | 1 | | H | H | CH₃ | H | - |
| 1.52 | NH₂ | CF₃ | H | R | H | H | | H | H | 2 | | H | H | CH₃ | H | - |
| 1.53 | NH₂ | C(O)N(H)S(O)₂CH₂CH₂CH₃ | H | R | CH₃ | H | S | H | H | 1 | | H | H | CH₃ | H | - |
| 1.54 | NH₂ | C(O)N(H)S(O)₂(4-Cl-Ph) | H | R | CH₃ | H | S | H | H | 1 | | H | H | CH₃ | H | - |
| 1.55 | NH₂ | C(O)N(H)S(O)₂Ph | H | R | CH₃ | H | S | H | H | 1 | | H | H | CH₃ | H | - |
| 1.56 | NH₂ | C(O)N(H)S(O)₂(4-NO₂-Ph) | H | R | CH₃ | H | S | H | H | 1 | | H | H | CH₃ | H | - |
| 1.57 | NH₂ | C(O)N(H)S(O)₂CF₃ | H | R | CH₃ | H | S | H | H | 1 | | H | H | CH₃ | H | - |
| 1.58 | NH₂ | Cl | H | rac | H | H | | H | H | 1 | | H | H | CH₃ | H | - |
| 1.59 | NH₂ | Cl | H | R | H | H | | H | H | 1 | | H | H | H | H | - |
| 1.60 | NH₂ | CN | H | rac | H | H | | H | H | 1 | | H | H | H | H | O |
| 1.61 | NH₂ | CN | H | S | H | H | | H | H | 1 | | H | H | CH₃ | H | - |
| 1.62 | NH₂ | CN | H | rac | H | H | | | | 0 | | H | H | H | H | O |
| 1.63 | NH₂ | CN | H | rac | H | H | | H | H | 2 | | H | F | H | H | - |
| 1.64 | NH₂ | CN | H | rac | H | H | | H | H | 1 | | H | H | F | H | O |
| 1.65 | NH₂ | S(O)CH₃ | H | R | H | H | | H | H | 2 | | H | H | H | H | - |
| 1.66 | NH₂ | S(O)₂CH₃ | H | R | H | H | | H | H | 2 | | H | H | H | H | - |
| 1.67 | NH₂ | CF₃ | H | R | H | H | | H | H | 1 | | H | H | CH₃ | H | - |
| 1.68 | NH₂ | S(O)₂CH₃ | H | R | H | H | | H | H | 2 | | H | H | CH₃ | H | - |
| 1.69 | NH₂ | S(O)CH₃ | H | R | H | H | | H | H | 2 | | H | H | CH₃ | H | - |
| 1.70 | NH₂ | CN | H | R | H | H | | H | H | 1 | | H | H | CH₃ | H | - |
| 1.71 | NH₂ | S(O)CH₃ | H | R | H | H | | H | H | 1 | | H | H | CH₃ | H | - |
| 1.72 | NH₂ | C(O)CH₃ | H | R | H | H | | H | H | 1 | | H | H | CH₃ | H | - |
| 1.73 | NH₂ | S(O)₂CH₃ | H | R | H | H | | H | H | 1 | | H | H | CH₃ | H | - |
| 1.74 | NH₂ | C(O)OCH₂CH₃ | H | R | H | H | | H | H | 1 | | H | H | CH₃ | H | - |
| 1.75 | NH₂ | C(O)OH | H | R | H | H | | H | H | 1 | | H | H | CH₃ | H | - |
| 1.76 | NH₂ | C(O)N(H)CH₃ | H | R | H | H | | H | H | 1 | | H | H | CH₃ | H | - |
| 1.77 | NH₂ | C(O)N(CH₃)₂ | H | R | H | H | | H | H | 1 | | H | H | CH₃ | H | - |
| 1.78 | NH₂ | C(O)NH₂ | H | R | H | H | | H | H | 1 | | H | H | CH₃ | H | - |
| 1.79 | NH₂ | 4-Cl-Ph | H | R | CH₃ | H | S | H | H | 1 | | H | H | CH₃ | H | - |
| 1.80 | NH₂ | C(H)=CH₂ | H | R | CH₃ | H | S | H | H | 1 | | H | H | CH₃ | H | - |
| 1.81 | NH₂ | C(CF₃)=CH₂ | H | R | CH₃ | H | S | H | H | 1 | | H | H | CH₃ | H | - |
| 1.82 | NH₂ | CF₃ | H | S | OH | H | S | H | H | 1 | | H | H | H | H | - |
| 1.83 | NH₂ | CF₃ | H | R | OH | H | S | H | H | 1 | | H | H | H | H | - |
| 1.84 | NH₂ | CF₃ | H | rac | OH | H | rac | H | H | 1 | | H | H | H | H | - |
| 1.85 | NH₂ | CF₃ | H | rac | OMe | H | rac | H | H | 1 | | H | H | H | H | - |
| 1.86 | NH₂ | CF₃ | H | s | OMe | H | S | H | H | 1 | | H | H | H | H | - |
| 1.87 | NH₂ | S(O)₂CH₃ | H | s | OH | H | S | H | H | 1 | | H | H | H | H | - |
| 1.88 | NH₂ | S(O)₂CH₃ | H | rac | OH | H | rac | H | H | 1 | | H | H | H | H | - |
| 1.89 | NH₂ | S(O)₂CH₃ | H | rac | OMe | H | rac | H | H | 1 | | H | H | H | H | - |
| 1.90 | NH₂ | CN | H | s | OH | H | s | H | H | 1 | | H | H | H | H | - |
| 1.91 | NH₂ | CN | H | rac | OH | H | rac | H | H | 1 | | H | H | H | H | - |
| 1.92 | NH₂ | CN | H | rac | OMe | H | rac | H | H | 1 | | H | H | H | H | - |
| 1.93 | NH₂ | CF₃ | H | R | OMe | H | S | H | H | 1 | | H | H | H | H | - |
| 1.94 | NH₂ | S(O)₂CH₃ | H | R | OMe | H | S | H | H | 1 | | H | H | H | H | - |
| 1.95 | NH₂ | CN | H | R | OMe | H | S | H | H | 1 | | H | H | H | H | - |
| 1.96 | NH₂ | C(O)OCH₂CH₃ | H | R | OMe | H | S | H | H | 1 | | H | H | H | H | - |
| 1.97 | NH₂ | CF₃ | H | R | OMe | H | R | H | H | 1 | | H | H | H | H | - |
| 1.98 | NH₂ | S(O)₂CH₃ | H | R | OMe | H | R | H | H | 1 | | H | H | H | H | - |
| 1.99 | NH₂ | CN | H | R | OMe | H | R | H | H | 1 | | H | H | H | H | - |
| 1.100 | NH₂ | C(O)OCH₂CH₃ | H | R | OMe | H | R | H | H | 1 | | H | H | H | H | - |
| 1.101 | NH₂ | CH₂OH | H | R | CH₃ | H | S | H | H | 1 | | H | H | CH₃ | H | - |
| 1.102 | NH₂ | CH₂OH | H | R | H | H | | H | H | 1 | | H | H | CH₃ | H | - |
| 1.103 | NH₂ | CH(CH₃)OH | H | R | CH₃ | H | S | H | H | 1 | | H | H | CH₃ | H | - |
| 1.104 | NH₂ | CH(CH₃)OH | H | R | H | H | | H | H | 1 | | H | H | CH₃ | H | - |
| 1.105 | NH₂ | C(CH₃)₂OH | H | R | CH₃ | H | S | H | H | 1 | | H | H | CH₃ | H | - |
| 1.106 | NH₂ | C(CH₃)₂OH | H | R | H | H | | H | H | 1 | | H | H | CH₃ | H | - |
| 1.107 | H | C(CH₃)₂OH | H | R | CH₃ | H | S | H | H | 1 | | H | H | CH₃ | H | - |
| 1.108 | H | C(CH₃)₂OH | H | R | H | H | | H | H | 1 | | H | H | CH₃ | H | - |
| 1.109 | H | CH(CH₃)OH | H | R | CH₃ | H | S | H | H | 1 | | H | H | CH₃ | H | - |
| 1.110 | H | CH(CH₃)OH | H | R | H | H | | H | H | 1 | | H | H | CH₃ | H | - |
| 1.111 | H | C(CH₃)₂F | H | R | CH₃ | H | S | H | H | 1 | | H | H | CH₃ | H | - |
| 1.112 | H | C(CH₃)₂F | H | R | H | H | | H | H | 1 | | H | H | CH₃ | H | - |
| 1.113 | H | CH(CH₃)F | H | R | CH₃ | H | S | H | H | 1 | | H | H | CH₃ | H | - |
| 1.114 | H | CH(CH₃)F | H | R | H | H | | H | H | 1 | | H | H | CH₃ | H | - |
| 1.115 | H | S(O)₂CH₃ | H | R | CH₃ | H | S | H | H | 1 | | H | H | CH₃ | H | - |
| 1.116 | H | S(O)₂CH₃ | H | R | H | H | | H | H | 1 | | H | H | CH₃ | H | - |
| 1.117 | NH₂ | C(O)CF₃ | H | R | CH₃ | H | S | H | H | 1 | | H | H | CH₃ | H | - |
| 1.118 | NH₂ | C(O)CF₃ | H | R | H | H | | H | H | 1 | | H | H | CH₃ | H | - |
| 1.119 | NH₂ | CH₃ | H | R | CH₃ | H | S | H | H | 1 | | H | H | CH₃ | H | - |
| 1.120 | NH₂ | CH₃ | H | R | H | H | | H | H | 1 | | H | H | CH₃ | H | - |
| 1.121 | NH₂ | cyclopropyl | H | R | CH₃ | H | S | H | H | 1 | | H | H | CH₃ | H | - |
| 1.122 | NH₂ | CH(CH₃)F | H | R | CH₃ | H | S | H | H | 1 | | H | H | CH₃ | H | - |
| 1.123 | NH₂ | CH(CH₃)F | H | R | H | H | | H | H | 1 | | H | H | CH₃ | H | - |
| 1.124 | NH₂ | C(CH₃)₂F | H | R | CH₃ | H | S | H | H | 1 | | H | H | CH₃ | H | - |
| 1.125 | NH₂ | C(CH₃)₂F | H | R | H | H | | H | H | 1 | | H | H | CH₃ | H | - |
| 1.126 | H | C(CH₃)₂OCH₃ | H | R | CH₃ | H | S | H | H | 1 | | H | H | CH₃ | H | - |
| 1.127 | NH₂ | C(CH₃)₂OCH₃ | H | R | CH₃ | H | S | H | H | 1 | | H | H | CH₃ | H | - |
| 1.128 | H | C(CH₃)₂OCH₃ | H | R | H | H | | H | H | 1 | | H | H | CH₃ | H | - |
| 1.129 | NH₂ | C(CH₃)₂OCH₃ | H | R | H | H | | H | H | 1 | | H | H | CH₃ | H | - |
| 1.130 | H | CHF₂ | H | R | CH₃ | H | S | H | H | 1 | | H | H | CH₃ | H | - |
| 1.131 | NH₂ | CHF₂ | H | R | CH₃ | H | S | H | H | 1 | | H | H | CH₃ | H | - |
| 1.132 | H | CHF₂ | H | R | H | H | | H | H | 1 | | H | H | CH₃ | H | - |
| 1.133 | NH₂ | CHF₂ | H | R | H | H | | H | H | 1 | | H | H | CH₃ | H | - |
| 1.134 | H | CF₃ | H | R | CH₃ | H | S | H | H | 1 | | H | H | CH₃ | H | - |
| 1.135 | H | CF₃ | H | R | H | H | | H | H | 1 | | H | H | CH₃ | H | - |
| 1.136 | NH₂ | S(O)CH₃ | H | R | OMe | H | S | H | H | 1 | | H | H | H | H | - |
| 1.137 | NH₂ | S(O)₂CH₃ | H | R | OH | H | S | H | H | 1 | | H | H | H | H | - |
| 1.138 | NH₂ | S(O)CH₃ | H | R | OH | H | S | H | H | 1 | | H | H | H | H | - |
| 1.139 | NH₂ | S(O)CH₃ | H | rac | OH | H | rac | H | H | 1 | | H | H | H | H | - |
| 1.140 | NH₂ | CN | H | R | OH | H | S | H | H | 1 | | H | H | H | H | - |
| 1.141 | NH₂ | C≡CH | H | R | CH₃ | H | S | H | H | 1 | | H | H | CH₃ | H | - |
| 1.142 | NH₂ | C≡CH | H | R | H | H | | H | H | 1 | | H | H | CH₃ | H | - |
| 1.143 | NH₂ | C≡CSi(CH₃)₃ | H | R | CH₃ | H | S | H | H | 1 | | H | H | CH₃ | H | - |
| 1.144 | NH₂ | C≡CSi(CH₃)₃ | H | R | H | H | | H | H | 1 | | H | H | CH₃ | H | - |
| 1.145 | NH₂ | OMe | H | R | CH₃ | H | S | H | H | 1 | | H | H | CH₃ | H | - |
| 1.146 | NH₂ | OMe | H | R | H | H | | H | H | 1 | | H | H | CH₃ | H | - |
| 1.147 | NH₂ | C≡CCH₃ | H | R | CH₃ | H | S | H | H | 1 | | H | H | CH₃ | H | - |
| 1.148 | NH₂ | C≡CCH₃ | H | R | H | H | | H | H | 1 | | H | H | CH₃ | H | - |
| 1.149 | H | C(O)CH₃ | H | R | CH₃ | H | S | H | H | 1 | | H | H | CH₃ | H | - |
| 1.150 | H | C(O)CH₃ | H | R | H | H | | H | H | 1 | | H | H | CH₃ | H | - |
| 1.151 | H | C(CH₃)₂OH | H | R | CH₃ | H | S | H | H | 1 | | H | H | H | H | - |
| 1.152 | H | C(CH₃)₂OH | H | R | H | H | | H | H | 1 | | H | H | H | H | - |
| 1.153 | H | C(CH₃)₂OH | H | R | H | H | | H | H | 2 | | H | H | H | H | - |
| 1.154 | H | C(CH₃)₂OH | H | rac | H | H | | H | H | 1 | | H | H | H | H | O |
| 1.155 | H | C(CH₃)₂OH | H | R | H | H | | H | H | 1 | | H | H | CH₂CH₃ | H | - |
| 1.156 | H | C(CH₃)₂OH | H | s | OH | H | S | H | H | 1 | | H | H | H | H | - |
| 1.157 | H | C(CH₃)₂OH | H | s | OCH₃ | H | S | H | H | 1 | | H | H | H | H | - |
| 1.158 | H | C(CH₃)₂OH | H | R | H | H | | H | H | 2 | | OCH₃ | H | H | H | - |
| 1.159 | H | C(CH₃)₂OH | H | R | H | H | | H | H | 2 | | CH₃ | H | H | H | - |
| 1.160 | H | C(CH₃)₂OH | H | R | H | H | | H | H | 2 | | H | H | CH₃ | H | - |
| 1.161 | H | C(CH₃)₂OH | H | R | CH₃ | H | S | H | H | 2 | | H | H | CH₂CH₃ | H | - |
| 1.162 | H | C(CH₃)₂OH | H | R | H | H | | H | H | 2 | | H | F | H | H | - |
| 1.163 | H | C(CH₃)₂OH | H | R | H | H | | H | H | 1 | | H | H | CH₃ | H | O |
| 1.164 | H | C(CH₃)₂OH | H | rac | H | H | | H | H | 1 | | H | H | F | H | O |
| 1.165 | H | C(CH₃)₂OH | H | rac | H | H | | | | 0 | | H | H | H | H | O |
| 1.166 | H | C(CH₃)₂OH | H | R | H | H | | H | H | 2 | | H | H | F | H | - |
| 1.167 | H | C(CH₃)₂OH | H | R | H | H | | H | H | 1 | | H | H | F | H | - |
| 1.168 | H | C(CH₃)₂OH | H | R | H | H | | H | H | 2 | | H | H | CH₂CH₃ | H | - |
| 1.169 | NH₂ | S(O)₂CH₃ | H | R | H | H | | H | H | 2 | | CH₃ | H | H | H | - |
| 1.170 | NH₂ | CF₃ | H | R | H | H | | H | H | 2 | | CH₃ | H | H | H | - |
| 1.171 | NH₂ | S(O)₂CH₃ | H | rac | H | H | | H | H | 2 | | CH₃ | H | H | H | - |
| 1.172 | NH₂ | CF₃ | H | rac | H | H | | H | H | 2 | | CH₃ | H | H | H | - |

Weiterer Gegenstand der vorliegenden Erfindung sind Verfahren zur Herstellung entsprechender Verbindungen der allgemeinen Formel (I) und/oder deren Salze und/oder deren agrochemisch verträglichen quarternierten Stickstoff-Derivate 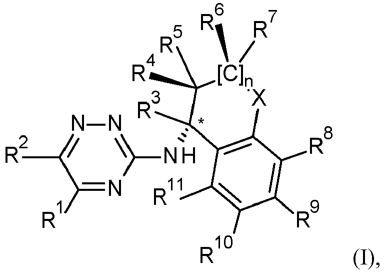 in welchen die Reste R¹ bis R¹¹ sowie X und n die vorstehenden Bedeutungen aufweisen, und wobei nach einem ersten Verfahren
a) eine Verbindung der allgemeinen Formel (II) 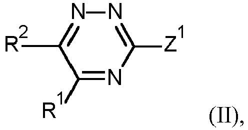 wobei R¹, R² die vorstehende Bedeutung aufweisen und Z¹ für einen austauschfähigen Rest oder eine Abgangsgruppe steht,
   mit einem Amin der allgemeinen Formel (III) oder einem Säureadditionssalz des Amins der allgemeinen Formel (III) 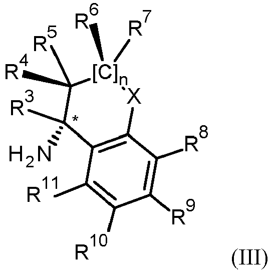 umgesetzt wird,
   wobei die Reste R³ bis R¹¹ sowie X und n die vorstehende Bedeutung aufweisen.
   Der austauschfähige Rest Z¹, bzw. die Abgangsgruppe Z¹ steht für Fluor, Chlor, Brom, Jod, ein (C₁-C₄)-Alkylsulfanyl oder ein (C₁-C₄)-Alkylsulfinyl oder ein (C₁-C₄)-Alkylsulfonyl, ein unsubstituiertes oder ein einfach oder mehrfach mit Fluor, Chlor, Brom oder (C₁-C₄)-Alkyl oder (C₁-C₄)-Alkoxy substituiertes Phenyl-(C₁-C₄)-alkylsulfonyl oder ein (C₁-C₄)-Alkylphenyl-sulfonyl.
   Gegebenenfalls kann ein Rest Z¹ in eine andere, besser austauschbare Gruppe überführt werden. So kann beispielsweise im Sinne eines Zweistufen-Eintopfverfahren ein (C₁-C₄)-Alkylsulfanyl mit einem Oxidationsmittel wie m-Chlorperbenzoesäure oder Oxone® in ein (C₁-C₄)-Alkylsulfinyl oder ein (C₁-C₄)-Alkylsulfonyl oder Mischungen davon überführt werden und anschließend mit einem Amin der allgemeinen Formel (III) oder einem Säureadditionssalz unter Verwendung einer Hilfsbase wie z.B. Triethylamin oder Kaliumcarbonat zur Reaktion gebracht werden.
   Gegebenenfalls kann die Umsetzung auch durch unterschiedliche Hilfsstoffe katalysiert werden, wie beispielswiese durch die Reagenzien Kaliumphosphat, Kupfer(I)iodid und N,N-Diethyl-2-hydroxybenzamid oder im Sinne der Buchwald-Hartwig-Kupplung durch spezielle Übergangsmetallkatalysatorsysteme.
   Die Verbindungen der allgemeinen Formel (II) sind kommerziell verfügbar oder können nach bekannten Verfahren hergestellt werden. Substituierte 1,2,4-Triazine der allgemeinen Formel (II) stellen eine dem Fachmann sehr gut bekannte Verbindungsklasse dar. Es gibt eine große Anzahl unterschiedlicher allgemein anwendbarer Synthesemethoden (für eine Übersicht siehe zum Beispiel Houben-Weyl Methods of Organic Chemistry, 4th Edition (1997), Vol. E 9c, Hetarenes IV, Seite 582-666 und dort zitierte Literatur).
   Die Amine der allgemeinen Formel (III) oder die Säureadditionssalz davon sind kommerziell verfügbar oder deren Synthese ist in WO 2004/069814 A1 beschrieben.
b.) Verbindungen der allgemeinen Formel (I) können auch dadurch hergestellt werden, dass zunächst eine Verbindung der allgemeinen Formel (II-a), 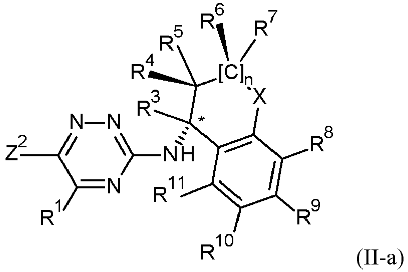 wobei Z² ein Rest aus der Gruppe Halogen, Cyano, (C₁-C₆)-Alkoxycarbonyl, (C₁-C₆)-Alkylcarbonyl und (C₁-C₆)-Alkylthio ist, der verändert oder ausgetauscht werden kann, nach dem unter a) beschriebenen Verfahren hergestellt wird.
   So kann beispielsweise in dem Fall, dass Z² Cyano bedeutet, die Cyano Gruppe mit Basen wie Natronlauge oder Kaliumhydroxid zu Carbonsäureamiden verseift werden.
   In dem Fall, dass Z² ein Halogenatom, bevorzugt Chlor, ist, kann dieses beispielsweise mit Alkoholen gegebenenfalls unter Verwendung von zusätzlichen Basen wie Natrium, Natriumhydrid oder dem Alkoholat des betreffenden Alkohols zu Alkoxyderivaten in der Position R² umgesetzt werden.
   In dem Fall, dass Z² ein Halogen, bevorzugt Brom oder Jod ist, kann dieses beispielsweise unter Palladium-Katalyse mit aromatischen, heteroaromatischen oder aliphatischen Boronsäuren (Suzuki-Reaktion) oder mit Alkenen oder Alkinen zu den entsprechenden Zielstrukturen der Formel (I) umgesetzt werden.
   Im dem Fall, dass Z² (C₁-C₆)-Alkylthio bedeutet kann diese Gruppe mit einem Oxidationsmittel wie m-Chlorperbenzoesäure oder Oxone® in ein (C₁-C₆)-Alkylsulfinyl oder ein (C₁-C₆)-Alkylsulfonyl übergeführt werden.
   Im dem Fall, dass Z² (C₁-C₆)-Alkoxycarbonyl bedeutet, können diese Ester beispielsweise mit Basen wie Natronlauge oder Kaliumhydroxid zu den entsprechenden Carbonsäuren [Formel 1, R² = C(O)OH] verseift werden. Die resultierenden Carbonsäuren können ihrerseits wiederum beispielsweise mit Aminen unter Verwendung von Hilfsreagenzien wie Propanphosphonsäureanhydrid (T3P) zu substituierten Carbonsäureamiden in der Position R² umgesetzt werden. Die resultierenden Carbonsäuren können beispielsweise auch in Carbonsäurechloride übergeführt und dann mit Aminen zu substituierten Carbonsäureamiden in der Position R² umgesetzt werden.
   Im dem Fall, dass Z² (C₁₋C₆)-Alkoxycarbonyl bedeutet, können diese Ester beispielsweise mit Grignard-Verbindungen (R^{A}MgHal) zu den entsprechenden Alkoholen [Formel 1, R² = C(R^{A})₂OH] umgesetzt werden.
   Im dem Fall, dass Z² (C₁-C₆)-Alkylcarbonyl bedeutet, können diese Ketone beispielsweise mit Grignard-Verbindungen (R^{A}MgHal) zu den entsprechenden Alkoholen [Formel 1, R² = C[(C₁-C₆)-Alkyl](R^{A})OH] umgesetzt werden.
   Gegebenenfalls kann auch ein Rest Z² in einen zunächst anderen Rest Z² übergeführt werden. So kann beispielsweise nach den zuvor beschriebenen Verfahren zunächst ein Intermediat des Typs (II-a) hergestellt werden, in dem der Rest Z² ein Halogen, bevorzugt Chlor ist, welches mit Nucleophilen wie beispielsweise Natriumcyanid in das Nitril (Z² = Cyano) oder beispielsweise mit Mercaptanen, gegebenenfalls unter Verwendung von zusätzlichen Basen wie Natrium, Natriumhydrid oder dem Thiolat des betreffenden Thiols in Verbindungen mit Z² = (C₁-C₆)-Alkylthio umgewandelt werden kann.
c.) Verbindungen der allgemeinen Formel (I), in denen R¹ Amino bedeutet, können auch dadurch hergestellt werden, in dem Guanidine des Typs (IV) oder Säureadditionssalze davon 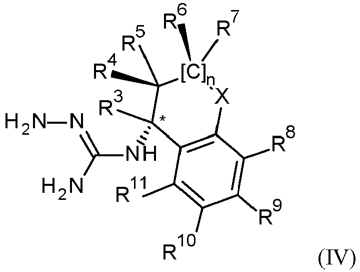 mit Acylcyaniden der Formel (V) 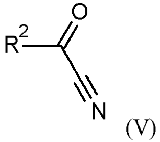 kondensiert werden (siehe zum Beispiel Houben-Weyl Methods of Organic Chemistry, 4th Edition (1997), Vol. E 9c, Hetarenes IV, Seite 597; Chem. Ber. 1960, 93, 2209).
d.) Verbindungen der allgemeinen Formel (II) in denen der Rest Z¹ für (C₁-C₄)-Alkylthio und der Rest R¹ für Amino steht können analog dem unter c.) beschrieben Verfahren hergestellt werden, wobei statt (IV) S-(C₁-C₄)-Alkylisothiosemicarbazide oder deren Säureadditionssalze der allgemeinen Formel (IVa) 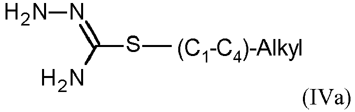 eingesetzt werden (siehe zum Beispiel Houben-Weyl Methods of Organic Chemistry, 4th Edition (1997), Vol. E 9c, Hetarenes IV, Seite 597; Bull. Chem. Soc. Jpn. 1978, 51, 1846).

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Reaktionsstationen (reaction stations) der Firma Radleys, Shirehill, Saffron Walden, Essex, CB 11 3AZ, England oder MultiPROBE Automated Workstations der Firma Perkin Elmer, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma Teledyne ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Herbizide werden in landwirtschaftlich genutzten Kulturen während verschiedener Anbauphasen eingesetzt. So erfolgt die Applikation einiger Produkte schon vor oder während der Saat. Andere werden ausgebracht, bevor die Kulturpflanze aufläuft, d.h. bevor der Keimling die Erdoberfläche durchbricht (Vorauflauf-Herbizide). Nachauflauf-Herbizide schließlich werden verwendet, wenn von der Kulturpflanze entweder bereits die Keimblätter oder Laubblätter ausgebildet sind.

Die erfindungsgemäßen Verbindungen können dabei sowohl im Vorlauf als auch im Nachlauf angewendet werden, wobei eine Verwendung der erfindungsgemäßen Verbindungen im Vorlauf bevorzugt ist.

Die Vorauflauf-Behandlung schließt sowohl die Behandlung der Anbaufläche vor der Aussaat (ppi = pre plant incorporation) als auch die Behandlung der angesäten, aber noch nicht bewachsenen Anbauflächen ein.

Die erfindungsgemäßen Verbindungen der Formel (I) und deren Salze, im folgenden auch synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotylen Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259),
- transgene Kulturpflanzen mit modifizierter Fettsäurezusammensetzung (WO 91/013972),
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461),
- gentechnisch veränderte Pflanzen mit reduzierter Photorespiration, die höhere Erträge und höhere Stresstoleranz aufweisen (EP 0305398),
- transgene Kulturpflanzen, die pharmazeutisch oder diagnostisch wichtige Proteine produzieren ("molecular pharming"),
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking").

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird anhand der nachfolgenden Beispiele näher erläutert, welche die Erfindung jedoch keinesfalls beschränken.

### A. Synthesebeispiele

### 6-Chlor-N³-[(1R,2S)-2,6-dimethyl-2,3-dihydro-1H-inden-1-yl]-1,2,4-triazin-3,5-diamin (Bspl. 1.3)

1.100 g (6.667 mmol) 3,6-Dichlor-1,2,4-triazin-5-anün, 1.290 g (8.001 mmol) (1R,2S)-2,6-Dimethylindan-1-amin und 5.210 g (26.669 mmol) Dicyclohexylmethylamin wurden in 10.0 ml 1-Methylpyrrolidin-2-on in einem Mikrowellenofen 1h bei 160°C erhitzt. Man goß das Gemisch auf Wasser und extrahierte dreimal mit Ethylacetat. Man wusch die vereinigten organischen Phasen nacheinander viermal mit Wasser und einmal mit gesättigter wäßriger Natriumchlorid-Lösung, trocknete über Natriumsulfat, destillierte das Lösungsmittel im Vakuum und reinigte den Rückstand durch Chromatographie (Start: Ethylacetat/n-Heptan (5 : 95), innerhalb von 30 min auf Ethylacetat/n-Heptan (60 : 40). Nach Kristallisation des Rohprodukts aus einem Gemisch von Ethylacetat und n-Heptan (4:1) erhielt man 1.210 g (44%) 6-Chlor-N³-[(1R,2S)-2,6-dimethyl-2,3-dihydro-1H-inden-1-yl]-1,2,4-triazin-3,5-diamin.

### 6-(4-Chlorphenyl)-N³-[(1R,2S)-2,6-dimethyl-2,3-dihydro-1H-inden-1-yl]-1,2,4-triazin-3,5-diamin (Bspl. 1.79)

Zu einer Lösung von 0.150 g (0.518 mmol) 6-Chlor-N³-[(1R,2S)-2,6-dimethyl-2,3-dihydro-1H-inden-1-yl]-1,2,4-triazin-3,5-diamin (siehe oben, Bspl. 1.3) in 4.0 ml 1,2-Dimethoxyethan gab man unter Argon nacheinander 0.185 g (0.776 mmol) 2-(4-Chlorphenyl)-4,4,5,5-tetramethyl-1,3,2-dioxaborolan, 0.018 g (0.026 mmol) Bis(triphenylphosphin)palladium(II)chlorid und 0.414 ml (1.035 mmol) einer 2.5 molaren wäßrigen Cäsiumcarbonat-Lösung. Man erhitzte das Gemisch in einem Mikrowellenofen 3h bei 80°C. Man gab Wasser und Ethylacetat zu und wusch die wäßrige Phase zweimal mit Ethylacetat. Nach Trocknen der vereinigten organischen Phasen über Natriumsulfat, destillierte man das Lösungsmittel im Vakuum und reinigte den Rückstand durch Chromatographie [Start: Ethylacetat/n-Heptan (5 : 95), innerhalb von 25 min auf Ethylacetat/n-Heptan (50 : 50)]. Man erhielt 0.153 g (81%) 6-(4-Chlorphenyl)-N^{3_}[(1R,2S)-2,6-dimethyl-2,3-dihydro-1H-inden-1-yl]-1,2,4-triazin-3,5-diamin.

### Ethyl-5-amino-3-[(1R)-1,2,3,4-tetrahydronaphthalin-1-ylamino]-1,2,4-triazin-6-carboxylat (Bspl 1.32)

Zu einer Lösung von 0.300g (1.400 mmol) Ethyl-5-amino-3-(methylsulfanyl)-1,2,4-triazin-6-carboxylat in 20 ml Trichlormethan tropfte man 0.759 g (3.081 mmol) meta-Chlorperbenzoesäure gelöst in 20 ml Trichlormethan. Man rührte 1h bei 0°C und gab nacheinander 0.543 g (4.201 mmol) N,N-Diisopropylethylamin und 0.309 g (2.100 mmol) (1R)-1,2,3,4-Tetrahydronaphthalin-1-amin gelöst in 2 ml Trichlormethan zu. Man rührte 2h bei 50°C, versetzte das Gemisch mit Wasser und Dichlormethan und extrahierte die wässrige Phase zweimal mit Dichlormethan. Nach Trocknen der vereinigten organischen Phase über Natriumsulfat, entfernte man das Lösungsmittel im Vakuum und nahm den Rückstand in Acetonitril auf. Nach Filtration vom unlöslichen Rückstand (3-Chlorbenzoesäure) entfernte man das Lösungsmittel im Vakuum und erhielt nach Reinigung des Rückstands durch Chromatographie [Start: Ethylacetat/n-Heptan (5 : 95), innerhalb von 60 min auf Ethylacetat/n-Heptan (45 : 55)] 0.338g (73%) Ethyl-5-amino-3-[(1R)-1,2,3,4-tetrahydronaphthalin-1-ylamino]-1,2,4-triazin-6-carboxylat.

### 5-Amino-3-[(1R)-1,2,3,4-tetrahydronaphthalin-1-ylaminol-1,2,4-triazin-6-carbonsäure (Bspl. 1.28)

0.245 g (0.782 mmol) Ethyl-5-amino-3-[(1R)-1,2,3,4-tetrahydronaphthalin-l-ylamino]-1,2,4-triazin-6-carboxylat (siehe oben, Bspl. 1.32) wurden in 6 ml Methanol aufgenommen und zu einer Lösung von 0.263 g (4.691 mmol) Kaliumhydroxid in 6 ml Wasser gegeben. Das Gemisch wurde 2 Stunden unter Rückfluss erhitzt. Nach Entfernen der Alkohole im Vakuum stellte man die verbliebene wässrige Lösung mit konzentrierter Salzsäure auf pH 2. Nach Filtration und Trocknen des entstandenen Niederschlags erhielt man 0.169 g (72%) 5-Amino-3-[(1R)-1,2,3,4-tetrahydronaphthalin-1-ylamino]-1,2,4-triazin-6-carbonsäure als farblosen Feststoff.

### 5-Amino-N-methyl-3-[(1R)-1,2,3,4-tetrahydronaphthalin-1-ylamino]-1,2,4-triazin-6-carboxamid (Bspl. 1.34)

Zu einer Lösung von 0.130 g (0.456 mmol) 5-Amino-3-[(1R)-1,2,3,4-tetrahydronaphthalin-1-ylamino]-1,2,4-triazin-6-carbonsäure (siehe oben, Bspl. 1.28), 0.017g (0.547 mmol) Methanamin (2 molare Lösung in Tetrahydrofuran) und 0.138 g (1.367 mmol) Triethylamin in 5 ml Dichlormethan gab man bei 0°C 0.377 g (0.592 mmol) Propanphosphonsäureanhydrid (T3P, 50%-ige Lösung in Tetrahydrofuran) und rührte 12h bei 25°C. Man versetzte das Gemisch mit Wasser und extarhierte die wässrige Phase zweimal mit Dichlormethan. Nach Trocknen der vereinigten organischen Phasen über Natriumsulfat destillierte man das Lösungsmittel im Vakuum und erhielt nach Reinigung des Rückstands durch Chromatographie [Start: Ethylacetat/n-Heptan (5 : 95), innerhalb von 40 min auf 100% Ethylacetat] 0.015 (11%) 5-Amino-N-methyl-3-[(1R)-1,2,3,4-tetrahydronaphthalin-1-ylamino]-1,2,4-triazin-6-carboxamid.

### 1-{5-Amino-3-[(1R)-2,3-dihydro-1H-inden-1-ylammo]-1,2,4-triazin-6-yl}ethanon (Bspl. 1.29)

1. Zu einer Lösung von 1,000 g (4.673 mmol) Ethyl-5-amino-3-(methylsulfanyl)-1,2,4-triazin-6-carboxylat in 20 ml Tetrahydrofuran gab man 0.785 g (18.692 mmol) Lithiumhydroxid und rührte 4h bei 25°C. Man entfernte das Lösungsmittel im Vakuum gab zum Rückstand 20 ml Wasser und stellte mit 4N Salzsäure auf pH 4. Nach Filtration und Trocknen des entstandenen Niederschlags erhielt man 0.800 g (90%) 5-Amino-3-(methylsulfanyl)-1,2,4-triazin-6-carbonsäure als farblosen Feststoff.
2. Zu einer lösung von 0.800 g (4.255 mmol) 5-Amino-3-(methylsulfanyl)-1,2,4-triazin-6-carbonsäure in 10 ml Dimethylformamid gab man nacheinander 1.79 ml Triethylamin, 0.781 g (5.106 mmol) 1-Hydroxybenzotriazol (HOBt), 0.975 g (5.106 mmol) N'-(3-Dimethylaminopropyl)-N-ethyl-carbodiimid Hydrochlorid (EDCxHCl) und 0.500 g (5.106 mmol) N-Methoxymethanamin und rührte 16h bei 25°C. Man versetzte das Gemisch mit 20 ml Wasser, und extrahierte die wässrige Phase dreimal mit Ethylacetat. Nach Trocknen der vereinigten organischen Phasen über Natriumsulfat destillierte man das Lösungsmittel im Vakuum und erhielt 0.870 g (88%) 5-Amino-N-methoxy-N-methyl-3-(methylsulfanyl)-1,2,4-triazin-6-carboxamid als gelbes Öl.
3. Zu einer Lösung von 0.600g (2.597 mmol) 5-Amino-N-methoxy-N-methyl-3-(methylsulfanyl)-1,2,4-triazin-6-carboxamid in 10 ml Tetrahydrofuran gab man bei 0°C 6.67 ml (12.987 mmol) Methylmagnesiumbromid (1 molare Lösung in THF) und rührte 5h bei 25°C. Man tropfte zum Gemisch gesättigte wäßrige Ammoniumchlorid-Lösung und extarhierte die wässrige Phase dreimal mit Ethylacetat. Nach Trocknen der vereinigten organischen Phasen über Natriumsulfat destillierte man das Lösungsmittel im Vakuum und erhielt nach Reinigung des Rückstands durch Chromatographie [Ethylacetat/n-Hexan (20 : 80)] 0.200 g (41%) 1-[5-Amino-3-(methylsulfanyl)-1,2,4-triazin-6-yl]ethanon.¹H-NMR (400MHz in CDCl₃): δ = 2.54 (3H, s), 2.62 (3H, s), 8.20 (1H, bs), 8.52 (1H, bs).
4. Zu einer Lösung von 0.150g (0.814 mmol) 1-[5-Amino-3-(methylsulfanyl)-1,2,4-triazin-6-yl]ethanon in 40 ml Trichlormethan tropfte man bei 0°C 0.442 g (1.791 mmol) meta-Chlorperbenzoesäure gelöst in 20 ml Trichlormethan. Man rührte 1h bei 20°C und gab nacheinander 0.316 g (2.443 mmol) N,N-Diisopropylethylamin und 0.163 g (1.221 mmol) (1R)-Indan-1-amin gelöst in 2 ml Trichlormethan zu. Man rührte 2h bei 50°C, versetzte das Gemisch mit Wasser und Dichlormethan und extrahierte die wässrige Phase zweimal mit Dichlormethan. Nach Trocknen der vereinigten organischen Phase über Natriumsulfat, entfernte man das Lösungsmittel im Vakuum und nahm den Rückstand in Acetonitril auf. Nach Filtration vom unlöslichen Rückstand (3-Chlorbenzoesäure) entfernte man das Lösungsmittel im Vakuum und erhielt nach Reinigung des Rückstands durch Chromatographie [Start: Ethylacetat/n-Heptan (5 : 95), innerhalb von 60 min auf Ethylacetat/n-Heptan (45 : 55)] 0.108g (49%) 1-{5-Amino-3-[(1R)-2,3-dihydro-1H-inden-1-ylamino]-1,2,4-triazin-6-yl}ethanon (Bspl. 1.29)

### N³-(3,4-Dihydro-2H-chromen-4-yl)-6-(trifluormethyl)-1,2,4-triazin-3,5-diamin (Bspl. 1.47)

1. Zu einer Lösung von 18.00 g (42.92 mmol) 5-Chlor-3-(methylsulfanyl)-6-(trifluormethyl)-1,2,4-triazin in 33 ml 1,4-Dioxan gab man 25 ml einer gesättigten wäßrigen Ammoniumhydroxid-Lösung und rührte 10 min bei 25°C. Man fügte 150 ml Wasser zu. Nach Filtration und Waschen des entstandenen Feststoffs mit Wasser und n-Hexan erhielt man 14.6 g (88%) 3-(Methylsulfanyl)-6-(trifluormethyl)-1,2,4-triazin-5-amin als braunen Feststoff. ¹H-NMR (400MHz in CDCl₃): δ = 2.61 (3H, s), 5.51 (2H, bs).
2. 0.250 g (1.130 mmol) 3-(Methylsulfanyl)-6-(trifluormethyl)-1,2,4-triazin-5-amin und 0.337 g (2.260 mmol) Chroman-4-amin werden in 2.0 ml 1-Methylpyrrolidin-2-on 30h bei 180°C erhitzt. Das Lösungsmittel wurde im Hochvakuum entfernt, der Rückstand in Ethylacetat und Wasser aufgenommen und die wässrige Phase dreimal mit Ethylacetat extrahiert. Man wusch die vereinigten organischen Phasen nacheinander einmal mit Wasser und einmal mit gesättigter wäßriger Natriumchlorid-Lösung, trocknete über Natriumsulfat, destillierte das Lösungsmittel im Vakuum und reinigte den Rückstand durch Chromatographie (Acetonitril/Wasser). Aus der Produktfraktion entfernte man das Acetonitril im Vakuum, stellte die verbliebene wässrige Lösung mit gesättigter wäßriger Natriumcarbonat-Lösung auf pH 9 und extrahierte dreimal mit Dichlormethan. Nach Trocknen der vereinigten organischen Phasen über Natriumsulfat destillierte man das Lösungsmittel im Vakuum und erhielt 0.035 g (10%) N³-(3,4-Dihydro-2H-chromen-4-yl)-6-(trifluormethyl)-1,2,4-triazin-3,5-diamin (Bspl. 1.47)

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾ δ₂ (Intensität₂);........; δᵢ; (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel 1.1:¹H-NMR(400,1 MHz, CDCl₃): δ= 7,517(0,5);7,400(3,3);7,383(4,1);7,378(4,2);7,259(82,8);7,254(7,8);7,206(0,9);7,201(1,4);7,188 (3,6);7,183(4,5);7,175(3,8);7,172(8,0);7,169(7,2);7,166(6,8);7,157(3,7);7,152(4,3);7,138(1,5);7,134(1,1);7,122(4,5);7,116(4,6);7,106(2,1);7,1 03(2,5);7,100(2,8);6,995(0,5);5,570(2,2);5,296(16,0);5,245(0,6);5,225(0,5);3,371(0,5);3,196(0,6);2,896(0,7);2,881(1,3);2,865(0,8);2,854(1,9); 2,839(3,6);2,823(2,4);2,813(3,0);2,796(4,8);2,780(3,6);2,754(1,3);2,738(0,9);2,714(0,4);2,149(0,5);2,138(0,8);2,127(1,0);2,116(1,7);2,105(2, 0);2,094(1,8);2,085(1,6);2,073(1,3);2,015(1,0);2,002(1,6);1,987(1,5);1,980(1,5);1,970(1,4);1,965(1,4);1,957(1,2);1,950(1,4);1,938(1,5);1,931( 1,5);1,923(1,4);1,916(2,0);1,906(1,5);1,896(2,3);1,887(1,8);1,881(2,1);1,872(2,2);1,864(1,8);1,861(1,8);1,855(1,7);1,852(1,7);1,845(1,5);1,83 6(1,3);1,827(0,8);1,818(0,7);1,810(0,6);1,802(0,5);1,636(0,7);1,628(0,7);1,624(0,7);1,606(0,6);1,292(0,3);1,284(0,4);1,274(0,5);1,256(1,2);1, 231(0,4);0,886(1,8);0,882(1,3);0,869(7,6);0,855(6,8);0,852(6,5);0,848(3,9);0,839(2,1);0,815(0,4);0,797(0,3);0,645(2,0);0,632(5,9);0,627(6,2); 0,623(6,2);0,606(1,7);0,008(0,8);0,000(30,3) |
| Beispiel 1.2: ¹H-NMR(400,1 MHz, CDCl₃): δ= 10,681(0,9);10,661(1,0);7,518(0,8);7,360(0,4);7,310(2,1);7,260(108,6);7,211(1,5);7,160(0,3);7, 089(1,7);7,070(3,6);7,036(2,6);7,016(1,3);7,000(3,6);6,559(1,1);5,158(1,1);5,138(2,1);5,117(1,1);3,165(1,3);3,146(1,4);3,127(1,5);3,108(1,5); 2,978(0,4);2,968(0,9);2,958(1,3);2,950(1,8);2,940(1,9);2,931(1,3);2,922(1,0);2,912(0,5);2,701(0,6);2,688(0,6);2,664(0,9);2,646(1,1);2,627(0, 9);2,607(0,6);2,550(1,3);2,528(0,9);2,512(1,2);2,490(0,9);2,352(0,4);2,302(16,0);1,273(12,3);1,266(1,3);1,256(12,1);1,232(0,3);1,009(0,5);1, 004(0,6);0,998(1,1);0,991(3,4);0,986(1,8);0,980(1,8);0,975(2,9);0,973(3,1);0,967(1,4);0,963(1,0);0,958(0,9);0,783(1,1);0,780(1,2);0,773(3,0); 0,771(3,0);0,763(3,0);0,757(2,0);0,748(1,0);0,737(0,3);0,050(0,7);0,008(0,9) |
| Beispiel 1.3: ¹H-NMR(400,0 MHz, CDCl₃): δ= 7,518(0,6);7,259(102,6);7,086(1,5);7,066(3,1);7,031(4,3);7,015(1,7);6,995(0,6);5,324(1,5);5,29 7(5,0);3,064(1,1);3,045(1,3);3,026(1,4);3,006(1,4);2,537(0,9);2,515(1,1);2,499(0,8);2,477(1,0);2,301(16,0);2,284(0,8);2,264(0,9);2,245(0,7);1 ,284(0,5);1,270(12,6);1,253(12,4);0,008(1,3);0,000(43,8);-0,009(1,3) |
| Beispiel 1.4: ¹H-NMR(400,1 MHz, CDCl₃): δ=7,520(2,0);7,361(0,5);7,334(5,7);7,315(7,1);7,261(358,3);7,213(1,6);7,205(2,1);7,191(7,1);7,18 7(7,3);7,174(16,0);7,169(16,0);7,155(7,5);7,151(7,7);7,137(3,0);7,122(9,3);7,103(5,7);7,075(0,5);7,051(0,4);6,997(2,1);5,935(0,3);5,901(0,5); 5,869(0,5);5,852(0,5);5,828(0,4);5,557(0,3);5,552(0,3);5,472(0,4);5,419(0,9);5,279(6,4);5,213(2,3);5,062(0,5);2,886(1,1);2,870(1,9);2,843(3, 3);2,827(6,1);2,808(4,5);2,792(6,4);2,775(4,1);2,749(1,9);2,734(1,2);2,123(1,6);2,090(3,9);2,079(3,2);2,064(2,7);2,045(1,2);1,893(6,7);1,878( 9,2);1,862(8,1);1,845(4,0);1,733(0,4);1,720(0,4);1,712(0,4);1,582(54,0);1,407(0,4);1,321(0,9);1,304(1,7);1,265(7,6);1,229(0,7);0,899(3,6);0,8 82(9,5);0,864(4,2);0,146(0,6);0,050(0,4);0,000(119,4) |
| Beispiel 1.5: ¹H-NMR(400,1 MHz, CDCl₃): δ= 7,386(0,4);7,370(0,4);7,365(0,4);7,262(6,1);7,260(6,5);7,171(0,4);7,167(0,4);7,154(0,9);7,140( 0,4);7,136(0,5);7,111(0,5);7,106(0,5);5,298(1,6);5,297(1,7);5,166(0,5);3,001(0,7);2,820(0,4);2,802(0,3);2,784(0,4);2,716(1,0);2,043(0,3);1,89 8(0,3);1,447(16,0);1,433(0,4);1,407(0,3);1,228(3,5);0,002(2,1);0,000(2,3) |
| Beispiel 1.6: ¹H-NMR(400,1 MHz, CDCl₃): δ= 7,259(9,2);7,081(0,4);7,071(0,6);7,063(0,7);7,014(0,4);5,297(4,7);3,000(0,5);2,715(0,6);2,714( 0,7);2,292(2,7);2,043(0,5);1,468(0,5);1,455(16,0);1,436(0,4);1,419(0,8);1,284(2,2);1,273(4,0);1,267(2,4);1,258(0,5) |
| Beispiel 1.7: ¹H-NMR(400,1 MHz, CDCl₃): δ= 7,352(0,3);7,265(0,4);7,259(18,3);7,255(0,7);7,254(0,6);7,253(0,6);7,252(0,6);7,251(0,7);7,250 (0,8);7,246(0,8);7,234(0,5);7,231 (0,4);7,217(0,5);5,085(0,3);1 ,451 (16,0); 1,287(3,3) |
| Beispiel 1.8: ¹H-NMR(400,0 MHz, CDCl₃): δ= 7,293(55,9);7,287(0,4);7,204(0,5);7,202(0,5);7,199(0,5);7,187(0,3);7,185(0,4);7,183(0,5);7,181 (0,4);6,904(0,3);6,888(0,3);6,885(0,6);6,873(0,6);6,871(0,5);6,852(0,5);6,849(0,4);4,312(0,3);2,261(0,5);2,247(0,5);1,458(16,0);0,041(0,5);0, 037(0,4);0,033(14,9);0,025(0,4) |
| Beispiel 1.14: ¹H-NMR(399,8 MHz, CDCl₃): δ= 12,248(0,5);9,846(0,4);8,814(1,2);7,519(2,1);7,371(0,7);7,329(9,6);7,310(10,3);7,260(374,7); 7,215(4,7);7,212(4,8);7,194(9,9);7,177(6,1);7,173(5,7);6,996(2,2);6,925(7,2);6,907(12,5);6,888(5,7);6,862(12,1);6,841(10,6);5,612(5,8);5,23 3(5,9);5,159(0,7);4,323(2,0);4,309(3,1);4,295(6,5);4,283(9,2);4,271(11,5);4,260(6,4);4,249(6,7);4,240(8,2);4,233(2,5);4,220(2,5);4,212(2,5);4 ,111(0,5);3,702(0,5);3,563(0,6);3,535(0,7);3,508(0,7);2,830(0,5);2,803(3,6);2,795(4,8);2,786(4,8);2,777(3,6);2,769(2,4);2,759(1,1);2,597(1,9) ;2,314(1,3);2,292(3,4);2,280(5,0);2,271(7,7);2,258(10,3);2,249(8,4);2,236(3,8);2,224( 1,8);2,212(1,4);2,177(0,7);2,071(0,5);2,055(0,5);2,044( 0,8);1,607(0,7);1,537(141,6);1,478(6,3);1,415(0,8);1,333(1,6);1,319(0,8);1,294(1,1);1,284(2,4);1,256(9,2);1,214(0,6);0,901(3,6);0,888(16,0); 0,873(15,7);0,855(3,4);0,830(0,9);0,687(0,6);0,652(5,1);0,640(13,9);0,634(14,5);0,625(11,6);0,615(3,8);0,146(1,7);0,068(0,7);0,000(374,6);-0,065(0,6);-0,149(2,0) |
| Beispiel 1.16: ¹H-NMR(400,0 MHz, CDCl₃): δ= 7,519(0,9);7,329(2,6);7,325(2,8);7,311(3,3);7,306(3,0);7,260(166,3);7,216(1,6);7,212(1,4);7,1 98(2,4);7,195(2,8);7,177(2,1);7,173(1,9);6,996(0,9);6,927(2,5);6,924(2,7);6,908(3,6);6,905(3,9);6,890(2,0);6,887(2,1);6,863(4,4);6,861(4,0);6 ,843(3,9);6,840(3,5);5,634(1,4);5,299(16,0);5,232(1,1);4,323(0,6);4,309(0,9);4,294(1,9);4,283(2,7);4,271(3,8);4,261(2,1);4,250(2,1);4,241(2, 5);4,234(0,9);4,221(0,8);4,213(0,8);4,091(1,0);2,804(1,0);2,796(1,4);2,787(1,3);2,778(1,0);2,288(0,9);2,277(1,4);2,267(2,2);2,255(2,8);2,245( 2,3);2,233(1,3);2,220(0,6);2,053(0,5);2,044(0,6);1,333(0,7);1,284(1,0);1,258(1,5);0,985(0,8);0,970(0,7);0,967(0,6);0,910(0,8);0,903(1,2);0,89 1(4,6);0,876(4,6);0,873(4,4);0,864(1,3);0,858(1,2);0,717(0,6);0,713(0,8);0,710(0,7);0,707(0,7);0,703(0,8);0,700(0,7);0,656(1,6);0,643(3,7);0, 638(3,9);0,634(3,7);0,628(3,2);0,618(1,2);0,008(1,8);0,000(56,6);-0,009(1,9) |
| Beispiel 1.17: ¹H-NMR(400,0 MHz, CDCl₃): δ= 7,295(0,6);7,292(0,7);7,276(1,0);7,275(1,0);7,274(1,0);7,273(1,2);7,272(1,2);7,271(1,2);7,270 (1,2);7,2694(1,2);7,2686(1,3);7,264(50,2);7,186(0,7);7,168(0,5);6,916(0,6);6,913(0,7);6,897(0,9);6,894(1,0);6,876(0,5);6,855(1,1);6,852(0,9); 6,834(0,9);6,832(0,8);5,301(5,2);4,268(0,6);4,261(0,7);4,253(0,7);4,240(0,5);4,232(0,6);3,754(9,5);3,386(16,0);3,268(8,3);3,238(4,3);3,203(9 ,8);3,160(2,3);2,219(0,6);0,008(0,6);0,000(18,8);-0,009(0,6) |
| Beispiel 1.18: ¹H-NMR(400,0 MHz, CDCl₃): δ= 7,275(1,0);7,261(80,3);6,897(1,1);6,889(1,2);6,875(1,1);6,867(1,2);6,801(1,2);6,794(1,0);6,78 0(1,2);6,772(0,9);5,646(0,8);5,300(8,4);5,221(0,6);4,302(1,0);4,294(1,0);4,287(1,1);4,278(0,9);4,261(1,1);4,254(1,4);4,239(1,2);4,232(1,5);4, 225(0,6);4,211(0,7);4,203(0,6);2,806(0,6);2,796(0,8);2,788(0,8);2,779(0,6);2,260(0,6);2,247(0,6);2,236(0,5);2,217(0,6);2,209(0,7);2,202(0,9); 2,195(1,3);2,182(16,0);2,136(0,8);2,044(0,8);1,625(0,7);1,259(0,9);0,910(0,5);0,895(2,7);0,881(2,7);0,878(2,5);0,868(0,5);0,863(0,8);0,662(0 ,9);0,652(1,8);0,649(2,2);0,644(2,2);0,640(2,1);0,635(1,7);0,623(0,6);0,008(1,0);0,000(27,7);-0,009(0,7) |
| Beispiel 1.19: ¹H-NMR(400,0 MHz, CDCl₃): δ= 10,842(0,7);7,519(5,9);7,373(0,6);7,322(0,8);7,314(0,7);7,293(2,6);7,260(1060,1);7,230(1,1); 7,210(2,9);6,996(5,9);6,789(1,3);6,768(1,4);6,759(1,5);6,737(1,6);6,614(0,8);5,298(1,2);5,279(1,1);4,405(0,9);4,389(1,3);4,372(1,0);4,361(0, 8);4,320(0,8);4,308(1,2);4,293(1,0);4,277(1,0);3,210(8,6);3,197(8,5);3,157(1,8);3,144(1,8);2,687(4,6);2,272(1,8);2,258(3,1);2,244(3,4);2,229( 1,8);2,172(15,9);2,007(16,0);1,284(0,7);1,255(0,8);0,146(1,2);0,033(0,7);0,008(13,9);0,000(371,9);-0,009(11,8);-0,050(1,0);-0,150(1,3) |
| Beispiel 1.20: ¹H-NMR(400,0 MHz, CDCl₃): δ= 7,519(1,6);7,309(3,6);7,305(3,8);7,290(3,9);7,286(4,4);7,260(273,2);7,229(0,7);7,213(2,5);7,2 09(2,6);7,192(4,2);7,174(3,2);7,170(2,8);6,996(1,6);6,922(3,5);6,919(3,9);6,903(5,2);6,900(5,8);6,885(2,9);6,882(3,1);6,861(6,1);6,858(5,5);6 ,841(5,3);6,838(4,8);5,571(1,3);5,299(14,8);5,233(1,8);4,321(0,9);4,311(1,2);4,307(1,1);4,293(3,0);4,283(2,9);4,278(3,4);4,267(4,1);4,258(3, 9);4,244(3,3);4,236(3,7);4,230(1,3);4,216(1,4);4,208(1,3);4,130(0,5);4,112(0,6);3,180(12,7);3,154(9,2);3,141(9,1);3,033(16,0);3,020(15,6);2, 315(1,2);2,307(1,3);2,299(0,8);2,286(1,4);2,276(1,8);2,264(3,2);2,252(1,9);2,243(1,9);2,236(1,9);2,230(2,2);2,223(2,3);2,209(1,5);2,196(0,9); 2,187(0,8);2,057(0,6);2,044(2,5);1,606(1,5);1,284(0,6);1,277(0,9);1,259(1,9);1,241(0,9);0,008(3,4);0,000(97,6);-0,008(2,9) |
| Beispiel 1.21: ¹H-NMR(400,0 MHz, CDCl₃): δ= 7,518(0,9);7,259(153,3);7,106(1,6);7,087(3,0);7,046(2,6);7,026(1,6);7,004(1,1);6,995(1,4);5,1 09(0,6);4,492(0,7);4,474(2,2);4,456(2,3);4,439(0,9);3,106(1,2);3,087(1,2);3,067(1,4);3,048(1,4);2,565(0,8);2,543(1,0);2,527(0,7);2,504(0,9);2 ,342(0,6);2,323(0,6);2,299(16,0);1,467(9,4);1,449(20,0);1,431(9,2);1,278(5,8);1,262(6,9);0,899(1,1);0,882(4,3);0,864(1,5);0,008(1,7);0,000(6 3,3);-0,009(1,9) |
| Beispiel 1.22: ¹H-NMR(400,0 MHz, CDCl₃): δ= 7,518(4,0);7,292(4,3);7,281(3,7);7,259(719,4);7,251(2,6);7,225(2,2);7,221(3,0);7,207(9,6);7,2 03(9,7);7,189(14,1);7,185(16,8);7,180(11,7);7,167(8,0);7,162(9,9);7,148(3,4);7,137(10,9);7,133(10,3);7,118(6,7);6,995(3,9);5,981(1,4);5,577 (0,7);5,271(3,4);5,152(2,9);2,888(2,3);2,862(3,6);2,846(6,5);2,831(3,7);2,822(4,3);2,806(7,1);2,790(4,2);2,765(2,3);2,748(1,4);2,625(10,0);2, 091(2,0);1,895(6,7);1,878(6,5);1,590(9,5);1,265(7,8);0,899(4,3);0,882(16,0);0,864(5,8);0,146(0,8);0,008(7,9);0,000(270,1);-0,009(7,5);-0,150(0,8) |
| Beispiel 1.23: ¹H-NMR(400,6 MHz, CDCl₃): δ= 7,107(6,9);6,888(3,3);6,869(1,1);6,860(1,4);6,850(1,7);5,142(1,0);2,876(0,6);2,858(0,7);2,839 (0,7);2,457(1,8);2,387(0,7);2,364(0,8);2,349(0,8);2,326(0,8);2,169(2,6);2,154(16,0);1,160(1,6);1,143(1,7);1,132(0,9);1,114(13,3);1,098(13,0) |
| Beispiel 1.24: ¹H-NMR(400,6 MHz, CDCl₃): δ= 7,315(5,8);7,299(7,3);7,259(51,8);7,247(23,0);7,231(13,1);7,223(7,8);7,213(8,2);7,205(5,2);7, 192(2,8);6,148(1,7);5,847(0,7);5,464(2,0);5,318(7,7);5,297(5,3);5,176(0,7);3,044(3,0);3,035(3,5);3,023(3,5);3,013(3,9);3,005(6,2);2,995(6,6); 2,983(6,5);2,974(6,4);2,928(3,4);2,908(6,9);2,888(5,4);2,868(3,6);2,848(2,1);2,634(2,2);2,612(4,9);2,594(1,5);2,044(1,5);1,893(2,8);1,645(16 ,0);1,283(0,7);1,276(0,7);1,258(1,8);1,240(0,5);0,882(0,7);0,008(5,1);0,000(121,0);-0,008(3,6);-0,022(0,6) |
| Beispiel 1.25: ¹H-NMR(400,0 MHz, CDCl₃): δ= 7,258(59,3);7,111(1,1);7,092(2,4);7,065(2,5);7,063(2,7);7,062(2,6);7,044(1,2);7,043(1,3);6,99 4(0,6);6,982(2,0);6,176(0,5);5,395(1,5);5,104(0,6);5,083(1,1);5,061(0,6);3,109(1,2);3,089(1,2);3,070(1,4);3,051(1,4);2,568(0,7);2,545(0,8);2, 530(0,6);2,508(0,7);2,368(0,6);2,350(0,7);2,331(0,7);2,310(16,0);1,577(0,6);1,271(6,4);1,254(5,4);0,008(0,8);0,000(24,7);-0,009(0,7) |
| Beispiel 1.26: ¹H-NMR(400,0 MHz, CDCl₃): δ= 7,518(1,1);7,259(204,8);7,118(1,7);7,098(3,2);7,057(2,9);7,038(1,6);7,013(1,2);6,995(1,2);5,1 36(0,7);3,114(1,2);3,095(1,2);3,075(1,4);3,057(1,4);2,728(7,5);2,574(0,6);2,554(0,7);2,537(0,6);2,514(0,6);2,347(0,7);2,307(16,0);1,547(1,6); 1,427(0,6);1,284(3,0);1,268(2,7);0,882(0,5);0,008(2,6);0,000(84,5);-0,009(2,3) |
| Beispiel 1.27: ¹H-NMR(400,0 MHz, d₆-DMSO): δ= 8,520(1,9);8,502(2,0);8,063(1,7);7,905(1,9);7,755(0,7);7,271(5,9);7,255(16,0);7,235(9,8);7 ,216(10,8);7,213(8,6);7,198(5,6);7,195(4,3);7,178(7,1);7,175(7,1);7,160(8,0);7,141(2,7);5,462(1,8);5,444(1,8);3,342(1,6);3,029(1,8);3,022(2, 1);3,008(2,1);3,000(2,4);2,990(3,0);2,982(3,2);2,969(3,0);2,961(2,9);2,856(1,8);2,836(3,7);2,816(3,1);2,796(2,5);2,776(1,4);2,675(1,0);2,670( 1,4);2,666(1,1);2,524(3,8);2,519(5,4);2,510(71,3);2,506(157,7);2,501(222,6);2,497(158,6);2,492(74,2);2,456(3,1);2,451(3,0);2,333(1,0);2,328 (1,4);2,323(1,0);2,319(0,5);2,042(2,0);2,020(5,6);2,010(2,3);1,999(5,5);1,989(5,0);1,978(2,3);1,968(4,9);1,946(1,7);0,008(1,7);0,006(0,6);0,0 00(60,8);-0,007(0,9);-0,009(2,0) |
| Beispiel 1.28: ¹H-NMR(400,0 MHz, d₆-DMSO): δ= 8,410(1,9);8,391(1,9);7,933(1,7);7,906(2,2);7,903(3,8);7,898(2,7);7,894(2,4);7,890(1,8);7, 887(2,0);7,820(1,6);7,721(1,0);7,718(1,1);7,699(1,2);7,696(1,3);7,661(1,0);7,568(1,6);7,548(2,8);7,528(1,2);7,177(4,2);7,171(4,8);7,159(9,3); 7,141(11,0);7,136(11,4);7,130(10,8);7,113(16,0);7,094(5,5);5,523(0,6);5,153(1,9);3,316(7,2);2,809(1,0);2,787(2,7);2,767(3,8);2,756(4,5);2,7 42(4,5);2,701(1,4);2,675(1,7);2,670(2,2);2,665(1,7);2,563(0,7);2,554(0,6);2,523(6,1);2,519(8,6);2,510(117,4);2,506(257,1);2,501(359,2);2,49 6(250,6);2,492(112,6);2,332(1,4);2,328(2,0);2,323(1,6);1,990(4,7);1,977(4,8);1,964(5,1);1,832(2,5);1,813(2,6);1,789(1,5);1,743(2,1);1,235(1, 8);0,008(2,4);0,000(90,6);-0,009(2,5) |
| Beispiel 1.29: ¹H-NMR(400,0 MHz, CDCl₃): δ= 8,466(1,0);7,518(1,8);7,326(2,3);7,310(2,4);7,292(2,0);7,291(1,6);7,282(11,8);7,279(13,7);7,2 74(9,9);7,273(9,4);7,272(9,9);7,2684(12,6);7,2677(12,6);7,266(11,0);7,265(11,3);7,2644(11,9);7,2637(10,1);7,263(10,2);7,259(339,4);7,2554 (9,1);7,2545(7,1);7,254(6,1);7,253(5,5);7,252(5,4);7,2512(5,7);7,2505(5,8);7,250(5,8);7,248(4,6);7,247(4,8);7,246(4,4);7,242(5,3);7,234(3,1); 7,228(3,9);7,226(4,0);7,210(2,5);7,202(1,3);6,995(1,9);6,093(1,0);5,509(1,2);5,434(1,1);5,340(0,7);4,301(0,5);4,130(0,5);3,312(7,6);3,083(1, 5);3,074(1,6);3,061(1,7);3,052(1,8);3,043(2,9);3,034(3,1);3,022(3,1);3,012(3,0);3,006(2,4);2,957(1,7);2,937(3,4);2,918(2,6);2,897(2,0);2,877( 1,3);2,720(16,0);2,708(12,2);2,655(1,2);2,043(2,4);1,988(1,8);1,967(3,2);1,956(2,0);1,948(3,2);1,935(3,1);1,928(1,8);1,916(2,8);1,895(1,4);1, 591(1,0);1,441(0,6);1,424(1,1);1,406(0,6);1,304(0,9);1,276(2,1);1,264(3,8);1,258(3,6);1,240(1,2);0,899(2,1);0,882(8,3);0,864(2,9);0,008(4,7); 0,006(1,5);0,0054(1,6);0,0046(2,1);0,004(2,8);0,003(4,2);0,002(6,3);0,000(147,9);-0,003(7,6);-0,0035(5,2);-0,0044(3,4);-0,005(2,5);-0,006(2,2);-0,007(2,0);-0,009(5,0);-0,011(1,3);-0,012(1,3);-0,013(1,0);-0,015(0,7) |
| Beispiel 1.30: ¹H-NMR(400,0 MHz, CDCl₃): δ= 8,467(1,2);7,518(1,4);7,283(2,7);7,270(2,2);7,259(259,2);7,228(1,7);7,224(2,0);7,210(5,9);7,2 06(5,9);7,192(8,5);7,187(9,3);7,182(6,3);7,164(5,8);7,143(7,6);7,124(4,2);6,995(1,5);6,059(1,2);5,661(0,6);5,438(1,2);5,197(1,5);3,312(6,4);3 ,006(1,6);2,911(0,8);2,896(1,4);2,869(2,1);2,852(3,8);2,839(2,7);2,830(2,9);2,814(4,5);2,798(2,7);2,787(1,2);2,771(1,7);2,715(16,0);2,708(13 ,8);2,095(1,4);2,043(1,4);1,904(4,6);1,887(4,5);1,579(1,1);1,424(0,8);1,265(3,2);1,240(0,8);1,237(0,8);1,220(0,6);0,899(1,6);0,882(5,7);0,864 (2,2);0,008(3,5);0,000(103,6);-0,009(3,7) |
| Beispiel 1.31: ¹H-NMR(400,0 MHz, CDCl₃): δ= 7,259(49,9);7,156(1,5);7,137(2,7);7,117(1,2);7,085(2,5);7,066(1,5);5,297(1,8);5,258(1,2);2,97 6(0,6);2,968(0,9);2,959(0,9);2,946(0,9);2,937(0,9);2,888(0,5);2,868(1,0);2,848(0,8);2,828(0,6);2,333(16,0);1,911(0,8);1,892(0,9);1,880(0,8);1 ,860(0,7);1,258(0,6);0,008(0,8);0,000(19,9);-0,009(0,7) |
| Beispiel 1.32: ¹H-NMR(400,0 MHz, CDCl₃): δ= 8,057(1,0);8,053(1,6);8,048(1,1);7,950(1,0);7,947(1,3);7,944(1,0);7,931(1,2);7,928(1,6);7,925 (1,3);7,885(1,3);7,518(2,6);7,475(0,7);7,473(0,7);7,470(0,8);7,467(0,6);7,455(0,9);7,453(0,9);7,450(1,0);7,447(0,9);7,358(1,7);7,338(2,6);7,3 18(1,8);7,310(1,9);7,292(2,9);7,283(3,1);7,276(3,4);7,274(3,3);7,273(3,4);7,2723(3,4);7,2715(3,4);7,271(3,4);7,270(3,5);7,269(3,6);7,2683(3, 8);7,2675(3,9);7,266(4,7);7,259(444,5);7,252(2,9);7,251(1,5);7,250(1,3);7,249(1,2);7,248(1,1);7,2473(1,0);7,2465(0,8);7,246(0,8);7,245(0,7); 7,244(0,6);7,2433(0,6);7,2425(0,5);7,242(0,5);7,241(0,6);7,239(0,6);7,216(2,3);7,211(3,0);7,197(7,9);7,193(8,0);7,179(10,7);7,174(9,8);7,17 2(6,6);7,167(8,8);7,153(6,2);7,149(8,2);7,130(11,3);7,111(5,5);7,109(5,4);6,995(2,5);6,082(1,5);5,368(1,4);5,182(2,0);4,481(3,6);4,463(10,3); 4,446(10,6);4,428(4,0);4,130(0,8);4,112(0,7);3,425(0,5);3,162(1,1);3,144(1,1);2,991(16,0);2,903(1,0);2,887(1,9);2,872(1,2);2,861(2,8);2,845( 5,1);2,830(2,7);2,819(3,4);2,804(5,7);2,788(3,4);2,778(1,3);2,761(2,0);2,745(1,1);2,678(15,8);2,086(1,9);2,043(4,1);1,894(5,3);1,878(5,7);1,8 61(3,3);1,600(0,6);1,460(44,0);1,442(95,1);1,425(43,9);1,372(2,0);1,363(3,3);1,354(4,8);1,347(5,1);1,336(3,1);1,332(2,9);1,304(1,4);1,281(2, 1);1,276(3,1);1,264(5,8);1,258(5,4);1,240(1,6);0,899(3,1);0,882(11,5);0,864(4,3);0,146(0,6);0,008(5,5);0,000(197,3);-0,009(5,9);-0,150(0,6) |
| Beispiel 1.33: ¹H-NMR(400,0 MHz, CDCl₃): δ= 7,876(0,7);7,518(1,4);7,323(1,7);7,309(1,9);7,295(0,9);7,2922(1,1);7,2915(1,1);7,290(0,8);7,2 89(0,9);7,2883(0,9);7,2875(0,9);7,287(0,9);7,286(0,9);7,285(0,9);7,2843(0,9);7,2835(0,9);7,283(0,9);7,282(1,1);7,281(1,2);7,280(1,5);7,279( 1,6);7,278(1,6);7,277(1,7);7,2764(1,6);7,2756(1,6);7,275(1,6);7,274(1,7);7,273(2,0);7,269(11,4);7,266(13,6);7,265(7,8);7,260(242,6);7,256(1 6,7);7,253(8,5);7,249(1,5);7,248(1,2);7,2474(1,0);7,2466(0,9);7,246(0,7);7,245(0,7);7,244(0,6);7,2434(0,6);7,2426(0,6);7,242(0,7);7,241(0,7) ;7,237(2,5);7,235(1,7);7,233(1,7);7,228(4,0);7,226(2,9);7,220(2,3);7,214(3,0);7,209(3,3);7,208(2,7);7,206(2,4);7,195(2,2);7,188(1,3);6,995(1, 4);5,984(0,8);5,484(0,9);5,364(0,8);4,484(3,0);4,466(9,0);4,448(9,2);4,431(3,2);3,068(1,3);3,058(1,5);3,046(1,5);3,036(1,6);3,028(2,6);3,018( 2,7);3,006(2,7);2,997(2,8);2,991(15,6);2,945(1,6);2,924(3,4);2,904(2,7);2,885(1,9);2,864(1,2);2,678(16,0);2,644(0,9);1,965(1,7);1,947(2,2);1, 945(2,9);1,933(1,8);1,926(3,0);1,923(2,3);1,915(2,2);1,912(2,8);1,905(1,9);1,894(2,7);1,873(1,5);1,621(0,7);1,603(0,9);1,586(0,8);1,464(28,5 );1,446(61,7);1,428(28,4);1,285(0,7);1,264(1,2);1,237(1,0);1,220(1,4);1,176(1,4);1,160(1,4);0,899(0,6);0,882(2,5);0,864(0,9);0,010(0,6);0,00 8(3,3);0,0064(1,2);0,0055(1,3);0,005(1,5);0,004(2,0);0,000(104,1);-0,005(1,9);-0,006(1,4);-0,007(1,0);-0,009(3,0) |
| Beispiel 1.34: ¹H-NMR(400,0 MHz, CDCl₃): δ= 7,518(0,7);7,333(0,7);7,322(0,7);7,317(0,8);7,271(0,5);7,270(0,6);7,269(0,7);7,2684(0,7);7,26 76(0,7);7,267(0,9);7,266(1,1);7,265(1,3);7,2644(1,6);7,2635(2,1);7,259(115,2);7,254(1,1);7,253(0,9);7,252(0,8);7,2514(0,7);7,2506(0,5);7,21 6(0,5);7,202(1,5);7,198(1,6);7,184(2,9);7,178(2,7);7,164(1,3);7,159(1,5);7,145(0,5);7,134(1,8);7,129(1,6);7,115(1,0);7,113(1,0);6,995(0,7);5, 298(5,9);2,992(16,0);2,979(15,9);2,861(0,6);2,844(1,0);2,822(0,7);2,806(1,1);2,791(0,7);1,912(0,9);1,896(1,3);1,881(1,2);1,865(0,6);1,557(0, 6);1,333(0,6);1,284(1,0);1,256(1,6);0,008(1,6);0,0063(0,6);0,0055(0,6);0,005(0,7);0,004(1,0);0,000(51,2);-0,003(2,7);-0,004(1,1);-0,005(0,8);-0,006(0,6);-0,007(0,5);-0,009(1,6) |
| Beispiel 1.35: ¹H-NMR(400,0 MHz, CDCl₃): δ= 7,343(1,1);7,325(1,4);7,280(0,6);7,272(3,6);7,269(4,3);7,260(51,6);7,240(0,9);7,235(1,8);7,22 7(0,9);7,221(1,0);7,217(1,1);7,212(0,8);7,202(0,7);5,298(4,8);3,055(0,5);3,042(0,6);3,033(0,6);3,024(1,0);3,015(1,1);3,002(1,2);2,991(16,0);2 ,978(15,5);2,944(0,7);2,924(1,4);2,903(1,1);2,884(0,7);1,935(1,0);1,924(0,5);1,916(1,0);1,903(0,9);1,884(0,9);1,284(0,6);1,256(0,9);0,008(0, 7);0,000(21,5);-0,009(0,7) |
| Beispiel 1.36: ¹H-NMR(400,0 MHz, d₆-DMSO): δ= 8,374(0,9);8,353(0,9);7,904(0,9);7,900(0,8);7,895(0,6);7,888(0,6);7,841(1,0);7,787(1,0);7, 698(0,6);7,662(0,6);7,548(0,6);7,096(2,5);7,077(3,5);7,000(2,7);6,981(1,9);6,907(3,0);5,017(0,9);3,330(2,2);3,015(1,4);2,995(1,4);2,970(0,6); 2,506(26,9);2,502(33,0);2,498(24,8);2,454(2,4);2,440(2,3);2,232(16,0);1,181(5,9);1,169(4,8);0,000(0,8) |
| Beispiel 1.37: ¹H-NMR(400,0 MHz, CDCl₃): δ= 7,518(2,8);7,294(3,2);7,259(523,1);7,233(16,3);7,230(14,8);7,215(20,4);7,211(17,4);7,197(23 ,8);7,193(17,0);7,180(16,5);7,161(15,7);7,143(26,4);7,125(13,5);7,123(12,9);6,995(2,8);6,257(4,7);6,238(4,7);5,612(1,9);5,414(13,1);5,298(9 ,5);5,153(5,5);5,133(5,6);3,083(0,8);2,907(1,9);2,891(3,4);2,864(5,5);2,848(10,0);2,823(8,1);2,808(12,7);2,792(7,1);2,781(2,6);2,766(4,2);2,7 49(2,4);2,161(1,7);2,112(2,6);2,090(4,4);2,078(5,2);1,893(14,7);1,876(16,0);1,861(7,9);1,842(3,3);1,587(3,8);1,333(0,7);1,284(1,2);1,257(2,1 );0,146(0,6);0,008(6,4);0,000(209,1);-0,007(1,6);-0,009(5,8);-0,149(0,5) |
| Beispiel 1.38: ¹H-NMR(400,0 MHz, CDCl₃): δ= 7,518(4,5);7,309(6,9);7,296(14,7);7,283(66,7);7,273(100,6);7,259(824,4);7,243(23,6);7,231(2 3,4);7,225(14,3);7,214(12,9);7,203(6,1);6,995(4,3);6,261(6,4);5,896(2,6);5,491(4,9);5,471(11,4);5,452(12,5);5,416(16,0);5,337(6,1);5,298(3, 6);3,972(1,2);3,083(4,6);3,074(5,3);3,061(5,5);3,052(6,4);3,043(9,5);3,034(9,8);3,021(9,9);3,012(9,7);2,954(7,6);2,933(15,0);2,913(11,7);2,8 94(8,5);2,873(4,7);2,735(2,1);2,672(3,2);2,653(5,2);2,643(6,3);2,621(6,3);2,004(7,3);1,981(2,7);1,960(6,9);1,940(8,6);1,927(8,6);1,908(7,7);1 ,887(3,6);1,578(4,7);1,370(1,7);1,333(1,3);1,286(2,9);1,255(4,7);0,880(1,0);0,146(1,1);0,008(9,9);0,000(321,3);-0,009(8,9);-0,150(1,1) |
| Beispiel 1.39: ¹H-NMR(400,0 MHz, CDCl₃): δ= 7,259(78,5);7,109(1,0);7,090(1,8);7,046(1,8);7,029(1,5);4,224(0,7);4,220(0,6);4,208(0,6);4,20 4(0,7);4,187(0,5);3,098(0,7);3,079(0,8);3,060(0,8);3,041(0,8);2,544(0,6);2,504(0,5);2,329(0,6);2,305(9,9);2,292(0,6);2,044(0,6);1,553(0,6);1, 290(2,8);1,269(16,0);1,258(3,5);1,253(13,3);0,899(1,6);0,882(6,0);0,864(2,2);0,008(1,0);0,000(32,0);-0,009(0,9) |
| Beispiel 1.40: ¹H-NMR(400,0 MHz, CDCl₃): δ= 7,260(52,4);7,094(1,8);7,075(3,6);7,048(2,9);7,039(3,4);7,020(1,4);4,130(0,8);4,112(0,8);3,36 4(5,3);3,139(6,3);3,077(1,2);3,057(1,3);3,038(1,5);3,019(1,5);2,555(1,0);2,532(1,2);2,516(0,8);2,494(1,0);2,305(16,0);2,043(4,0);1,290(7,4);1 ,273(7,5);1,258(3,2);1,240(1,3);0,899(0,7);0,882(2,6);0,864(1,0);0,008(0,6);0,000(21,6);-0,009(0,6) |
| Beispiel 1.41: ¹H-NMR(400,0 MHz, CDCl₃): δ= 7,259(37,2);7,101(1,4);7,082(2,8);7,043(3,5);7,026(2,3);5,287(0,5);3,087(1,1);3,068(1,2);3,04 8(1,3);3,029(1,4);2,990(13,9);2,977(13,8);2,559(0,8);2,537(1,0);2,521(0,7);2,499(0,8);2,302(16,0);2,283(0,6);2,043(1,8);1,283(4,8);1,276(3,4 );1,266(6,3);1,258(3,1);1,240(0,8);0,899(1,3);0,882(4,3);0,864(1,7);0,000(14,2) |
| Beispiel 1.42: ¹H-NMR(400,0 MHz, CDCl₃): δ= 8,084(0,6);7,260(72,0);7,106(1,3);7,086(2,5);7,044(2,2);7,025(2,0);6,995(0,5);4,202(1,9);4,18 4(6,1);4,166(6,2);4,148(2,1);3,739(1,3);3,723(3,8);3,707(4,0);3,691(1,4);3,095(0,9);3,075(0,9);3,056(1,0);3,036(1,1);2,644(2,3);2,628(4,5);2, 612(2,2);2,564(0,6);2,541(0,7);2,525(0,5);2,503(0,6);2,323(0,7);2,303(12,8);2,286(0,6);2,043(0,7);1,562(0,6);1,291(8,3);1,273(16,0);1,255(7, 3);1,240(0,5);0,899(0,7);0,882(2,5);0,864(0,9);0,008(0,9);0,000(29,9);-0,009(0,9) |
| Beispiel 1.43: ¹H-NMR(400,0 MHz, CDCl₃): δ= 7,259(35,9);7,097(1,1);7,077(2,2);7,039(2,6);7,023(1,8);4,278(2,0);4,260(6,2);4,242(6,3);4,22 5(2,1);4,196(6,1);4,181(6,1);3,091(0,9);3,072(0,9);3,053(1,0);3,034(1,0);2,562(0,6);2,539(0,7);2,501(0,6);2,305(12,3);2,042(1,2);1,323(7,7);1 ,306(16,0);1,288(9,9);1,275(2,8);1,268(3,7);1,258(2,1);1,240(0,6);0,899(0,9);0,882(3,3);0,864(1,2);0,000(14,5) |
| Beispiel 1.44: ¹H-NMR(400,0 MHz, CDCl₃): δ= 7,260(30,5);7,099(1,0);7,079(2,2);7,046(2,7);7,030(2,0);4,130(0,9);4,112(0,9);3,083(0,8);3,06 4(0,9);3,044(1,0);3,025(1,1);3,004(16,0);2,999(5,0);2,554(0,7);2,532(0,8);2,516(0,6);2,493(0,8);2,312(12,9);2,292(0,6);2,043(4,5);1,287(4,3); 1,279(3,9);1,276(2,9);1,270(4,5);1,262(4,0);1,258(3,6);1,240(1,4);0,000(13,1) |
| Beispiel 1.45: ¹H-NMR(400,0 MHz, CDCl₃): δ= 7,518(1,0);7,271(1,1);7,269(1,3);7,2664(2,0);7,2656(2,3);7,265(2,7);7,259(176,5);7,253(0,8); 7,120(1,7);7,101(3,4);7,065(3,0);7,046(1,7);7,003(1,7);6,995(1,6);6,110(0,5);5,098(0,9);5,076(0,5);4,130(1,4);4,113(1,5);3,340(7,2);3,117(1, 2);3,098(1,2);3,078(1,4);3,059(1,4);2,574(0,7);2,552(0,8);2,536(0,6);2,515(0,7);2,319(16,0);2,043(6,6);1,551(0,6);1,281(4,5);1,276(4,4);1,26 5(4,8);1,258(5,4);1,241(2,1);0,899(0,7);0,882(2,7);0,864(1,0);0,008(2,3);0,000(71,4);-0,009(2,0) |
| Beispiel 1.46: ¹H-NMR(400,0 MHz, CDCl₃): δ= 8,176(3,1);7,518(3,6);7,259(655,3);7,210(1,4);6,995(3,8);6,886(5,1);6,879(6,7);6,865(9,2);6,8 58(12,7);6,844(5,1);6,833(12,4);6,827(8,2);6,809(10,8);6,803(8,2);5,886(1,6);5,298(15,7);5,255(4,8);2,871(2,9);2,844(4,5);2,829(8,1);2,813( 4,4);2,802(4,7);2,786(8,5);2,769(5,1);2,743(2,9);2,727(1,9);2,487(16,0);2,060(2,8);1,883(9,0);1,548(18,4);1,333(1,2);1,284(2,2);1,256(4,8);0, 008(8,1);0,000(266,2);-0,009(8,8) |
| Beispiel 1.47: ¹H-NMR(400,0 MHz, CDCl₃): δ= 7,518(2,3);7,259(432,0);7,246(6,2);7,231(6,9);7,227(6,1);7,210(11,4);7,192(7,5);7,189(6,6);6, 995(2,4);6,935(8,7);6,932(10,0);6,916(13,9);6,913(15,6);6,897(7,2);6,894(7,8);6,876(16,0);6,873(14,9);6,855(14,1);6,853(13,0);5,929(0,9);5, 298(9,1);5,268(4,4);4,325(2,6);4,315(3,3);4,309(2,9);4,296(6,4);4,287(6,4);4,281(7,1);4,272(5,8);4,255(6,4);4,247(7,2);4,232(6,5);4,225(8,3); 4,219(3,6);4,204(3,4);4,196(2,9);2,842(1,1);2,625(5,8);2,273(2,4);2,182(2,6);1,566(3,1);1,370(3,6);1,333(1,3);1,286(5,5);1,256(10,0);0,880(1 ,6);0,008(5,5);0,000(175,3);-0,009(5,1) |
| Beispiel 1.48: ¹H-NMR(400,6 MHz, CDCl₃): δ= 7,366(4,1);7,348(4,3);7,288(4,3);7,285(4,2);7,268(9,0);7,266(8,9);7,265(8,7);7,261(30,3);7,24 9(4,9);7,246(4,5);7,148(0,5);7,127(0,6);7,088(0,6);6,957(7,3);6,955(7,4);6,938(12,8);6,936(12,9);6,920(6,1);6,918(6,0);6,886(8,9);6,866(7,9); 6,220(0,5);5,589(0,7);5,352(5,0);5,298(16,0);4,768(1,5);4,420(2,0);2,614(2,3);2,513(0,9);2,445(0,8);1,372(3,7);1,333(0,7);1,286(5,3);1,255(7 ,8);0,880(1,2);0,863(0,6);0,000(15,2);-0,009(0,6) |
| Beispiel 1.49: ¹H-NMR(400,0 MHz, CDCl₃): δ= 7,736(0,5);7,519(0,9);7,260(182,3);7,017(0,6);7,009(0,6);6,996(1,3);6,990(0,8);6,980(1,5);6,9 68(1,6);6,958(1,3);6,939(1,9);6,932(1,2);6,918(2,6);6,910(2,0);6,898(2,2);6,897(2,3);6,890(1,8);6,825(4,5);6,813(4,5);6,803(2,9);6,791(2,7);5 ,299(16,0);4,301(0,7);4,291(0,8);4,284(0,7);4,272(2,1);4,263(2,0);4,256(2,3);4,246(2,8);4,237(1,6);4,224(1,4);4,216(1,7);4,196(0,5);4,188(0, 5);2,487(4,1);2,485(4,2);2,265(0,7);2,146(0,9);1,286(0,6);1,257(1,0);0,008(2,2);0,000(69,0);-0,009(1,9) |
| Beispiel 1.50: ¹H-NMR(400,0 MHz, CDCl₃): δ= 7,258(72,2); 7,160(1,7); 7,141(2,9); 7,126(1,1); 7,086(2,5); 7,067(1,6); 5,297(3,7); 5,256(0,9); 3,968(0,8); 3,958 (0,8); 3,404(3,4); 2,990(0,5); 2,980(0,6); 2,972(0,9); 2,963(0,9); 2,951(0,9); 2,941(0,9); 2,890(0,5); 2,870(1,1); 2,850(0,9); 2,831(0,6); 2,333(16,0); 1,916(1,1); 1,905(0,6); 1,897(1,1); 1,884(1,0); 1,876(0,6); 1,865(1,0); 0,008(1,0); 0,000(30,3); -0,008(1,0) |
| Beispiel 1.51: ¹H-NMR(400,6 MHz, MeOD): δ= 7,082(2,0);7,063(3,3);7,014(2,8);6,994(2,0);6,982(3,4);5,178(0,5);4,903(0,6);4,877(77,9);3,34 0(5,9);3,336(11,4);3,332(16,5);3,328(11,3);3,323(5,6);3,122(20,8);3,093(1,2);3,074(1,3);3,055(1,3);3,036(1,3);2,532(0,8);2,509(1,1);2,494(0, 7);2,471(1,0);2,388(0,9);2,369(1,1);2,352(0,8);2,279(16,0);1,380(1,1);1,319(1,8);1,309(2,6);1,280(8,5);1,263(8,2) |
| Beispiel 1.52: ¹H-NMR(400,0 MHz, CDCl₃): δ= 7,258(58,1);7,099(0,8);7,020(10,3);7,017(9,6);5,249(0,8);2,808(0,7);2,792(1,2);2,770(0,8);2,7 54(1,2);2,737(0,7);2,624(0,5);2,282(16,0);1,867(1,3);1,851(1,2);1,564(0,8);1,258(0,9);0,008(0,8);0,000(23,8);-0,009(0,6) |
| Beispiel 1.53: ¹H-NMR(400,6 MHz, MeOD): δ= 6,987(2,0);6,968(3,4);6,917(2,7);6,892(3,6);4,803(0,6);4,780(59,4);3,244(7,3);3,240(14,3);3,2 35(21,4);3,231(17,3);3,227(7,8);3,218(2,4);3,212(3,2);3,206(2,3);3,192(3,3);2,998(1,2);2,979(1,3);2,960(1,5);2,941(1,4);2,437(0,8);2,414(1,1 );2,400(0,7);2,377(1,0);2,312(0,6);2,293(0,8);2,290(0,8);2,272(1,3);2,255(0,9);2,238(0,5);2,185(16,0);1,786(1,8);1,780(0,6);1,773(1,2);1,767( 2,8);1,761(1,2);1,754(1,3);1,748(2,9);1,742(1,2);1,736(0,6);1,729(1,8);1,224(0,9);1,214(1,7);1,187(10,9);1,170(10,7);1,008(0,5);0,991(6,6);0, 972(13,0);0,953(5,7) |
| Beispiel 1.54: ¹H-NMR(400,6 MHz, MeOD): δ= 8,002(4,1);7,981(4,6);7,963(0,9);7,488(1,8);7,481(3,6);7,466(2,0);7,460(3,3);7,071(2,2);7,052 (3,6);7,003(2,8);6,983(1,7);6,960(3,3);5,508(2,9);4,877(66,3);3,340(6,6);3,336(13,0);3,332(18,9);3,328(12,8);3,323(6,3);3,079(1,2);3,060(1,4 );3,041(1,4);3,022(1,4);2,519(0,8);2,495(1,1);2,481(0,7);2,458(1,0);2,391(0,6);2,370(0,9);2,351(1,2);2,334(0,9);2,284(0,7);2,265(16,0);1,318( 0,9);1,309(1,3);1,263(9,9);1,247(9,6) |
| Beispiel 1.55: ¹H-NMR(400,6 MHz, MeOD): δ= 8,035(2,9);8,018(2,9);7,990(1,0);7,987(1,1);7,983(0,6);7,969(1,5);7,966(1,0);7,661(0,7);7,643 (0,7);7,614(1,0);7,599(0,7);7,595(1,3);7,577(0,6);7,515(1,4);7,495(4,1);7,476(2,9);7,461(0,9);7,076(2,1);7,057(3,5);7,006(2,7);6,986(1,7);6,9 68(3,5);5,510(1,3);4,899(0,8);4,877(97,6);3,351(0,6);3,347(0,8);3,340(12,9);3,336(25,8);3,332(37,5);3,328(25,2);3,323(12,6);3,085(1,2);3,06 6(1,3);3,047(1,4);3,028(1,4);2,524(0,9);2,501(1,1);2,486(0,7);2,463(1,1);2,394(0,6);2,373(0,9);2,355(1,2);2,338(0,9);2,271(16,0);1,381(0,8);1 ,320(1,3);1,311(1,8);1,270(11,6);1,253(11,3) |
| Beispiel 1.56: ¹H-NMR(400,6 MHz, MeOD): δ= 8,336(4,2);8,331(1,7);8,318(2,6);8,314(6,7);8,244(7,1);8,239(2,1);8,227(1,7);8,222(4,3);8,215 (0,8);7,072(2,0);7,053(3,4);7,006(2,7);7,004(2,7);6,985(2,5);6,958(3,1);5,509(16,0);4,878(103,7);4,857(0,8);3,340(8,7);3,336(16,9);3,332(24, 2);3,328(16,7);3,324(8,2);3,081(1,1);3,062(1,3);3,055(0,6);3,043(1,4);3,024(1,4);2,520(0,8);2,497(1,1);2,483(0,7);2,459(0,9);2,395(0,7);2,37 3(1,0);2,355(1,4);2,338(1,0);2,315(0,6);2,277(4,8);2,265(14,9);1,380(0,8);1,361(0,5);1,327(0,9);1,318(1,5);1,308(2,7);1,282(3,0);1,264(10,3); 1,247(8,7) |
| Beispiel 1.57: ¹H-NMR(400,6 MHz, MeOD): δ= 7,088(1,9);7,070(3,1);7,017(2,5);6,996(3,7);5,510(4,5);4,904(1,7);4,878(144,6);3,340(10,9);3, 336(21,4);3,332(31,7);3,328(21,9);3,323(10,9);3,102(1,1);3,084(1,2);3,065(1,3);3,047(4,8);2,538(0,8);2,515(1,1);2,500(0,7);2,477(1,0);2,422 (0,6);2,401(0,9);2,383(1,2);2,370(0,9);2,366(1,0);2,286(16,0);1,906(0,7);1,848(0,6);1,400(0,6);1,380(1,2);1,361(0,6);1,329(1,5);1,320(2,5);1, 310(5,3);1,286(10,6);1,269(10,6);1,200(0,5);1,034(0,8);1,016(0,6);0,922(1,0);0,904(0,5) |
| Beispiel 1.58: ¹H-NMR(400,0 MHz, CDCl₃): δ= 7,258(53,4);7,122(3,0);7,111(2,9);7,061(2,3);7,041(1,3);5,323(1,8);4,130(0,6);4,112(0,6);2,93 0(0,8);2,921(0,9);2,908(0,8);2,900(0,9);2,859(0,6);2,839(1,3);2,819(1,0);2,800(0,6);2,652(0,6);2,642(0,5);2,631(0,6);2,323(16,0);2,043(2,7);1 ,856(1,1);1,845(0,5);1,837(1,0);1,824(1,0);1,805(0,9);1,586(0,7);1,276(1,4);1,265(1,6);1,258(2,5);1,240(0,9);0,899(0,9);0,882(3,3);0,864(1,2) ;0,008(0,7);0,000(22,7);-0,009(0,7) |
| Beispiel 1.59: ¹H-NMR(400,0 MHz, CDCl₃): δ= 7,518(2,0);7,332(4,5);7,315(6,2);7,291(0,6);7,289(0,7);7,283(0,8);7,259(368,1);7,254(17,7);7, 247(11,0);7,244(17,6);7,241(10,6);7,235(1,7);7,233(2,1);7,224(6,4);7,223(4,8);7,212(4,5);7,208(2,9);7,206(3,9);7,204(2,7);7,202(2,2);7,197( 1,7);7,193(3,3);7,184(1,5);6,995(1,9);5,533(1,3);5,292(4,2);4,148(1,1);4,130(3,3);4,112(3,4);4,095(1,2);3,038(1,6);3,029(1,9);3,016(1,8);3,00 7(2,0);2,998(3,2);2,989(3,4);2,977(3,3);2,967(3,3);2,921(2,5);2,900(5,2);2,880(4,0);2,861(2,7);2,840(1,7);2,681(1,4);2,671(1,7);2,651(1,7);2, 641(1,4);2,043(16,0);1,913(2,1);1,895(2,5);1,892(4,1);1,881(2,3);1,874(4,0);1,871(2,6);1,863(2,6);1,860(3,8);1,852(2,2);1,842(3,7);1,839(2,3 );1,820(1,8);1,575(1,2);1,304(0,6);1,276(6,1);1,265(3,0);1,258(11,6);1,240(5,1);0,899(1,6);0,882(6,2);0,864(2,3);0,008(4,6);0,006(1,5);0,005 4(1,7);0,0046(1,9);0,000(157,2);-0,006(1,8);-0,007(1,6);-0,009(4,6);-0,011(0,6) |
| Beispiel 1.60: ¹H-NMR(400,0 MHz, d₆-DMSO): δ= 13,312(0,7);8,719(2,2);8,697(2,1);8,076(0,7);7,946(0,6);7,905(3,4);7,902(6,3);7,897(4,5);7 ,892(3,9);7,888(2,9);7,885(3,2);7,882(1,7);7,715(1,9);7,710(1,8);7,698(2,2);7,695(2,2);7,693(2,1);7,690(2,1);7,621(0,9);7,566(3,5);7,546(5,5) ;7,525(2,4);7,169(12,3);7,150(16,0);7,133(5,0);7,129(3,2);6,871(5,0);6,868(5,5);6,852(7,5);6,849(8,9);6,833(4,2);6,831(4,2);6,791(9,5);6,788 (9,4);6,782(1,8);6,769(8,9);6,766(7,3);5,542(0,6);5,150(1,8);4,403(0,6);4,385(0,8);4,301(2,4);4,280(2,3);4,192(2,3);3,305(109,6);3,275(0,5);3 ,059(1,2);2,998(0,6);2,892(0,7);2,674(2,2);2,669(3,2);2,665(2,4);2,591(0,9);2,523(10,2);2,518(14,6);2,509(177,1);2,505(379,9);2,500(526,5); 2,496(365,4);2,491(164,4);2,450(1,2);2,385(0,8);2,381(1,0);2,376(0,7);2,336(1,1);2,332(2,1);2,327(3,2);2,322(2,2);2,095(2,2);2,072(2,5);2,0 47(2,4);1,988(1,5);1,908(0,5);1,352(0,8);1,334(1,8);1,316(0,8);1,281(0,6);1,259(0,8);1,236(2,1);1,175(0,7);1,157(0,6);0,008(3,6);0,000(121,1 );-0,009(3,6) |
| Beispiel 1.61: ¹H-NMR(400,0 MHz, d₆-DMSO): δ= 8,614(0,9);8,593(0,9);7,139(2,3);7,119(3,3);7,027(3,6);7,011(3,0);5,398(0,8);5,377(0,8);4, 038(0,8);4,021(0,8);3,306(29,7);2,911(0,7);2,896(0,7);2,761(0,8);2,741(0,7);2,721(0,5);2,674(0,6);2,669(0,8);2,665(0,6);2,526(4,6);2,523(2,6 );2,518(3,3);2,509(42,9);2,505(92,7);2,500(129,0);2,496(90,7);2,491(41,7);2,468(0,6);2,455(0,6);2,450(0,8);2,445(0,8);2,417(0,7);2,407(0,7); 2,395(0,6);2,332(0,6);2,327(0,8);2,322(0,6);2,249(16,0);1,988(3,6);1,980(1,3);1,970(0,5);1,958(1,2);1,949(1,2);1,927(1,0);1,192(1,0);1,175(2 ,0);1,157(1,0);0,008(0,8);0,000(28,0);-0,009(0,9) |
| Beispiel 1.62: ¹H-NMR(400,6 MHz, d₆-DMSO): δ= 8,867(1,2);8,850(1,2);7,899(4,9);7,897(3,5);7,895(3,5);7,894(3,9);7,890(2,7);7,883(2,1);7, 880(2,5);7,877(1,4);7,694(1,6);7,691(1,7);7,689(1,6);7,686(1,5);7,674(1,9);7,671(1,9);7,669(1,9);7,666(1,7);7,550(2,5);7,546(0,6);7,535(0,7); 7,530(4,0);7,510(1,8);7,402(1,5);7,385(1,8);7,369(1,0);7,351(0,7);7,238(2,4);7,235(2,4);7,219(4,8);7,217(4,7);7,216(4,7);7,199(2,9);7,197(2, 8);6,898(3,7);6,896(4,3);6,879(6,8);6,877(7,8);6,859(10,1);6,839(6,0);5,632(1,0);4,739(1,3);4,716(1,7);4,693(0,8);4,406(1,9);4,393(2,1);4,38 2(1,9);4,369(1,7);3,325(0,7);3,000(16,0);2,891(0,5);2,593(16,2);2,526(1,8);2,521(2,3);2,512(17,7);2,508(35,8);2,503(48,5);2,499(34,2);2,494 (15,5);2,087(4,1);1,990(1,8);1,259(0,6);1,234(1,4);1,193(0,6);1,183(0,6);1,175(1,1);1,167(0,6);1,158(0,6);0,986(0,9);0,978(1,4);0,970(0,9);0, 962(1,3);0,000(1,3) |
| Beispiel 1.63: ¹H-NMR(400,0 MHz, d₆-DMSO): δ= 8,614(5,1);8,591(5,1);7,994(1,5);7,975(1,6);7,735(1,3);7,588(1,6);7,204(4,3);7,188(6,0);7, 181(6,0);7,166(3,9);6,967(6,8);6,958(9,6);6,952(15,4);6,928(16,0);5,464(1,1);5,069(3,3);4,038(0,9);4,020(0,8);3,307(174,7);3,276(0,6);3,029 (0,5);2,998(1,0);2,781(4,0);2,743(7,3);2,698(1,9);2,674(3,2);2,669(4,3);2,665(3,3);2,591(1,1);2,525(9,4);2,523(13,6);2,518(18,4);2,510(238,4 );2,505(517,3);2,500(721,0);2,496(506,0);2,491(228,9);2,466(0,6);2,451(1,1);2,332(3,0);2,327(4,2);2,323(2,9);2,072(1,4);1,988(5,9);1,964(6, 9);1,952(7,3);1,940(7,8);1,830(2,6);1,809(4,2);1,791(4,4);1,765(3,0);1,741(2,4);1,723(2,9);1,701(3,0);1,334(0,9);1,235(1,0);1,192(1,3);1,175( 2,4);1,157(1,3);0,008(4,8);0,000(163,2);-0,009(5,0);-0,150(0,5) |
| Beispiel 1.64: ¹H-NMR(400,0 MHz, d₆-DMSO): δ= 8,738(3,3);8,718(3,3);8,117(1,2);7,899(2,8);7,893(2,5);7,889(2,0);7,884(1,7);7,881(1,9);7, 701(1,7);7,683(1,9);7,558(1,2);7,537(1,8);7,517(0,9);7,031(2,8);7,024(4,7);7,003(16,0);6,982(11,7);6,823(8,3);6,811(9,4);6,800(6,5);6,789(6, 6);5,530(0,9);5,122(2,9);5,108(2,8);4,302(2,0);4,282(4,0);4,263(4,0);4,201(3,4);4,181(3,4);4,056(0,6);4,039(2,0);4,021(1,9);4,003(0,7);3,307( 45,4);2,998(15,9);2,675(1,0);2,670(1,5);2,666(1,0);2,591(16,0);2,523(5,1);2,510(89,1);2,506(185,7);2,501(253,3);2,496(181,3);2,492(85,3);2, 332(1,2);2,328(1,6);2,323(1,2);2,097(3,5);2,033(3,9);1,988(9,8);1,352(0,5);1,335(1,0);1,317(0,6);1,279(1,1);1,247(5,0);1,193(2,4);1,175(4,8); 1,157(2,5);0,875(2,3);0,858(7,4);0,841(2,8);0,008(1,5);0,000(41,0);-0,008(1,6) |
| Beispiel 1.66: ¹H-NMR(400,6 MHz, CDCl₃): δ= 7,519(4,8); 7,399(0,9); 7,360(1,4); 7,286(5,9); 7,260(870,0); 7,237(2,2); 7,221(5,3); 7,217(5,5); 7,203(8,4); 7,1 98(9,1); 7,194(5,7); 7,176(4,4); 7,149(6,4); 7,131(4,0); 6,997(4,8); 6,046(1,5); 5,570(0,8); 5,357(1,2); 5,149(1,8); 4,149(1,2); 4,131(3,2); 4,113(3,3); 4,095(0,9); 3,457(1,5); 3,364(4,0); 3,325(16,0); 2,898(1,4); 2,855(3,5); 2,831(2,9); 2,815(4,3); 2,799(2,6); 2,772(1,6); 2,086(1,6); 2,045(15,6); 1,901(5,6); 1,885(5,5); 1,547(43,6); 1,277(5,0); 1,260(9,9); 1,242(4,2); 0,899(1,2); 0,882(4,1); 0,864(1,7); 0,008(6,8); 0,000(220,5); -0,009(6,3) |
| Beispiel 1.67: ¹H-NMR(400,0 MHz, CDCl₃): δ= 7,518(0,6);7,270(0,5);7,269(0,5);7,268(0,6);7,2674(0,7);7,2666(0,7);7,266(0,9);7,265(1,0);7,2 64(1,3);7,2634(1,7);7,2625(2,3);7,259(93,3);7,256(1,3);7,255(0,6);7,209(0,6);7,165(1,1);7,146(1,8);7,120(0,6);7,089(1,5);7,068(0,9);6,995(0, 5);2,966(0,5);2,954(0,5);2,944(0,5);2,874(0,6);2,626(2,2);2,335(9,9);1,921(0,6);1,919(0,8);1,907(0,5);1,900(0,8);1,897(0,6);1,889(0,6);1,887( 0,7);1,868(0,7);1,551(16,0);0,882(1,1);0,008(1,0);0,000(43,0);-0,009(1,3) |
| Beispiel 1.68: ¹H-NMR(400,0 MHz, CDCl₃): δ= 7,260(80,3);7,071(1,5);7,030(11,6);7,027(11,2);6,996(0,6);5,108(0,5);4,130(1,4);4,112(1,4);3, 361(1,2);3,323(5,5);2,991(0,9);2,816(0,6);2,801(1,2);2,785(0,6);2,776(0,8);2,760(1,4);2,743(0,8);2,679(0,9);2,290(16,0);2,044(6,9);1,872(1,6 );1,857(1,7);1,276(2,2);1,259(4,3);1,241(1,9);0,899(0,6);0,882(2,0);0,864(0,8);0,008(1,0);0,000(34,8);-0,009(1,0) |
| Beispiel 1.69: ¹H-NMR(400,0 MHz, CDCl₃): δ= 7,269(0,6);7,268(0,7);7,2673(0,8);7,2665(0,9);7,266(1,0);7,265(1,2);7,264(1,4);7,263(1,9);7,2 60(59,9);7,257(0,7);7,221(0,9);7,124(1,2);7,017(11,5);7,014(10,5);6,970(2,5);5,298(2,2);3,025(1,4);2,997(15,3);2,988(4,2);2,803(0,7);2,787( 1,2);2,766(1,0);2,750(1,6);2,734(1,0);2,708(0,7);2,692(0,5);2,312(4,2);2,284(16,0);2,266(0,8);2,052(0,9);2,043(2,3);2,040(0,8);1,927(0,6);1,9 20(0,7);1,913(0,8);1,901(1,0);1,897(0,9);1,879(1,2);1,865(1,5);1,849(1,4);1,699(0,5);1,693(0,6);1,684(0,8);1,677(1,1);1,668(1,3);1,663(1,4);1 ,447(1,0);1,276(0,7);1,258(1,7);1,240(0,6);0,008(0,7);0,003(0,5);0,002(0,9);0,000(24,9);-0,0026(1,2);-0,0034(0,9);-0,004(0,5);-0,008(0,7) |
| Beispiel 1.70: ¹H-NMR(400,0 MHz, d₆-DMSO): δ= 8,619(1,0);8,598(1,0);7,139(2,3);7,119(3,3);7,027(4,0);7,011(2,8);5,399(0,8);5,379(0,8);3, 316(31,9);2,912(0,7);2,897(0,7);2,762(0,8);2,741(0,7);2,723(0,5);2,519(0,6);2,510(8,2);2,506(17,9);2,501(25,1);2,496(17,5);2,492(7,7);2,446 (0,5);2,427(0,6);2,419(0,6);2,407(0,7);2,397(0,6);2,249(16,0);1,981(1,2);1,972(0,5);1,960(1,2);1,950(1,1);1,929(1,0);0,008(0,5);0,000(18,3) |
| Beispiel 1.71: ¹H-NMR(400,0 MHz, CDCl₃): δ= 7,260(44,1);7,157(1,7);7,138(3,5);7,083(2,2);7,062(1,3);4,130(0,6);4,112(0,6);3,026(2,0);2,99 8(16,0);2,990(5,3);2,974(0,7);2,965(0,9);2,956(0,9);2,943(0,9);2,935(0,8);2,885(0,6);2,865(1,2);2,845(0,9);2,825(0,6);2,335(15,0);2,043(2,9); 1,906(1,1);1,895(0,6);1,887(1,1);1,874(1,0);1,866(0,6);1,855(1,0);1,651(0,9);1,463(0,5);1,446(1,1);1,428(0,5);1,276(0,9);1,258(1,9);1,240(0, 9);0,000(18,5);-0,009(0,7) |
| Beispiel 1.72: ¹H-NMR(400,0 MHz, CDCl₃): δ= 7,278(0,5);7,276(0,8);7,275(0,8);7,268(69,7);7,219(0,7);7,167(2,0);7,148(3,2);7,114(1,1);7,08 8(2,5);7,067(1,5);5,491(0,8);5,300(0,7);3,017(0,5);3,008(0,8);3,004(0,5);2,995(0,6);2,986(0,9);2,977(0,9);2,965(0,9);2,956(0,9);2,880(1,0);2, 861(0,8);2,840(1,7);2,717(5,5);2,330(16,0);1,969(0,8);1,949(1,4);1,938(0,9);1,930(1,4);1,927(1,0);1,919(1,1);1,917(1,3);1,909(0,8);1,898(1,2 );1,877(0,7);1,737(6,4);1,277(1,0);1,265(2,3);1,259(1,8);1,241(0,5);1,237(1,3);1,220(1,2);0,899(1,3);0,882(4,6);0,864(1,7);0,008(0,8);0,000(3 0,3);-0,009(0,9) |
| Beispiel 1.73: ¹H-NMR(400,0 MHz, CDCl₃): δ= 7,519(0,5);7,260(87,4);7,210(0,6);7,172(1,8);7,152(3,1);7,097(3,4);7,078(1,5);6,996(0,5);5,44 9(0,6);5,431(0,6);4,130(1,2);4,112(1,2);3,326(4,5);2,991(0,7);2,987(0,8);2,978(0,9);2,966(0,8);2,956(0,9);2,882(1,0);2,862(0,8);2,842(0,6);2, 341(16,0);2,044(5,6);1,934(0,9);1,923(0,6);1,915(0,9);1,902(0,9);1,883(0,8);1,571(7,3);1,276(1,9);1,270(1,1);1,265(1,1);1,259(3,8);1,241(1,7 );0,899(0,5);0,882(1,9);0,864(0,7);0,008(0,9);0,000(36,3);-0,009(1,2) |
| Beispiel 1.74: ¹H-NMR(400,0 MHz, d₆-DMSO): δ= 7,828(0,5);7,556(0,5);7,138(1,7);7,118(2,4);7,020(3,5);7,006(1,6);4,345(1,1);4,329(3,6);4, 311(3,9);4,293(1,3);3,315(13,9);2,999(1,8);2,922(0,5);2,915(0,6);2,900(0,5);2,894(0,5);2,592(1,7);2,511(6,2);2,506(13,4);2,501(18,7);2,497( 13,0);2,492(5,8);2,244(12,2);1,995(1,1);1,989(1,3);1,974(1,1);1,964(1,0);1,943(0,9);1,343(7,1);1,325(16,0);1,307(6,9);1,246(0,8);1,175(0,7); 0,858(1,5);0,840(0,5);0,000(8,0) |
| Beispiel 1.75: ¹H-NMR(400,0 MHz, d₆-DMSO): δ= 8,416(0,6);8,396(0,6);7,905(0,6);7,816(0,6);7,140(2,2);7,121(3,1);7,029(7,0);7,010(2,0);5, 416(0,5);3,313(0,9);3,169(1,3);2,954(0,5);2,931(0,6);2,915(0,8);2,900(0,8);2,767(0,8);2,748(0,7);2,729(0,5);2,524(0,7);2,519(1,0);2,510(16,9 );2,506(37,5);2,501(52,7);2,497(37,4);2,492(17,4);2,456(1,4);2,451(1,5);2,447(1,3);2,248(16,0);1,996(1,3);1,987(0,6);1,975(1,4);1,965(1,3);1 ,953(0,6);1,944(1,2);0,008(0,9);0,000(33,6);-0,009(1,1) |
| Beispiel 1.76: ¹H-NMR(400,0 MHz, CDCl₃): δ= 7,266(0,5);7,260(40,9);7,158(1,6);7,138(3,7);7,079(2,0);7,058(1,2);2,990(15,9);2,977(16,0);2, 968(0,8);2,958(0,8);2,945(0,8);2,936(0,8);2,868(1,0);2,848(0,8);2,829(0,5);2,327(13,4);2,043(2,1);1,913(1,0);1,902(0,5);1,894(1,0);1,881(0,9 );1,862(0,9);1,602(1,0);1,276(1,0);1,265(0,9);1,258(1,7);1,240(0,7);0,899(0,5);0,882(1,9);0,864(0,7);0,000(18,9);-0,009(0,5) |
| Beispiel 1.77: ¹H-NMR(400,0 MHz, CDCl₃): δ= 7,519(0,5);7,270(0,6);7,269(0,7);7,2674(1,0);7,2665(1,1);7,265(1,7);7,260(80,5);7,256(0,7);7, 157(2,6);7,149(2,5);7,139(3,4);7,076(2,3);7,056(1,4);4,148(0,9);4,130(2,9);4,112(2,9);4,095(1,0);3,364(5,0);3,140(6,3);2,973(0,5);2,965(0,8); 2,956(0,9);2,943(0,9);2,935(0,8);2,885(0,6);2,864(1,3);2,844(1,0);2,825(0,7);2,331(16,0);2,043(13,5);1,934(0,5);1,913(1,3);1,902(0,6);1,894( 1,2);1,881(1,2);1,873(0,6);1,862(1,1);1,587(0,8);1,276(4,2);1,258(8,8);1,241(4,1);0,882(0,9);0,008(1,0);0,000(36,9);-0,009(1,1) |
| Beispiel 1.78: ¹H-NMR(400,0 MHz, d₆-DMSO): δ= 8,035(0,6);7,363(0,6);7,131(2,2);7,112(3,0);7,019(6,3);7,000(1,9);6,999(1,8);4,039(0,8);4, 021(0,8);3,315(18,2);2,945(0,5);2,930(0,6);2,923(0,6);2,913(0,8);2,906(0,8);2,891(0,8);2,884(0,7);2,761(0,9);2,741(0,7);2,722(0,6);2,519(0,6 );2,510(8,4);2,506(18,4);2,501(25,7);2,497(17,8);2,492(7,8);2,429(0,5);2,245(16,0);2,008(0,5);1,988(4,2);1,977(0,6);1,965(1,4);1,955(1,3);1, 943(0,6);1,933(1,2);1,193(1,1);1,175(2,2);1,157(1,1);0,000(3,9) |
| Beispiel 1.79: ¹H-NMR(400,0 MHz, CDCl₃): δ= 7,636(5,3);7,631(2,1);7,615(6,7);7,485(7,8);7,480(2,4);7,469(2,6);7,464(5,4);7,259(32,9);7,08 6(3,9);7,069(3,3);7,039(2,9);7,019(1,4);5,095(2,4);4,129(1,4);4,111(1,4);3,067(1,0);3,048(1,1);3,029(1,2);3,010(1,3);2,554(1,0);2,530(1,2);2, 515(0,9);2,492(1,0);2,311(16,0);2,282(1,2);2,262(0,9);2,042(6,0);1,338(0,7);1,303(11,4);1,287(11,8);1,276(4,7);1,265(6,0);1,258(6,3);1,240( 2,5);0,899(2,6);0,882(7,2);0,864(3,2);0,008(0,6);0,000(12,3) |
| Beispiel 1.80: ¹H-NMR(400,0 MHz, CDCl₃): δ= 7,259(60,2);7,076(1,6);7,057(3,5);7,048(2,8);7,026(2,7);7,007(1,2);6,786(2,5);6,757(2,7);6,74 2(2,9);6,713(2,9);6,088(1,6);6,044(1,4);5,519(4,0);5,516(3,9);5,491(3,8);5,488(3,9);5,057(2,4);4,130(1,6);4,112(1,6);4,094(0,5);3,054(1,1);3, 035(1,2);3,016(1,3);2,996(1,3);2,539(0,9);2,517(1,1);2,501(0,8);2,478(1,0);2,296(16,0);2,280(1,0);2,260(1,0);2,242(0,8);2,044(7,4);1,304(0,6 );1,281(12,9);1,276(4,6);1,264(15,2);1,258(7,2);1,240(4,0);0,899(1,8);0,882(6,9);0,864(2,6);0,008(0,6);0,000(26,1);-0,009(0,8) |
| Beispiel 1.81: ¹H-NMR(400,0 MHz, CDCl₃): δ= 7,518(2,1);7,290(0,5);7,259(358,1);7,209(2,2);7,105(1,7);7,086(3,3);7,066(1,9);7,046(2,7);7,0 27(1,3);6,995(2,0);6,348(1,9);6,345(5,0);6,341(4,9);6,338(1,7);5,981(1,8);5,977(5,0);5,974(5,0);5,970(1,8);4,961(1,9);3,094(1,2);3,074(1,3);3 ,055(1,5);3,036(1,5);2,559(0,9);2,535(1,1);2,519(0,8);2,497(1,1);2,343(0,5);2,312(16,0);2,284(0,9);2,264(0,5);2,026(0,5);1,732(0,5);1,541(3, 7);1,495(0,6);1,491(0,6);1,323(3,0);1,294(12,5);1,284(2,1);1,277(9,4);1,269(4,8);1,259(3,3);1,243(2,6);1,209(3,0);1,193(3,0);0,882(1,5);0,86 4(0,6);0,008(3,6);0,000(141,8);-0,009(4,1);-0,050(0,9) |
| Beispiel 1.87: ¹H-NMR(400.6 MHz, d₆-DMSO): |
| δ= 8.6232 (2.4); 8.6013 (2.4); 7.9920 (1.4); 7.2133 (7.2); 7.2063 (16.0); 7.1953 (2.5); 7.1842 (2.7); 7.1762 (2.8); 7.1658 (4.0); 7.1570 (3.7); 7.1444 (2.1); 7.1205 (7.1); 7.1025 (3.6); 6.9008 (0.9); 5.7607 (1.4); 5.5596 (0.7); 5.4129 (3.7); 5.4004 (4.2); 5.2366 (1.4); 5.2171 (2.4); 5.1976 (1.4); 4.4550 (1.0); 4.4366 (2.7); 4.4188 (3.1); 4.4052 (2.3); 4.3870 (0.8); 3.4495 (0.6); 3.3886 (0.9); 3.3684 (7.5); 3.3478 (382.5); 3.3244 (6.7); 3.3055 (20.7); 3.2816 (0.5); 3.1942 (2.8); 3.1761 (2.8); 3.1556 (3.4); 3.1375 (3.0); 2.9765 (0.7); 2.7490 (1.9); 2.7296 (1.9); 2.7105 (1.6); 2.6910 (1.2); 2.6781 (0.6); 2.5179 (28.9); 2.5137 (57.4); 2.5092 (76.8); 2.5047 (56.0); 2.4765 (0.5); 1.9958 (0.7); 1.2431 (0.8) |
| Beispiel 1.82: ¹H-NMR(400.6 MHz, MeOD): |
| δ= 7.2752 (0.8); 7.2396 (16.0); 7.2278 (11.9); 5.4090 (1.4); 4.9255 (0.5); 4.8765 (83.3); 4.4781 (1.2); 4.4608 (3.0); 4.4443 (3.1); 4.4268 (1.3); 3.3372 (8.3); 3.3332 (15.6); 3.3291 (23.1); 3.3252 (16.8); 3.3212 (8.9); 3.3096 (4.7); 3.2884 (4.9); 3.2705 (4.5); 2.8872 (2.1); 2.8695 (2.1); 2.8478 (1.9); 2.8303 (1.8) |
| Beispiel 1.101: ¹H-NMR(400.0 MHz, CDCl₃): |
| δ= 7.2596 (45.1); 7.0743 (1.5); 7.0554 (3.0); 7.0344 (3.0); 7.0212 (2.6); 7.0021 (1.2); 5.5520 (1.0); 5.2974 (1.7); 4.8164 (16.0); 4.1475 (0.6); 4.1296 (1.7); 4.1117 (1.7); 4.0939 (0.6); 3.0502 (1.0); 3.0309 (1.2); 3.0117 (1.3); 2.9924 (1.3); 2.5334 (1.0); 2.5107 (1.1); 2.4949 (0.9); 2.4722 (1.0); 2.2933 (14.9); 2.2660 (0.8); 2.2470 (1.0); 2.2288 (0.8); 2.0430 (7.9); 2.0074 (0.6); 1.2757 (3.6); 1.2711 (11.0); 1.2579 (6.8); 1.2543 (11.0); 1.2401 (2.4); 0.0080 (0.6); -0.0002 (16.9); -0.0084 (0.5) |
| Beispiel 1.140: ¹H-NMR(400.6 MHz, MeOD): |
| δ= 7.3046 (3.6); 7.2843 (7.3); 7.2732 (2.7); 7.2582 (5.1); 7.2393 (4.9); 7.2209 (3.0); 7.2034 (0.9); 5.4693 (1.3); 4.8742 (111.1); 4.6656 (1.8); 4.1465 (1.3); 4.1286 (3.6); 4.1108 (3.6); 4.0930 (1.2); 3.3375 (9.8); 3.3334 (18.8); 3.3293 (26.8); 3.3252 (18.4); 3.3211 (9.1); 3.2149 (1.0); 3.2033 (1.0); 3.1740 (1.5); 3.1628 (1.3); 3.0096 (3.7); 3.0067 (3.8); 2.9685 (2.5); 2.6912 (0.9); 2.6096 (0.6); 2.0317 (16.0); 1.3442 (1.0); 1.3305 (1.0); 1.3085 (0.8); 1.2770 (4.3); 1.2591 (8.4); 1.2413 (4.1); 0.9204 (0.8) |
| Beispiel 1.91: ¹H-NMR(400.6 MHz, MeOD): |
| δ= 7.3040 (7.6); 7.2837 (16.0); 7.2771 (8.8); 7.2730 (7.0); 7.2591 (10.8); 7.2576 (11.3); 7.2375 (10.6); 7.2200 (6.7); 7.2028 (2.3); 5.6777 (0.7); 5.5083 (1.9); 5.4679 (2.9); 4.8977 (2.2); 4.8747 (217.5); 4.8496 (2.7); 4.6663 (4.1); 3.3526 (0.8); 3.3375 (19.6); 3.3333 (38.2); 3.3292 (56.7); 3.3252 (39.3); 3.3210 (20.3); 3.3042 (1.2); 3.3000 (1.1); 3.2956 (0.9); 3.2150 (2.3); 3.2023 (2.2); 3.1737 (3.4); 3.1611 (3.0); 3.0095 (7.7); 3.0063 (8.1); 2.9685 (5.4); 2.9652 (5.4); 2.7232 (1.0); 2.6908 (2.2); 2.0315 (2.0); 1.3463 (1.4); 1.3273 (2.4); 1.3082 (6.4); 1.2768 (1.2); 1.2589 (1.5); 1.2411 (0.8); 0.9372 (3.1); 0.9203 (11.0); 0.9026 (4.2) |
| Beispiel 1.90: ¹H-NMR(400.0 MHz, d₆-DMSO): |
| δ= 8.6554 (2.4); 8.6334 (2.5); 8.0343 (0.6); 8.0131 (0.6); 7.6352 (0.7); 7.2244 (0.6); 7.2075 (3.8); 7.2044 (5.0); 7.1999 (5.8); 7.1938 (15.3); 7.1918 (16.0); 7.1804 (2.1); 7.1685 (2.7); 7.1607 (2.9); 7.1500 (3.8); 7.1417 (3.6); 7.1284 (2.0); 7.0868 (5.6); 7.0683 (3.5); 5.5649 (0.6); 5.4050 (3.8); 5.3923 (4.1); 5.3636 (0.9); 5.3495 (0.8); 5.2155 (1.5); 5.1956 (2.4); 5.1758 (1.5); 4.4438 (0.5); 4.4251 (1.7); 4.4074 (2.2); 4.3936 (2.0); 4.3755 (0.9); 3.3651 (1.0); 3.3142 (134.9); 3.1796 (2.0); 3.1614 (2.2); 3.1409 (2.6); 3.1226 (2.3); 2.7377 (1.9); 2.7179 (1.8); 2.6964 (1.7); 2.6945 (1.7); 2.6787 (1.5); 2.6694 (0.8); 2.6648 (0.5); 2.5550 (0.6); 2.5506 (0.6); 2.5228 (2.4); 2.5181 (3.4); 2.5094 (37.0); 2.5049 (76.7); 2.5003 (105.5); 2.4957 (73.2); 2.4912 (33.5); 2.3271 (0.7); 1.9828 (1.0); -0.0002 (14.4) |
| Beispiel 1.139: ¹H-NMR(400.0 MHz, d₆-DMSO): |
| δ= 7.2584 (2.0); 7.2411 (4.0); 7.2317 (1.3); 7.2279 (1.3); 7.2124 (2.4); 7.2081 (1.5); 7.1954 (1.4); 7.1905 (2.2); 7.1866 (1.6); 7.1716 (1.4); 5.7518 (6.8); 5.1244 (0.8); 4.5305 (0.9); 4.0380 (0.9); 4.0202 (0.9); 3.3143 (5.4); 3.0973 (0.5); 3.0853 (0.5); 3.0570 (0.8); 3.0446 (0.7); 2.9808 (0.7); 2.9427 (16.0); 2.8866 (1.3); 2.8465 (0.9); 2.5226 (0.7); 2.5179 (1.0); 2.5091 (10.4); 2.5046 (21.5); 2.5000 (29.5); 2.4954 (20.4); 2.4909 (9.3); 1.9874 (4.3); 1.1921 (1.3); 1.1743 (2.6); 1.1565 (1.2); -0.0002 (6.2) |
| Beispiel 1.138: ¹H-NMR(400.0 MHz, d₆-DMSO): |
| δ= 7.8905 (0.8); 7.8870 (0.6); 7.8853 (0.6); 7.2586 (2.1); 7.2410 (4.2); 7.2316 (1.4); 7.2280 (1.4); 7.2126 (2.4); 7.2083 (1.6); 7.1955 (1.4); 7.1906 (2.2); 7.1717 (1.4); 5.7520 (2.2); 5.1234 (0.7); 4.5287 (0.9); 4.0381 (1.5); 4.0203 (1.5); 3.3125 (6.6); 3.0975 (0.6); 3.0849 (0.6); 3.0571 (0.8); 3.0449 (0.8); 2.9810 (0.9); 2.9428 (16.0); 2.8865 (1.3); 2.8460 (0.9); 2.5227 (1.0); 2.5180 (1.5); 2.5093 (14.8); 2.5047 (30.6); 2.5002 (42.0); 2.4956 (28.9); 2.4910 (13.1); 1.9875 (6.9); 1.2368 (0.6); 1.1922 (2.0); 1.1744 (4.0); 1.1566 (2.0); 1.0703 (0.5); 1.0542 (0.5); - 0.0002 (8.5) |
| Beispiel 1.88: ¹H-NMR(400.6 MHz, MeOD): |
| δ= 7.3208 (1.9); 7.3033 (4.2); 7.2851 (3.5); 7.2797 (2.2); 7.2781 (1.9); 7.2758 (2.0); 7.2741 (1.8); 7.2619 (3.2); 7.2603 (3.4); 7.2584 (2.7); 7.2429 (3.8); 7.2381 (2.9); 7.2244 (2.2); 7.2201 (1.8); 7.2066 (0.7); 5.5092 (16.0); 5.4823 (0.8); 4.8741 (41.5); 4.6780 (1.2); 4.1466 (0.6); 4.1288 (1.9); 4.1109 (1.9); 4.0931 (0.7); 3.3373 (5.4); 3.3333 (10.4); 3.3293 (14.6); 3.3252 (10.7); 3.3212 (6.0); 3.2945 (22.6); 3.2228 (0.9); 3.2106 (0.8); 3.1809 (1.1); 3.1694 (1.1); 3.0132 (2.5); 3.0101 (2.6); 2.9722 (1.8); 2.9690 (1.8); 2.0318 (9.0); 1.3090 (0.9); 1.2771 (2.6); 1.2592 (5.2); 1.2414 (2.6) |
| Beispiel 1.137: ¹H-NMR(400.6 MHz, MeOD): |
| δ= 7.3216 (2.9); 7.3041 (6.2); 7.2858 (5.2); 7.2764 (2.9); 7.2610 (4.8); 7.2438 (5.7); 7.2389 (4.4); 7.2254 (3.2); 7.2081 (1.1); 5.5097 (16.0); 5.4802 (1.2); 4.8751 (78.2); 4.6759 (2.0); 4.1469 (0.5); 4.1291 (1.6); 4.1112 (1.6); 4.0936 (0.5); 3.3372 (11.1); 3.3333 (20.8); 3.3293 (28.7); 3.3253 (21.4); 3.3214 (12.1); 3.2961 (30.2); 3.2227 (1.4); 3.2112 (1.4); 3.1813 (1.8); 3.1712 (1.7); 3.0107 (3.9); 2.9697 (2.6); 2.0321 (7.3); 1.3092 (1.2); 1.2773 (2.0); 1.2595 (4.0); 1.2417 (2.0) |
| Beispiel 1.95: ¹H-NMR(400.6 MHz, CDCl₃): |
| δ= 8.0523 (0.7); 7.5198 (0.6); 7.4128 (0.6); 7.3932 (0.9); 7.2875 (0.7); 7.2862 (0.7); 7.2831 (1.3); 7.2741 (4.2); 7.2717 (5.6); 7.2614 (106.3); 7.2557 (1.7); 7.2548 (1.5); 7.2540 (1.4); 7.2533 (1.3); 7.2524 (1.2); 7.2508 (0.8); 7.2500 (0.9); 7.2492 (0.8); 7.2476 (0.7); 7.2468 (0.6); 7.2461 (0.6); 7.2444 (0.7); 7.2436 (0.6); 7.2405 (1.1); 7.2357 (1.6); 7.2342 (1.7); 7.2285 (1.2); 7.2222 (1.0); 7.2127 (0.9); 7.2032 (1.0); 7.1921 (0.7); 6.9977 (0.6); 6.8516 (0.7); 6.8318 (0.8); 5.4976 (0.7); 5.4842 (0.8); 5.4760 (0.7); 5.4633 (0.7); 5.3954 (1.3); 4.3177 (0.6); 4.2169 (0.6); 4.2089 (1.0); 4.1971 (0.6); 3.4071 (2.1); 3.3972 (16.0); 3.1904 (0.6); 3.1494 (1.2); 3.1258 (0.8); 3.0797 (0.5); 3.0688 (0.6); 3.0479 (0.9); 3.0366 (0.9); 3.0064 (0.5); 2.0877 (1.0); 1.7916 (0.8); 0.0080 (1.6); -0.0002 (57.9); -0.0085 (1.5) |
| Beispiel 1.93: ¹H-NMR(400.6 MHz, de-DMSO): |
| δ= 7.2885 (1.1); 7.2705 (1.9); 7.2501 (2.9); 7.2469 (3.7); 7.2288 (2.3); 7.2109 (1.1); 7.2080 (0.8); 7.1982 (1.7); 7.1934 (1.3); 7.1805 (1.7); 7.1759 (1.4); 7.1629 (0.6); 7.1584 (0.5); 4.2042 (0.9); 3.4093 (0.6); 3.4037 (0.6); 3.3990 (0.6); 3.3949 (0.7); 3.3923 (0.8); 3.3886 (2.1); 3.3854 (1.3); 3.3775 (2.2); 3.3743 (1.8); 3.3658 (3.8); 3.3618 (5.6); 3.3391 (2197.7); 3.3212 (3.4); 3.3197 (3.3); 3.3143 (1.3); 3.3133 (1.1); 3.3092 (0.6); 3.3077 (0.7); 3.3070 (0.7); 3.2977 (1.7); 3.2903 (0.7); 3.2879 (0.7); 3.2839 (0.8); 3.2741 (16.0); 3.1010 (0.8); 3.0201 (0.6); 3.0065 (0.6); 2.6757 (1.2); 2.6710 (1.7); 2.6664 (1.2); 2.5248 (3.8); 2.5201 (4.9); 2.5114 (97.7); 2.5068 (214.7); 2.5022 (302.0); 2.4976 (207.4); 2.4931 (92.7); 2.4729 (0.8); 2.4660 (0.6); 2.4611 (0.7); 2.4570 (0.6); 2.3385 (0.6); 2.3339 (1.4); 2.3293 (1.9); 2.3246 (1.4); 2.3201 (0.7); 1.9886 (1.3); 1.2475 (0.8); 1.1745 (0.8); 0.8584 (1.8); 0.8407 (0.6); 0.0079 (0.7); -0.0002 (33.6); -0.0085 (1.1) |
| Beispiel 1.136: ¹H-NMR(400.0 MHz, CDCl₃): |
| δ= 7.5186 (0.5); 7.3097 (0.8); 7.2597 (85.7); 7.2497 (2.0); 7.2433 (1.6); 7.2219 (0.5); 7.2180 (0.5); 7.2108 (0.5); 4.1305 (0.9); 4.1126 (1.0); 3.3917 (16.0); 3.3899 (13.6); 3.1606 (0.6); 3.1200 (1.1); 3.0413 (0.8); 3.0286 (0.8); 2.9999 (5.7); 2.9610 (0.8); 2.0436 (4.3); 1.5464 (2.3); 1.2764 (1.3); 1.2585 (2.8); 1.2407 (1.2); 0.0080 (1.4); -0.0002 (47.7); -0.0085 (1.6) |
| Beispiel 1.83: ¹H-NMR(400.0 MHz, CDCl₃): |
| δ= 7.2595 (41.1); 7.2437 (5.6); 7.2257 (2.3); 7.2064 (0.9); 7.1920 (1.5); 7.1740 (2.6); 7.1212 (0.9); 4.1457 (1.2); 4.1278 (3.5); 4.1099 (3.6); 4.0921 (1.2); 3.2229 (1.0); 3.2110 (1.0); 3.1814 (1.5); 3.1700 (1.4); 3.0202 (3.2); 2.9791 (2.2); 2.0414 (16.0); 1.2748 (4.4); 1.2570 (9.0); 1.2391 (4.3); 0.0698 (1.0); -0.0002 (15.0) |
| Beispiel 1.84: ¹H-NMR(400.0 MHz, CDCl₃): |
| δ= 7.5182 (1.3); 7.3092 (0.7); 7.2593 (233.6); 7.2445 (6.8); 7.2300 (5.3); 7.2128 (8.0); 7.1757 (2.7); 7.1611 (2.9); 6.9952 (1.5); 5.2976 (16.0); 4.8038 (1.3); 4.1288 (1.0); 4.1111 (1.0); 3.2326 (2.8); 3.2203 (2.8); 3.1913 (4.2); 3.1791 (3.9); 3.0275 (7.3); 3.0244 (7.3); 2.9860 (5.1); 2.9830 (4.9); 2.8929 (0.8); 2.0422 (4.6); 1.5706 (4.1); 1.2754 (1.4); 1.2576 (3.3); 1.2397 (1.7); 0.0080 (2.5); -0.0002 (79.4); -0.0085 (2.1) |
| Beispiel 1.105: ¹H-NMR(400.0 MHz, CDCl₃): |
| δ= 7.2594 (27.4); 7.0824 (1.0); 7.0628 (3.7); 7.0221 (1.5); 7.0180 (1.2); 7.0015 (0.8); 4.1291 (1.0); 4.1112 (1.0); 3.0592 (0.7); 3.0400 (0.7); 3.0206 (0.8); 3.0015 (0.8); 2.5349 (0.6); 2.5124 (0.6); 2.4966 (0.5); 2.4741 (0.6); 2.2962 (9.3); 2.2746 (0.8); 2.2550 (0.8); 2.2377 (0.6); 2.0423 (4.6); 1.7104 (16.0); 1.7090 (15.8); 1.2829 (7.4); 1.2756 (2.1); 1.2661 (7.5); 1.2577 (3.3); 1.2398 (1.5); -0.0002 (10.0) |
| Beispiel 1.103: ¹H-NMR(400.0 MHz, CDCl₃): |
| δ= 7.5186 (0.5); 7.2597 (84.6); 7.0875 (1.8); 7.0685 (3.4); 7.0533 (2.7); 7.0268 (2.6); 7.0075 (1.4); 6.9957 (0.6); 5.6023 (0.9); 5.2980 (3.4); 5.1096 (1.1); 5.0930 (3.1); 5.0764 (3.2); 5.0598 (1.1); 4.1478 (1.1); 4.1300 (3.5); 4.1121 (3.6); 4.0942 (1.2); 3.0667 (1.2); 3.0476 (1.3); 3.0282 (1.4); 3.0089 (1.5); 2.5409 (1.0); 2.5186 (1.2); 2.5027 (0.9); 2.4797 (1.0); 2.2977 (15.7); 2.2811 (1.2); 2.2623 (1.2); 2.2435 (0.8); 2.0432 (16.0); 1.6467 (10.4); 1.6452 (9.8); 1.6301 (10.7); 1.6287 (9.8); 1.2833 (7.5); 1.2798 (7.4); 1.2761 (6.2); 1.2665 (7.7); 1.2630 (7.5); 1.2582 (10.6); 1.2404 (4.8); 0.0079 (1.1); -0.0002 (33.3); -0.0085 (1.2) |
| Beispiel 1.96: ¹H-NMR(400.0 MHz, CDCl₃): |
| δ= 7.2595 (28.9); 7.2464 (1.2); 7.2336 (0.7); 3.3848 (16.0); 2.0432 (1.2); 1.4658 (1.6); 1.4480 (3.2); 1.4301 (1.6); 1.2582 (0.8); -0.0002 (10.7) |
| Beispiel 1.141: ¹H-NMR(400.0 MHz, CDCl₃): |
| δ= 7.2591 (70.4); 7.0837 (1.2); 7.0638 (2.8); 7.0321 (3.2); 7.0157 (3.0); 5.3266 (0.9); 4.1302 (0.9); 4.1123 (0.9); 3.6459 (8.5); 3.0621 (1.0); 3.0428 (1.2); 3.0236 (1.2); 3.0042 (1.3); 2.5441 (0.9); 2.5213 (1.0); 2.5056 (0.8); 2.4826 (0.9); 2.2998 (16.0); 2.2653 (0.6); 2.0431 (4.0); 1.2757 (2.9); 1.2711 (10.4); 1.2579 (4.8); 1.2544 (9.9); 1.2403 (1.4); 0.8988 (0.7); 0.8818 (2.3); 0.8641 (0.9); 0.0079 (0.7); -0.0002 (24.8); - 0.0085 (0.8) |
| Beispiel 1.143: ¹H-NMR(400.0 MHz, CDCl₃): |
| δ= 7.2591 (8.0); 7.0042 (0.5); 2.2925 (2.3); 1.2597 (2.0); 1.2429 (1.7); 0.8818 (0.6); 0.3010 (0.6); 0.2922 (16.0); 0.2833 (0.5); -0.0002 (3.0) |
| Beispiel 1.145: ¹H-NMR(400.0 MHz, CDCl₃): |
| δ= 7.2593 (56.4); 7.0821 (2.0); 7.0620 (1.2); 7.0133 (0.9); 6.9952 (0.8); 5.1820 (0.6); 5.1602 (1.0); 5.1391 (0.6); 4.1305 (0.5); 4.1126 (0.5); 4.0816 (1.0); 4.0458 (16.0); 4.0113 (1.0); 3.0207 (0.5); 3.0009 (0.6); 2.2940 (6.2); 2.0434 (2.3); 1.5600 (1.0); 1.2828 (5.6); 1.2764 (1.1); 1.2659 (5.5); 1.2585 (1.7); 1.2406 (0.7); 0.0079 (0.6); -0.0002 (20.1); -0.0085 (0.6) |
| Beispiel 1.147: ¹H-NMR(400.0 MHz, CDCl₃): |
| δ= 7.26 (21.0); 7.08 (0.6); 7.06 (1.3); 7.02 (2.8); 7.01 (0.6); 3.03 (0.5); 3.01 (0.6); 2.99 (0.6); 2.29 (7.4); 2.17 (17.4); 1.27 (5.4); 1.25 (5.2); 0.00 (18.5) |
| Beispiel 1.149: ¹H-NMR (400.6 MHz, CDCl₃): δ= 8.81 (1.8); 8.70 (1.3); 7.26 (21.0); 7.14 (1.9); 7.12 (3.9); 7.08 (3.6); 7.06 (2.6); 7.00 (1.5); 5.61 (0.6); 5.27 (0.7); 3.16 (0.8); 3.14 (0.9); 3.12 (1.0); 3.10 (1.0); 2.76 (5.9); 2.74 (7.0); 2.60 (0.7); 2.58 (0.9); 2.57 (0.6); 2.45 (0.6); 2.43 (1.0); 2.42 (1.0); 2.40 (0.6); 2.33 (0.6); 2.31 (17.5); 1.58 (2.1); 1.34 (2.5); 1.32 (2.7); 1.29 (3.4); 1.27 (3.0); 1.01 (0.6); 0.99(0.6) 0.00 (19.6) |
| Beispiel 1.107: ¹H-NMR (400.6 MHz, CDCl₃): |
| δ= 8.3244 (0.7); 7.2607 (5.0); 7.1277 (1.8); 7.1082 (3.2); 7.0592 (5.9); 7.0407 (1.8); 5.7635 (0.2); 5.3068 (0.4); 3.4285 (0.4); 3.1276 (1.2); 3.1085 (1.3); 3.0890 (1.5); 3.0699 (1.5); 2.5807 (1.0); 2.5595 (1.2); 2.5422 (0.9); 2.5210 (1.1); 2.4098 (0.5); 2.3906 (1.1); 2.3735 (1.3); 2.3722 (1.3); 2.3535 (1.0); 23336 (0.6); 2.3039 (17.6); 1.6388 (63.1); 1.2982 (10.7); 1.2813 (10.9); -0.0002 (0.7) |
| Beispiel 1.109: ¹H-NMR (400.6 MHz, CDCl₃) |
| δ= 8.2973 (3.3); 7.2606 (34.0); 7.1296 (1.3); 7.1095 (2.3); 7.0590 (2.2); 7.0493 (1.6); 7.0433 (2.0); 5.0781 (1.5); 5.0617 (1.5); 5.0453 (0.5); 3.1276 (0.8); 3.1084 (0.9); 3.0889 (1.0); 3.0697 (1.0); 2.5872 (0.7); 2.5655 (0.8); 2.5488 (0.6); 2.5270 (0.7); 2.3792 (0.6); 2.3602 (0.7); 2.3412 (0.6); 2.3268 (0.8); 2.3068 (11.5); 1.6290 (15.8); 1.6126 (16.0); 1.5892 (0.6); 1.5838 (0.6); 1.5786 (0.5); 1.3247 (0.7); 1.3074 (1.3); 1.3013 (6.7); 1.2845 (6.8); 1.2644 (1.1); 1.2594 (1.2); 1.2562 (1.2); 0.8820 (1.4); 0.8643 (0.6); -0.0002 (5.2) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277° C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

In den nachfolgenden Tabellen werden folgende Abkürzungen verwendet:

**Unerwünschte Pflanzen / Weeds:**

| | | | |
|---|---|---|---|
| ABUTH: | *Abutilon theophrasti* | ALOMY: | *Alopecurus myosuroides* |
| AMARE: | *Amaranthus retroflexus* | AVEFA: | *Avena fatua* |
| CYPES: | *Cyperus esculentus* | ECHCG: | *Echinochloa crus-galli* |
| LOLMU: | *Lolium multiflorum* | MATIN: | *Matricaria inodora* |
| PHBPU: | *Ipomoea purpurea* | POLCO: | *Polygonum convolvulus* |
| SETVI: | *Setaria viridis* | STEME: | *Stellaria media* |
| VERPE: | *Veronica persica* | VIOTR: | *Viola tricolor* |

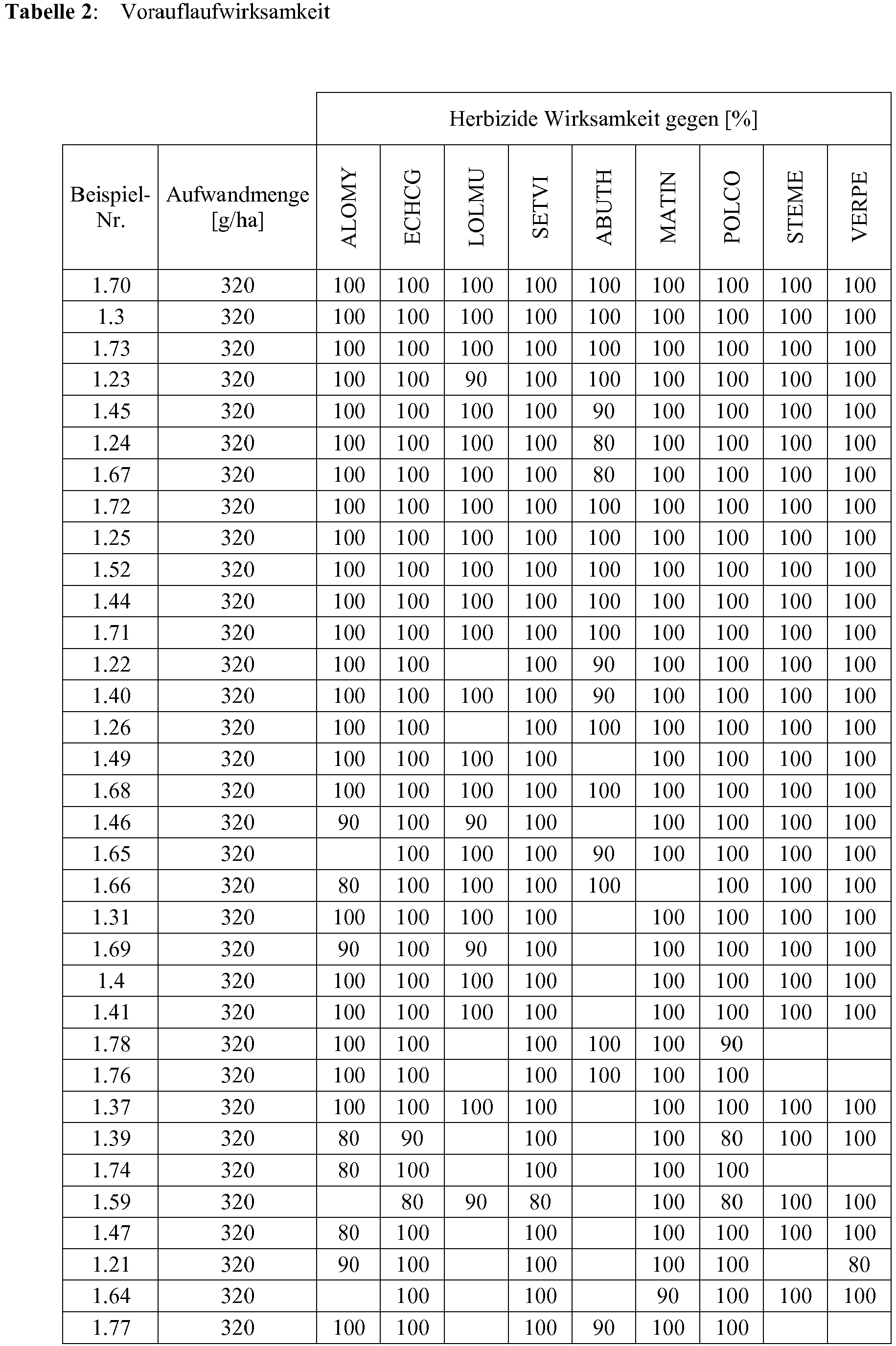

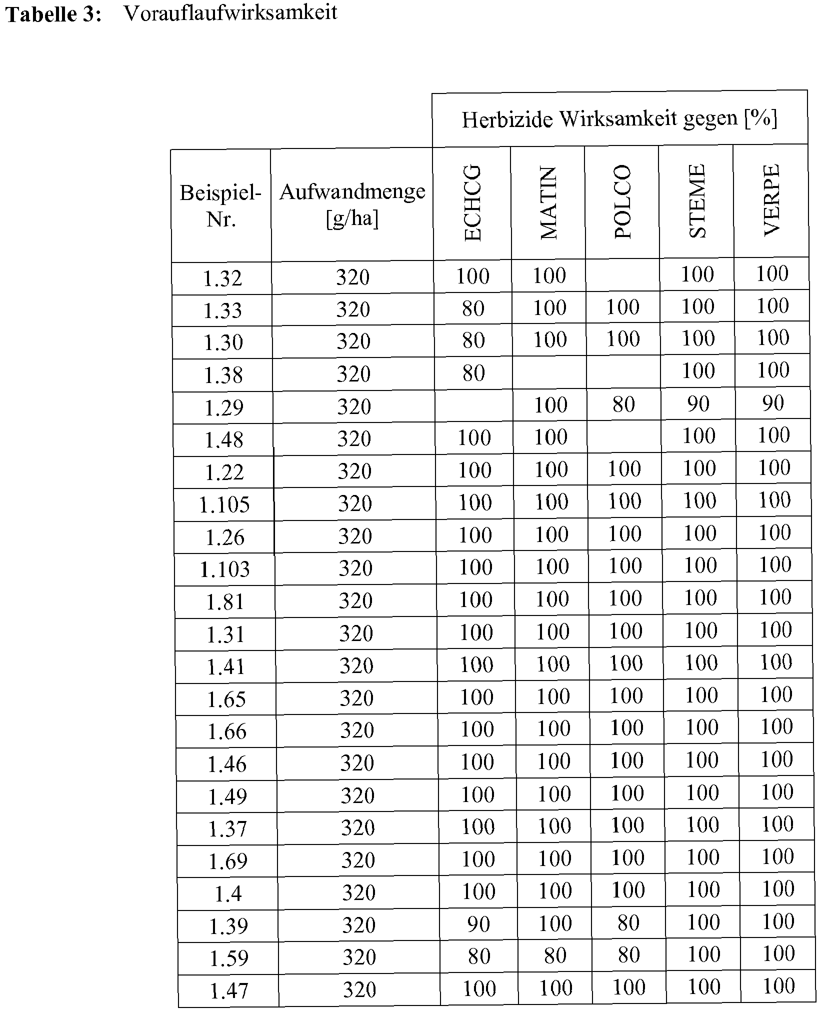

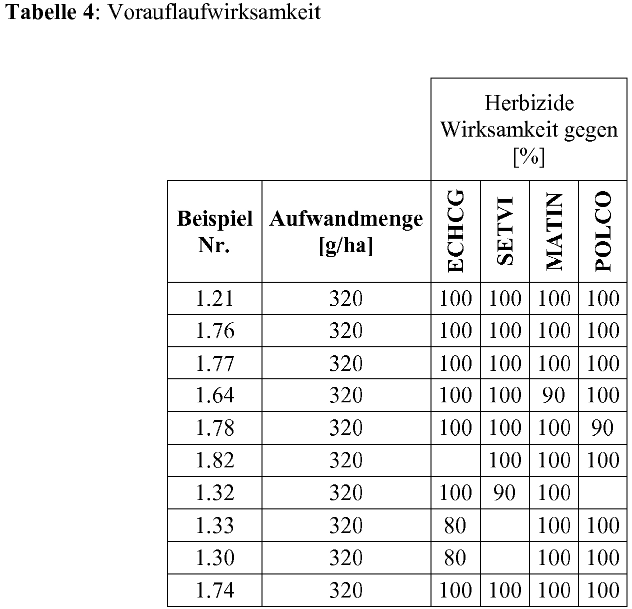

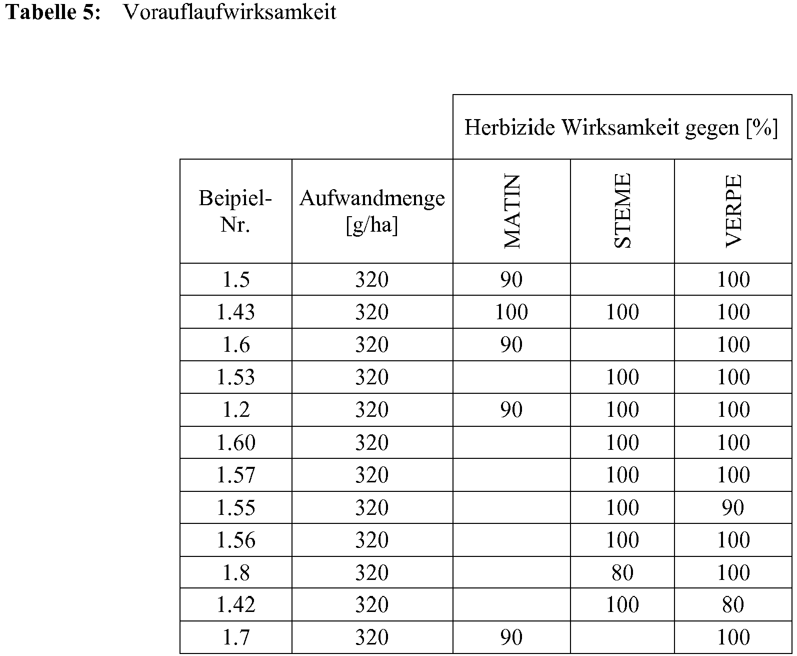

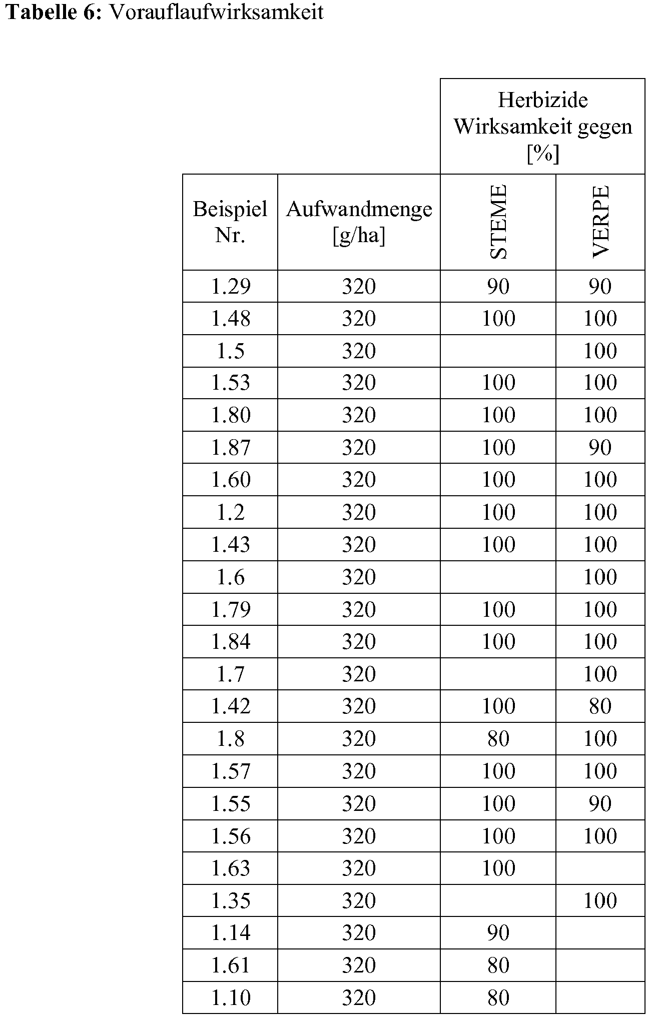

Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen aus den Tabellen 2 bis 6 sehr gute herbizide Wirkung gegen Schadpflanzen wie *Veronica persicam, Stellaria media* und *Matricaria inodora* im Vorauflaufverfahren bei einer Aufwandmenge von 320 g/ha und weniger Aktivsubstanz pro Hektar. Die erfindungsgemäßen Verbindungen eignen sich deshalb im Vorauflaufverfahren zur Bekämpfung von unerwünschtem Pflanzenwuchs.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 1/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

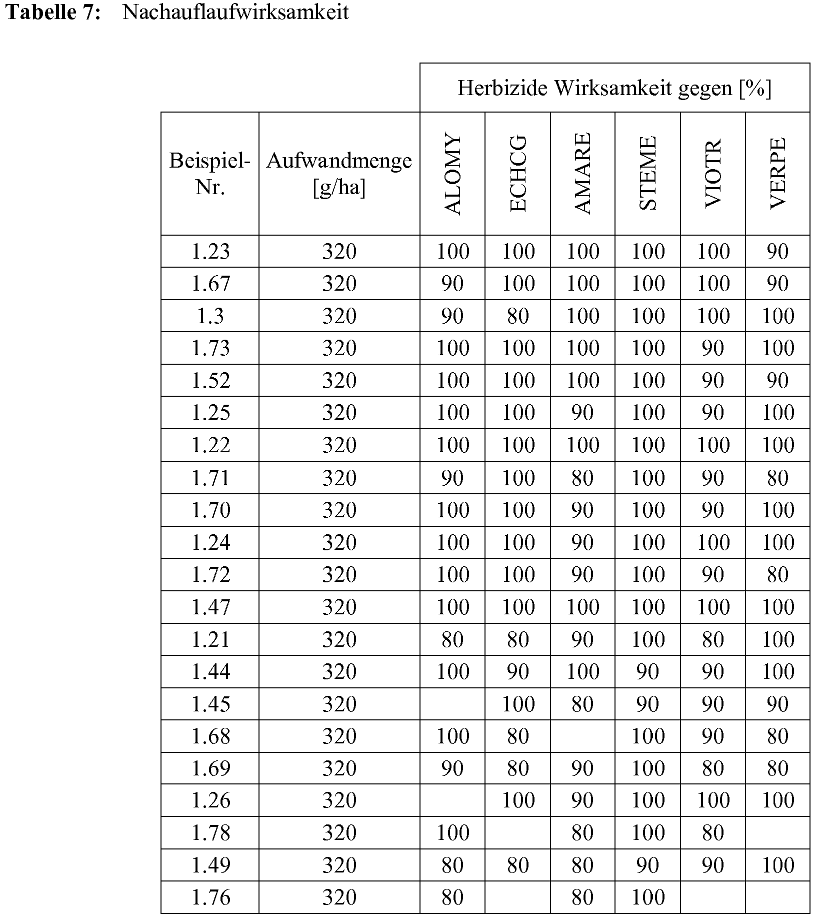

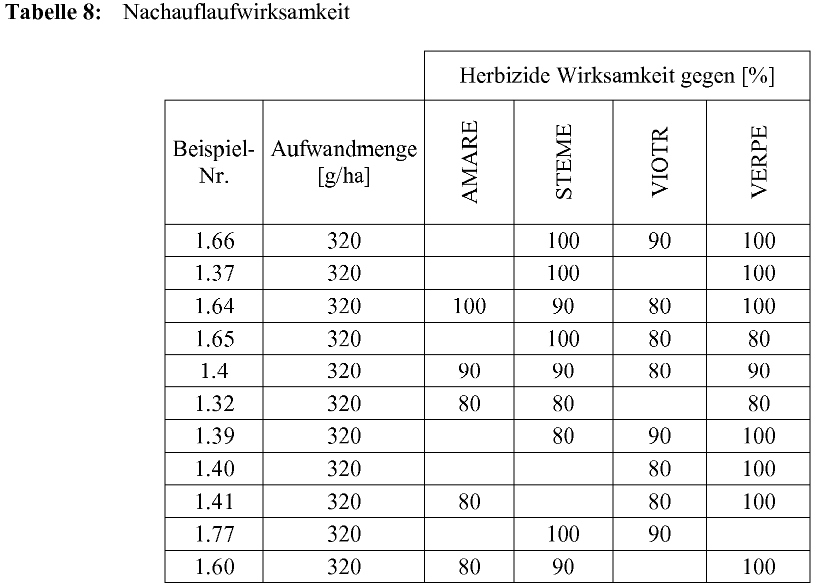

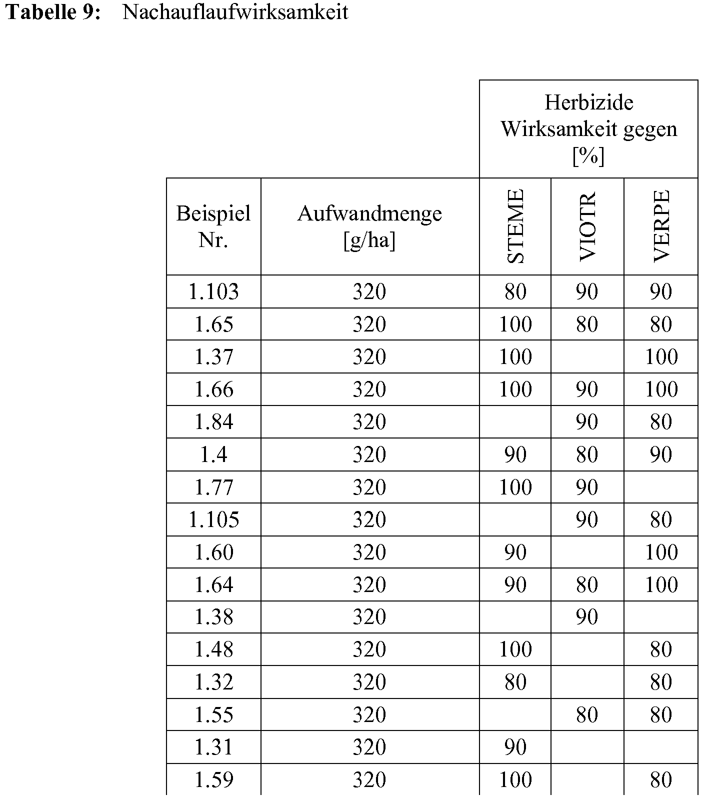

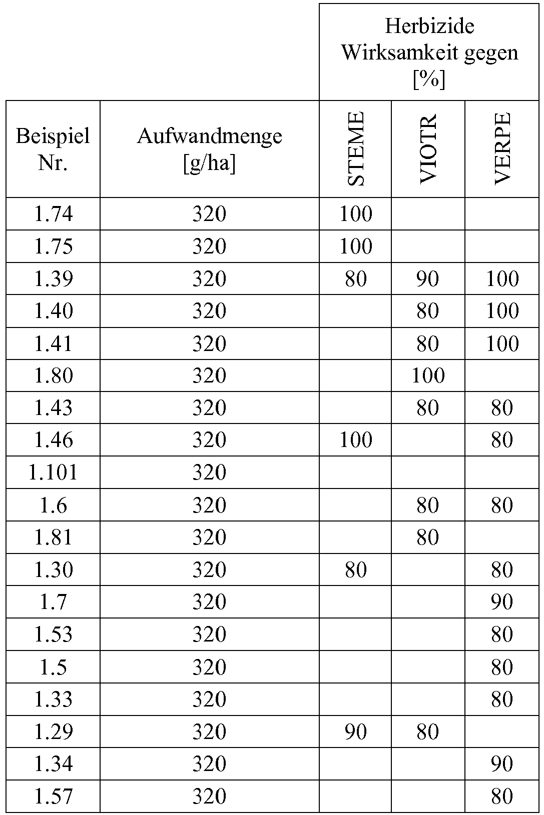

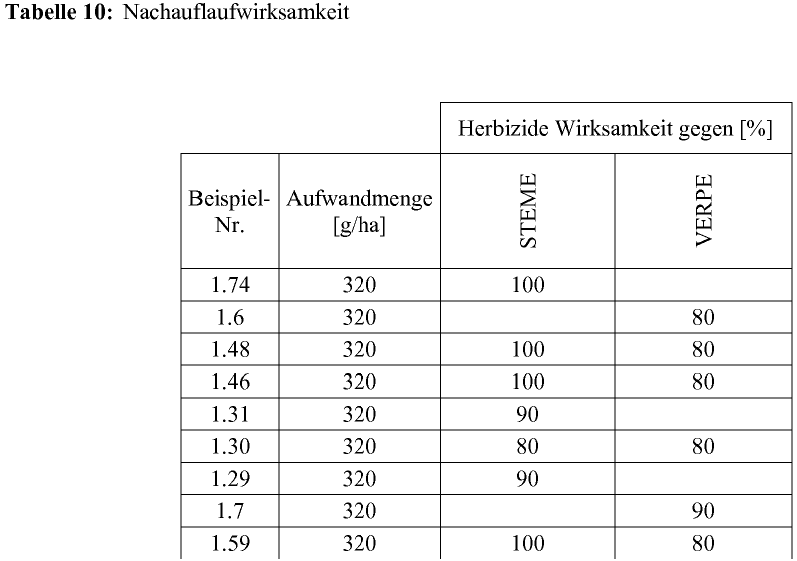

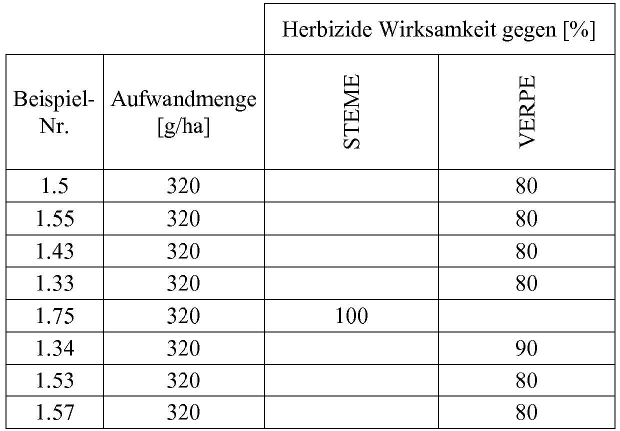

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise haben die Verbindungen aus den Tabellen 7 bis 10 sehr gute herbizide Wirkung gegen Schadpflanzen wie beispielsweise *Veronica persicam* und *Stellaria media* im Nachauflaufverfahren bei einer Aufwandmenge von 320 g/ha und weniger Aktivsubstanz pro Hektar. Die erfindungsgemäßen Verbindungen eignen sich deshalb im Nachauflaufverfahren zur Bekämpfung von unerwünschtem Pflanzenwuchs.

## Patentansprüche

1. Verbindungen der allgemeinen Formel (I) 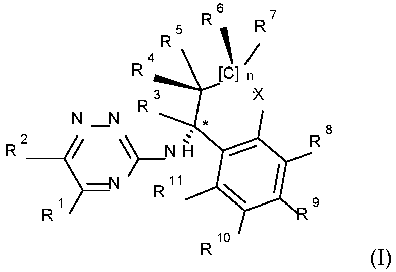 und deren agrochemisch verträglichen Salze, in welchen
R¹ Wasserstoff, Amino, Hydrazino, Hydroxyamino, ((C₁-C₆)-alkyl)-amino, ((C₃-C₆)-cycloalkyl)-amino, Di-((C₁-C₆)-alkyl)-amino, (C₁-C₆)-Alkylcarbonylamino, Bis-((C₁-C₆)-alkyl)-carbonylamino bedeutet;
R² ausgewählt ist aus der Gruppe, bestehend aus Wasserstoff, Halogen, Hydroxy, Nitro, Amino, Cyano, C(O)OH, C(O)NH₂; (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl; (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl; (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl; (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl; Tri-(C₁-C₆)-alkylsilyl-(C₂-C₆)-alkinyl, Di-(C₁-C₆)-alkylsilyl-(C₂-C₆)-alkinyl, (C₁-C₆)-alkylsilyl-(C₂-C₆)-alkinyl; Phenylsilyl-(C₂-C₆)-alkinyl; (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können; (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy; Aminocarbonyl-(C₁-C₆)-alkyl, Di-(C₁-C₆)-Alkylaminocarbonyl-(C₁-C₆)-alkyl; N-((C₁-C₆)-Haloalkanoyl)-amino-carbonyl, ((C₆-C₁₄)-aryl)amino-carbonyl, Di-((C₆-C₁₄)-aryl)-amino-carbonyl, ((C₁-C₆)-alkyl)amino-carbonyl, Di-((C₁-C₆)-alkyl)-amino-carbonyl, N-((C₁-C₆)-Alkylsulfonyl)-amino-carbonyl, N-((C₁-C₆)-Haloalkylsulfonyl)-amino-carbonyl, N-((C₆-C₁₄)-Arylsulfonyl)-amino-carbonyl; (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy; (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(Ci-C₆)-haloalkoxycarbonyloxy; (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl,(C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy; Hydroxy-(C₁-C₆)-alkyl, Hydroxy-(C₁-C₆)-alkoxy Cyano-(C₁-C₆)-alkoxy, Cyano-(C₁-C₆)-alkyl; und (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (Ci-C₆)-Alkylthio-(Ci-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfbnyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio, oder
R¹ mit R² über eine Bindung miteinander verbunden sein kann, so dass sich ein 5-bis 7-gliedriger teilhydrierter Carbocyclus oder Heterocyclus mit mindestens einem Heteroatom ausgewählt aus N, O, S und P ergibt, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert ist;
R³ ausgewählt ist aus der Gruppe bestehend aus Wasserstoff, (C₁-C₆)-Alkyl, (C₁-C₆)-Alkoxy, (C₁-C₆)-Alkoxy-(C₁-C₆)-Alkyl, (C₁-C₆)-Alkoxycarbonyl und Aminocarbonyl;
R⁴ und R⁵ jeweils unabhängig voneinander, ausgewählt sind aus der Gruppe, bestehend aus Wasserstoff, (C₁-C₆)-Alkyl, (C₁-C₆)-Haloalkyl, Hydroxy, (C₁-C₆)-Alkoxy und (C₁-C₆)-Halogenalkoxy; oder
R⁴ und R⁵ zusammen mit dem Kohlenstoffatom, an welchem sie gebunden sind, einen gesättigten drei- bis siebengliedrigen Ring bilden, der einen oder mehrere Heteroatome ausgewählt aus Sauerstoff, Schwefel und Stickstoff enthalten kann;
R⁶ und R⁷ jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-ArylO_{XY}, (C₆-C₁₄)-Arylcarbonyl und (C₆-C₁₄)-Aryloxycarbonyl; oder die Reste R⁶ und R⁷ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
n 0, 1 oder 2 ist;
R⁸, R⁹, R¹⁰ und R¹¹ jeweils unabhängig voneinander, ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro, wobei die Reste R⁹ und R¹⁰ ringbildend durch eine -O-CH₂-O- Gruppe verbunden sein können;
X für eine Bindung, CH₂, O, S, Carbonyl, NH, CR¹²R¹³,NR¹⁴,CH₂O oder CH₂S steht, wobei bei den beiden letztgenannten Gruppen das Kohlenstoffatom an den aromatischen Teil und das Heteroatom O oder S an den teilhydrierten Teil des Amins gebunden ist;
R¹² und R¹³ jeweils unabhängig voneinander, ausgewählt sind aus der Gruppe, bestehend aus Wasserstoff, (C₁-C₆)-Alkyl und (C₁-C₆)-Haloalkyl; und
R¹⁴ ausgewählt ist aus der Gruppe, bestehend aus Wasserstoff, (C₁-C₆)-Alkyl und (C₁-C₆)-Haloalkyl.

2. Verbindungen der allgemeinen Formel (I) nach Anspruch 1, **dadurch gekennzeichnet, dass** R¹ ausgewählt ist aus der Gruppe, bestehend aus Wasserstoff, Amino, ((C₁-C₆)-alkyl)amino, ((C₃-C₆)-cycloalkyl)amino, und Di-((C₁-C₆)-alkyl)amino.

3. Verbindungen der allgemeinen Formel (I) nach Anspruch 1 oder 2, **dadurch gekennzeichnet, dass** R² ausgewählt ist aus der Gruppe, bestehend aus
Wasserstoff, Halogen, Hydroxy, Nitro, Amino, Cyano, C(O)OH, C(O)NH₂;
(C₁-C₆)-Alkyl, (C₁-C₆)-Halogenalkyl;
(C₁-C₆)-Alkylcarbonyl, (C₁-C₆)-Haloalkylcarbonyl, (C₁-C₆)-Alkoxycarbonyl, (C₁-C₆)-Haloalkoxycarbonyl;
(C₂-C₆)-Alkenyl, (C₂-C₆)-Halogenalkenyl;
(C₂-C₆)-Alkinyl, (C₂-C₆)-Halogenalkinyl;
(C₆-C₁₄)-Aryl, welches am Arylteil mit Halogen, (C₁-C₆)-Alkyl und/oder (C₁-C₆)-Haloalkyl substituiert sein kann;
(C₁-C₆)-Alkoxy-(C₁-C₆)-Alkyl, Hydroxy-(C₁-C₆)-alkyl;
((C₁-C₆)-alkyl)amino-carbonyl, Di-((C₁-C₆)-alkyl)aminocarbonyl,
N-((C₁-C₆)-Alkylsulfonyl)amino-carbonyl, N-((C₁-C₆)-Haloalkylsulfonyl)amino-carbonyl, (C₃-C₈)-Cycloalkylcarbonyl, (C₃-C₈)-Cycloalkoxycarbonyl;
(C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylsulfinyl, (C₄-C₁₄)-Arylsulfonyl und (C₆-C₁₄)-Arylsulfinyl.

4. Verbindungen der allgemeinen Formel (I) nach einem der Ansprüche 1 bis 3, **dadurch gekennzeichnet, dass** R³ Wasserstoff ist.

5. Verbindungen der allgemeinen Formel (I) nach einem der Ansprüche 1 bis 4, **dadurch gekennzeichnet, dass** R⁴ und R⁵ jeweils unabhängig voneinander ausgewählt sind aus der Gruppe, bestehend aus Wasserstoff, Hydroxy, (C₁-C₆)-Alkyl, (C₁-C₆)-Alkylphenyl und (C₁-C₆)-Alkoxy.

6. Verbindungen der allgemeinen Formel (I) nach einem der Ansprüche 1 bis 5, **dadurch gekennzeichnet, dass** R⁴ und R⁵ zusammen mit dem Kohlenstoffatom, an welchem sie gebunden sind, einen gesättigten drei- bis siebengliedrigen Ring bilden, der ein Sauerstoffatom enthalten kann.

7. Verbindungen der allgemeinen Formel (I) nach einem der Ansprüche 1 bis 6, **dadurch gekennzeichnet, dass** R⁶ und R⁷ unabhängig voneinander, ausgewählt sind aus der Gruppe, bestehend aus Wasserstoff, (C₁-C₆)-Alkyl und (C₆-C₁₄)-Aryl.

8. Verbindungen der allgemeinen Formel (I) nach einem der Ansprüche 1 bis 7, **dadurch gekennzeichnet, dass** R⁸ ausgewählt ist aus der Gruppe, bestehend aus Wasserstoff, Halogen, (C₁-C₆)-Alkyl, (C₁-C₆)-Alkoxy und (C₆-C₁₄)-Aryl.

9. Verbindungen der allgemeinen Formel (I) nach einem der Ansprüche 1 bis 8, **dadurch gekennzeichnet, dass** R⁹ ausgewählt ist aus der Gruppe, bestehend aus Wasserstoff, Halogen, (C₁-C₆)-Alkyl und (C₁-C₆)-Alkoxy.

10. Verbindungen der allgemeinen Formel (I) nach einem der Ansprüche 1 bis 9, **dadurch gekennzeichnet, dass** R¹⁰ ausgewählt ist aus der Gruppe, bestehend aus aus Wasserstoff, Halogen, Cyano, Aminocarbonyl, Hydroxycarbonyl, (C₁-C₆)-Alkyl, (C₁-C₆)-Haloalkyl, (C₁-C₆)-Alkoxy, (C₂-C₆)-Alkenyl, (C₂-C₆)-Alkinyl, (C₁-C₆)-Alkyl-(C₂-C₆)-alkinyl, Hydroxy-(C₁-C₆)-alkyl-(C₂-C₆)-alkinyl, (C₁-C₆)-Alkoxy-(C₂-C₆)-alkinyl und Aryl-(C₂-C₆)-alkinyl.

11. Verbindungen der allgemeinen Formel (I) nach einem der Ansprüche 1 bis 10, **dadurch gekennzeichnet, dass** R¹¹ ausgewählt ist aus der Gruppe, bestehend aus Wasserstoff und (C₁-C₆)-Alkyl.

12. Verbindungen der allgemeinen Formel (I) nach einem der Ansprüche 1 bis 11, **dadurch gekennzeichnet, dass** X ausgewählt ist aus der Gruppe bestehend aus einer chemischen Bindung, CH₂, O, S, Carbonyl, NH, CH(C₁-C₆)Alkyl, N(C₁-C₆)Alkyl, OCH₂, und SCH₂, wobei bei den beiden letztgenannten Gruppen das Kohlenstoffatom an den aromatischen Teil und das Heteroatom O oder S an den teilhydrierten Teil des Amins gebunden ist.

13. Verbindungen der allgemeinen Formel (I) nach einem der Ansprüche 1 bis 12, **dadurch gekennzeichnet, dass** die Laufzahl n 0 oder 1 beträgt.

14. Verbindungen der allgemeinen Formel (I) nach einem der Ansprüche 1 bis 13, **dadurch gekennzeichnet, dass**
R¹ ausgewählt ist aus der Gruppe, bestehend aus Wasserstoff, Amino, ((C₁-C₃)-alkyl)amino, ((C₃-C₆)-cycloalkyl)amino und Di((C₁-C₃)-alkyl)amino;
R² ausgewählt ist aus der Gruppe, bestehend aus Cl, Br, Cyano, C(O)OH, C(O)NH₂; (C₁-C₆)-Alkyl, (C₁-C₆)-Halogenalkyl, (C₁-C₆)-Alkylcarbonyl, (C₁-C₆)-Haloalkylcarbonyl; (C₁-C₆)-Alkoxycarbonyl, (C₁-C₆)-Haloalkoxycarbonyl; (C₂-C₆)-Alkenyl, (C₂-C₆)-Halogenalkenyl: (C₂-C₆)-Alkinyl, (C₂-C₆)-Halogenalkinyl; Phenyl, welches mit F, Cl, Br, Methyl und/oder Trifluormethyl substituiert sein kann; (C₁-C₆)-Alkoxy-(C_{I}-C₆)-alkyl und Hydroxy-(C₁-C₆)-alkyl; ((C₁-C₂)-alkyl-amino-carbonyl, Di-((C₁-C₂)-alkyl)amino-carbonyl, N-((C₁-C₂)-Alkylsulfonyl)amino-carbonyl, N-((C₁-C₂)-Haloalkylsulfonyl)amino-carbonyl; (C₃-C₆)-Cycloalkylcarbonyl, (C₃-C₆)-Cycloalkoxycarbonyl; (C₁-C₃)-Alkylsulfonyl, (C₁-C₃)-Alkylsulfinyl, (C₁-C₃)-Haloalkylsulfonyl, (C₁-C₆)-Halogenalkylsulfinyl, (C₄-C₁₄)-Arylsulfbnyl und (C₆-C₁₄)-Arylsulfinyl;
R³ Wasserstoff ist;
R⁴ und R⁵ jeweils unabhängig voneinander ausgewählt sind aus der Gruppe, bestehend aus Wasserstoff, (C₁-C₆)-Alkyl und (C₁-C₆)-Alkoxy; oder
R⁴ und R⁵ zusammen mit dem Kohlenstoff, an welchen sie gebunden sind, einen gesättigten drei- bis sechsgliedrigen Ring bilden, der ein Sauerstoffatom enthalten kann;
R⁶ und R⁷ unabhängig voneinander ausgewählt sind aus der Gruppe, bestehend aus Wasserstoff, Methyl und Phenyl;
R^{S} ausgewählt ist aus der Gruppe, bestehend aus Wasserstoff, Halogen, (C₁-C₃)-Alkyl und (C₆-C₈)-Aryl;
R⁹ ausgewählt ist aus der Gruppe, bestehend aus Wasserstoff, Chlor, Fluor und (C₁-C₃)-Alkoxy;
R¹⁰ ausgewählt ist aus der Gruppe, bestehend aus Wasserstoff, Fluor, Chlor, Brom, Iod, Cyano, Methoxy (OCH₃), (C₁-C₃)-Alkyl, (C₁-C₃)-Haloalkyl, (C₁-C₃)-Alkoxy, (C₂-C₄)-Alkenyl, (C₂-C₄)-Alkinyl, (C₁-C₃)-Alkyl-(C₂-C₄)-alkinyl, Hydroxy-(C₁-C₃)-Alkyl-(C₂-C₄)-alkinyl, (C₁-C₃)-Alkoxy-(C₂-C₄)-alkinyl und Phenyl-(C₂-C₄)-alkinyl;
R¹¹ ausgewählt ist aus der Gruppe, bestehend aus Wasserstoff und (C₁-C₄)-Alkyl;
X ausgewählt ist aus der Gruppe bestehend aus einer chemischen Bindung, CH₂, O, S, CHCH₃, NCH₃, OCH₂, und SCH₂ wobei bei den beiden letztgenannten Gruppen das Kohlenstoffatom an den aromatischen Teil und das Heteroatom O oder S an den teilhydrierten Teil des Amins gebunden ist; und
n 0 oder 1 beträgt.

15. Verbindungen der allgemeinen Formel (I) 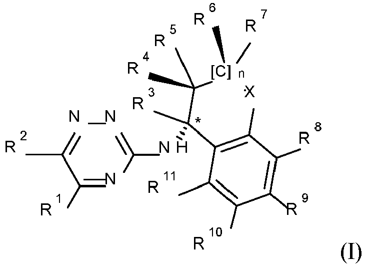 nach einem der Ansprüche 1 bis 14, **dadurch gekennzeichnet, dass** das chirale Kohlenstoffatom mit der Kennzeichnung (*) eine (R)-Konfiguration aufweist.

16. Verbindungen der allgemeinen Formel (I) 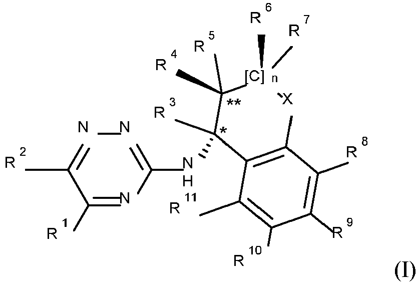 nach einem der Ansprüche 1 bis 14, **dadurch gekennzeichnet, dass** das chirale Kohlenstoffatom mit der Kennzeichnung (*) eine (R)-Konfiguration und das chirale Kohlenstoffatom mit der Kennzeichnung (**) eine (S)-Konfiguration aufweist.

17. Verfahren zur Herstellung von Verbindungen der allgemeinen Formel (I) oder deren agrochemisch verträgliche Salze: 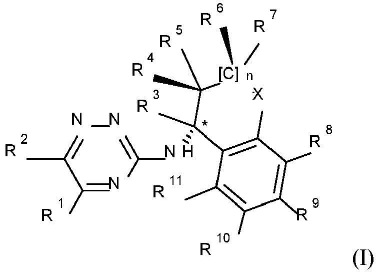 worin die Reste R¹ bis R¹¹ und X sowie n wie in einem der Ansprüche 1 bis 16 definiert sind, und wobei
- eine Verbindung der allgemeinen Formel (II) 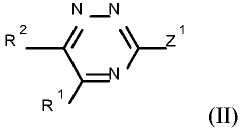 wobei R¹, R² gemäß einem der Ansprüche 1 bis 16 definiert sind und Z¹ für einen austauschfähigen Rest oder eine Abgangsgruppe steht, ausgewählt aus der Gruppe, bestehend aus Fluor, Chlor, Brom, Jod, ein (C₁-C₄)-Alkylsulfanyl, ein (C₁-C₄)-Alkylsulfinyl, ein (C₁-C₄)-Alkylsulfonyl, ein unsubstituiertes oder ein einfach oder mehrfach mit Fluor, Chlor, Brom oder (C₁-C₄)-Alkyl, ein (C₁-C₄)-Alkoxy substituiertes Phenyl-(C₁-C₄)-alkylsulfonyl und ein (C₁-C₄)-Alkylphenyl-sulfonyl;
- mit einem Amin der allgemeinen Formel (III) oder einem Säureadditionssalz des Amins der allgemeinen Formel (III) 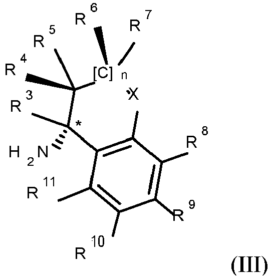 umgesetzt wird,
wobei die Reste R³ bis R¹¹ sowie X und n gemäß den Ansprüchen 1 bis 16 definiert sind.

18. Verfahren zur Herstellung von Verbindungen der allgemeinen Formel (I): 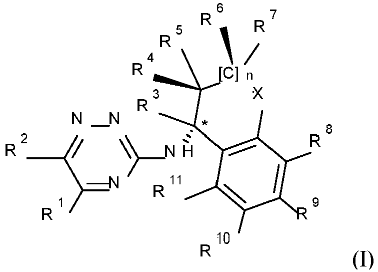 worin die Reste R¹ bis R¹¹ und X sowie n wie in einem der Ansprüche 1 bis 16 definiert sind, und wobei
- eine Verbindung der allgemeinen Formel (II-a), 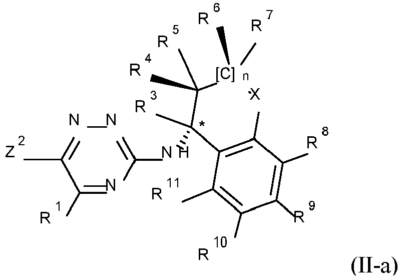 wobei Z² ausgewählt ist aus der Gruppe, bestehend aus Halogen, Cyano, (C₁-C₆)-Alkoxycarbonyl, (C₁-C₆)-Alkylcarbonyl und (C₁-C₆)-Alkylthio, gemäß dem Verfahren nach Anspruch 17 hergestellt wird, und anschließend
- der Rest Z² nach bekannten Verfahren zu einen Rest R² umgesetzt wird, wobei R² gemäß einem der Ansprüche 1 bis 16 definiert ist.

19. Verfahren zur Herstellung von Verbindungen der allgemeinen Formel (I) oder deren agrochemisch verträglichen Salze: 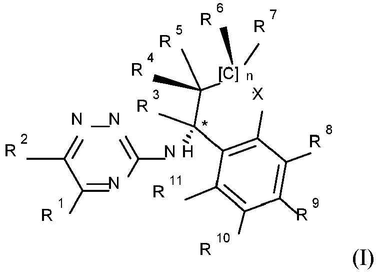 worin R¹ Amino bedeutet und die Reste R² bis R¹¹ und X sowie n wie in einem der Ansprüche 1 bis 16 definiert sind, und wobei
- eine Guanidineverbindung der allgemeinen Formel (IV) oder eines Säureadditionssalzes davon 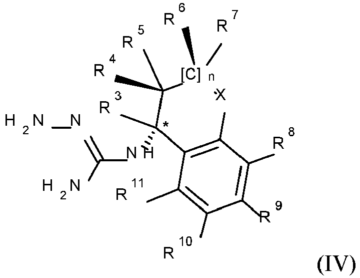
- mit Acylcyaniden der Formel (V) 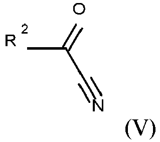 kondensiert werden, wobei der Rest R² wie in den Ansprüchen 1 bis 16 definiert ist.

20. Herbizides Mittel oder pflanzenwachstumsregulierendes Mittel, **dadurch gekennzeichnet, dass** es eine oder mehrere Verbindungen der allgemeinen Formel (I) oder deren Salze nach einem der Ansprüche 1 bis 16 enthält.

21. Verfahren zur Bekämpfung von Schadpflanzen oder zur Wachstumsregulierung von Pflanzen, **dadurch gekennzeichnet, dass** man eine wirksame Menge von einer oder mehreren Verbindungen der allgemeinen Formel (I) oder deren Salzen nach einem der Ansprüche 1 bis 16 auf Pflanzen, Pflanzenteile, Pflanzensamen oder auf eine Anbaufläche appliziert.

22. Verwendung von Verbindungen der allgemeinen Formel (I) oder deren Salzen nach einem der Ansprüche 1 bis 16 als Herbizide oder als Pflanzenwachstumsregulatoren.

23. Verwendung nach Anspruch 22, **dadurch gekennzeichnet, dass** die Verbindungen der allgemeinen Formel (I) oder deren Salze zur Bekämpfung von Schadpflanzen oder zur Wachstumsregulierung von Pflanzen in Kulturen von Nutz- oder Zierpflanzen eingesetzt werden.

24. Verwendung nach den Ansprüchen 22 oder 23, **dadurch gekennzeichnet, dass** die Kulturpflanzen transgene Kulturpflanzen sind.

## Claims

1. Compounds of the general formula (I) 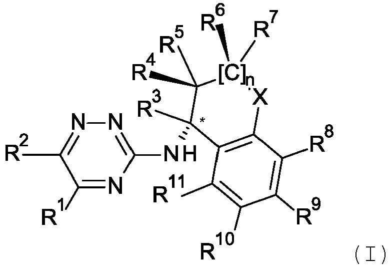 and the agrochemically acceptable salts thereof in which
R¹ represents hydrogen, amino, hydrazino, hydroxyamino, ((C₁-C₆)-alkyl)-amino, ((C₃-C₆)-cycloalkyl)-amino, di-((C₁-C₆)-alkyl)-amino, (C₁-C₆)-alkylcarbonylamino, bis-((C₁-C₆)-alkyl)-carbonylamino;
R² is selected from the group consisting of hydrogen, halogen, hydroxy, nitro, amino, cyano, C(O)OH, C(O)NH₂; (C₁-C₆)-alkyl, (C₁-C₆)-haloalkyl, (C₁-C₆)-alkylcarbonyl, (C₁-C₆)-haloalkylcarbonyl, (C₁-C₆)-alkylcarbonyloxy, (C₁-C₆)-haloalkylcarbonyloxy, (C₁-C₆)-alkylcarbonyl-(C₁-C₄)-alkyl; (C₁-C₆)-alkoxy, (C₁-C₆)-haloalkoxy, (C₁-C₆)-alkoxycarbonyl, (C₁-C₆)-haloalkoxycarbonyl, (C₁-C₆)-alkoxycarbonyl-(C₁-C₆)-alkyl, (C₁-C₆)-haloalkoxycarbonyl-(C₁-C₆)-alkyl, (C₁-C₆)-alkoxycarbonyl-(C₁-C₆)-haloalkyl, (C₁-C₆)-haloalkoxycarbonyl-(C₁-C₆)-haloalkyl; (C₂-C₆)-alkenyl, (C₂-C₆)-haloalkenyl, (C₂-C₆)-alkenylcarbonyl, (C₂-C₆)-haloalkenylcarbonyl, (C₂-C₆)-alkenyloxy, (C₂-C₆)-haloalkenyloxy, (C₂-C₆)-alkenyloxycarbonyl, (C₂-C₆)-haloalkenyloxycarbonyl; (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl; tri-(C₁-C₆)-alkylsilyl-(C₂-C₆)-alkynyl, di-(C₁-C₆)-alkylsilyl- (C₂-C₆)-alkynyl, (C₁-C₆)-alkylsilyl-(C₂-C₆)-alkynyl; phenylsilyl-(C₂-C₆)-alkynyl; (C₆-C₁₄)-aryl, (C₆-C₁₄)-aryloxy, (C₆-C₁₄)-arylcarbonyl and (C₆-C₁₄)-aryloxycarbonyl, each of which may be substituted in the aryl moiety by halogen, (C₁-C₆)-alkyl and/or (C₁-C₆)-haloalkyl; (C₆-C₁₄)-aryl- (C₁-C₆)-alkyl, (C₆-C₁₄)-aryl-(C₁-C₆)-alkoxy, (C₆-C₁₄)-aryl-(C₁-C₆)-alkylcarbonyl, (C₆-C₁₄)-aryl-(C₁-C₆)-alkylcarbonyloxy, (C₆-C₁₄)-aryl-(C₁-C₆)-alkoxycarbonyl, (C₆-C₁₄)-aryl- (C₁-C₆)-alkoxycarbonyloxy; aminocarbonyl- (C₁-C₆)-alkyl, di-(C₁-C₆)-alkylaminocarbonyl-(C₁-C₆)-alkyl; N-((C₁-C₆)-haloalkanoyl) aminocarbonyl, ((C₆-C₁₄)-aryl) aminocarbonyl, di-((C₆-C₁₄)-aryl)aminocarbonyl, ((C₁-C₆)-alkyl)aminocarbonyl, di-((C₁-C₆)-alkyl)aminocarbonyl, N-((C₁-C₆)-alkylsulfonyl)aminocarbonyl, N-((C₁-C₆)-haloalkylsulfonyl) aminocarbonyl, N-((C₆-C₁₄)-arylsulfonyl)aminocarbonyl; (C₁-C₆)-alkoxy- (C₁-C₆)-alkyl, (C₁-C₆)-alkoxy-(C₁-C₆)-alkoxy, (C₁-C₆)-alkoxycarbonyl-(C₁-C₆)-alkoxy; (C₃-C₈)-cycloalkyl which may optionally be substituted in the cycloalkyl radical by (C₁-C₆)-alkyl and/or halogen; (C₃-C₈)-cycloalkoxy, (C₃-C₈)-cycloalkyl-(C₁-C₆)-alkyl, (C₃-C₈)-cycloalkyl- (C₁-C₆)-haloalkyl, (C₃-C₈)-cycloalkyl- (C₁-C₆)-alkoxy, (C₃-C₈)-cycloalkyl-(C₁-C₆)-haloalkoxy, (C₃-C₈)-cycloalkylcarbonyl, (C₃-C₈)-cycloalkoxycarbonyl, (C₃-C₈)-cycloalkyl-(C₁-C₆)-alkylcarbonyl, (C₃-C₈)-cycloalkyl-(C₁-C₆)-haloalkylcarbonyl, (C₃-C₈)-cycloalkyl-(C₁-C₆)-alkoxycarbonyl, (C₃-C₈)-cycloalkyl-(C₁-C₆)-haloalkoxycarbonyl, (C₃-C₈)-cycloalkylcarbonyloxy, (C₃-C₈)-cycloalkoxycarbonyloxy, (C₃-C₈)-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl-(C₁-C₆)-alkoxycarbonyloxy, (C₃-C₈)-cycloalkyl-(C₁-C₆)-haloalkoxycarbonyloxy; (C₃-C₈)-cycloalkenyl, (C₃-C₈) cycloalkenyloxy, (C₃-C₈)-cycloalkenyl-(C₁-C₆)-alkyl, (C₃-C₈)-cycloalkenyl-(C₁-C₆)-haloalkyl, (C₃-C₈)-cycloalkenyl-(C₁-C₆)-alkoxy, (C₃-C₈)-cycloalkenyl-(C₁-C₆)-haloalkoxy, (C₃-C₈)-cycloalkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl-(C₁-C₆)-haloalkylcarbonyl, (C₃-C₈)-cycloalkenyl- (C₁-C₆)-alkoxycarbonyl, (C₃-C₈)-cycloalkenyl- (C₁-C₆)-haloalkoxycarbonyl, (C₃-C₈)-cycloalkenylcarbonyloxy, (C₃-C₈)-cycloalkenyloxycarbonyloxy, (C₃-C₈)-cycloalkenyl-(C₁-C₆)-alkylcarbonyloxy, (C₃-C₈)-cycloalkenyl-(C₁-C₆)-haloalkylcarbonyloxy, (C₃-C₈)-cycloalkenyl-(C₁-C₆)-alkoxycarbonyloxy, (C₃-C₈)-cycloalkenyl-(C₁-C₆)-haloalkoxycarbonyloxy; hydroxy-(C₁-C₆)-alkyl, hydroxy-(C₁-C₆)-alkoxy, cyano-(C₁-C₆)-alkoxy, cyano-(C₁-C₆)-alkyl; and (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆)-alkylsulfonyl-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio-(C₁-C₆)-haloalkyl, (C₁-C₆)-haloalkylsulfinyl-(C₁-C₆)-haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆)-alkylthiocarbonyloxy, (C₁-C₆)-haloalkylthiocarbonyloxy, (C₁-C₆)-alkylthio-(C₁-C₆)-alkyl, (C₁-C₆)-alkylthio-(C₁-C₆)-alkoxy, (C₁-C₆)-alkylthio-(C₁-C₆)-alkylcarbonyl, (C₁-C₆)-alkylthio-(C₁-C₆)-alkylcarbonyloxy; (C₄-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio and (C₃-C₆)-alkynylthio; or
R¹ may be attached to R² via a bond, resulting in a 5-to 7-membered partially hydrogenated carbocycle or heterocycle having at least one heteroatom selected from N, O, S and P, which carbocycle or heterocycle is optionally substituted by one or more substituents selected from the group consisting of hydroxy, =O, =N-O-H, =N-O-(C₁-C₆)-alkyl, =N-O-benzyl, =N-O-phenyl, phenyl, phenyl substituted by one or more identical or different halogen atoms, (C₁-C₆)-alkyl, (C₃-C₆)-cycloalkyl and (C₁-C₆)-haloalkyl;
R³ is selected from the group consisting of hydrogen, (C₁-C₆)-alkyl, (C₁-C₆)-alkoxy, (C₁-C₆)-alkoxy-(C₁-C₆)-alkyl, (C₁-C₆)-alkoxycarbonyl and aminocarbonyl;
R⁴ and R⁵ are each independently of one another selected from the group consisting of hydrogen, (C₁-C₆)-alkyl, (C₁-C₆)-haloalkyl, hydroxy, (C₁-C₆)-alkoxy and (C₁-C₆)-haloalkoxy; or
R⁴ and R⁵ together with the carbon atom to which they are attached form a saturated three- to seven-membered ring which may contain one or more heteroatoms selected from oxygen, sulfur and nitrogen;
R⁶ and R⁷ are each independently of one another selected from the group consisting of hydrogen, (C₁-C₆)-alkyl, (C₁-C₆)-haloalkyl, (C₁-C₆)-alkoxy, (C₁-C₆)-haloalkoxy, (C₆-C₁₄)-aryl, (C₆-C₁₄)-aryloxy, (C₆-C₁₄)-arylcarbonyl and (C₆-C₁₄)-aryloxycarbonyl; or the R⁶ and R⁷ radicals together form a (C₁-C₇)-alkylene group which may contain one or more oxygen and/or sulfur atoms, where the (C₁-C₇)-alkylene group may be mono- or polysubstituted by halogen and the respective halogen substituents may be identical or different;
n is 0, 1 or 2;
R⁸, R⁹, R¹⁰ and R¹¹ are each independently of one another selected from the group consisting of hydrogen, halogen, cyano, C(O)OH, C(O)NH₂, (C₁-C₆)-alkyl, (C₁-C₆)-alkylcarbonyl, (C₁-C₆)-alkyloxycarbonyl, (C₁-C₆)-alkylaminocarbonyl, (C₁-C₆)-dialkylaminocarbonyl, (C₁-C₆)-haloalkyl, (C₁-C₆)-alkoxy, (C₁-C₆)-haloalkoxy, (C₂-C₆)-alkenyl, (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl and nitro, where the R⁹ and R¹⁰ radicals may be attached to one another via an -O-CH₂-O- group forming a ring;
X represents a bond, CH₂, O, S, carbonyl, NH, CR¹² R¹³, NR¹⁴, CH₂O or CH₂S, where in the two last-mentioned groups the carbon atom is attached to the aromatic moiety and the heteroatom O or S is attached to the partially hydrogenated moiety of the amine;
R¹² and R¹³ are each independently of one another selected from the group consisting of hydrogen, (C₁-C₆)-alkyl and (C₁-C₆)-haloalkyl; and
R¹⁴ is selected from the group consisting of hydrogen, (C₁-C₆)-alkyl and (C₁-C₆)-haloalkyl.

2. Compounds of the general formula (I) according to Claim 1, **characterized in that** R¹ is selected from the group consisting of hydrogen, amino, ((C₁-C₆)-alkyl) amino, ((C₃-C₆)-cycloalkyl)amino and di-((C₁-C₆)-alkyl) amino.

3. Compounds of the general formula (I) according to Claim 1 or 2, **characterized in that** R² is selected from the group consisting of
hydrogen, halogen, hydroxy, nitro, amino, cyano, C(O)OH, C(O)NH₂;
(C₁-C₆)-alkyl, (C₁-C₆)-haloalkyl;
(C₁-C₆)-alkylcarbonyl, (C₁-C₆)-haloalkylcarbonyl, (C₁-C₆)-alkoxycarbonyl, (C₁-C₆)-haloalkoxycarbonyl; (C₂-C₆)-alkenyl, (C₂-C₆)-haloalkenyl;
(C₂-C₆)-alkynyl, (C₂-C₆)-haloalkynyl;
(C₆-C₁₄)-aryl which may be substituted in the aryl moiety by halogen, (C₁-C₆)-alkyl and/or (C₁-C₆)-haloalkyl;
(C₁-C₆)-alkoxy-(C₁-C₆)-alkyl, hydroxy-(C₁-C₆)-alkyl; .((C₁-C₆)-alkyl) aminocarbonyl, di-((C₁-C₆)-alkyl)aminocarbonyl,
N-((C₁-C₆)-alkylsulfonyl) aminocarbonyl, N-((C₁-C₆)-haloalkylsulfonyl)aminocarbonyl, (C₃-C₈)-cycloalkylcarbonyl, (C₃-C₈)-cycloalkoxycarbonyl; (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylsulfinyl, (C₄-C₁₄)-arylsulfonyl and (C₆-C₁₄)-arylsulfinyl.

4. Compounds of the general formula (I) according to any of Claims 1 to 3, **characterized in that** R³ is hydrogen.

5. Compounds of the general formula (I) according to any of Claims 1 to 4, **characterized in that** R⁴ and R⁵ are each independently of one another selected from the group consisting of hydrogen, hydroxy, (C₁-C₆)-alkyl, (C₁-C₆)-alkylphenyl and (C₁-C₆)-alkoxy.

6. Compounds of the general formula (I) according to any of Claims 1 to 5, **characterized in that** R⁴ and R⁵ together with the carbon atom to which they are attached form a saturated three- to seven-membered ring which may contain an oxygen atom.

7. Compounds of the general formula (I) according to any of Claims 1 to 6, **characterized in that** R⁶ and R⁷ are independently of one another selected from the group consisting of hydrogen, (C₁-C₆)-alkyl and (C₆-C₁₄)-aryl.

8. Compounds of the general formula (I) according to any of Claims 1 to 7, **characterized in that** R⁸ is selected from the group consisting of hydrogen, halogen, (C₁-C₆)-alkyl, (C₁-C₆)-alkoxy and (C₆-C₁₄)-aryl.

9. Compounds of the general formula (I) according to any of Claims 1 to 8, **characterized in that** R⁹ is selected from the group consisting of hydrogen, halogen, (C₁-C₆)-alkyl and (C₁-C₆)-alkoxy.

10. Compounds of the general formula (I) according to any of Claims 1 to 9, **characterized in that** R¹⁰ is selected from the group consisting of hydrogen, halogen, cyano, aminocarbonyl, hydroxycarbonyl, (C₁-C₆)-alkyl, (C₁-C₆)-haloalkyl, (C₁-C₆)-alkoxy, (C₂-C₆)-alkenyl, (C₂-C₆)-alkynyl, (C₁-C₆)-alkyl-(C₂-C₆)-alkynyl, hydroxy-(C₁-C₆)-alkyl-(C₂-C₆)-alkynyl, (C₁-C₆)-alkoxy-(C₂-C₆)-alkynyl and aryl-(C₂-C₆)-alkynyl.

11. Compounds of the general formula (I) according to any of Claims 1 to 10, **characterized in that** R¹¹ is selected from the group consisting of hydrogen and (C₁-C₆) -alkyl.

12. Compounds of the general formula (I) according to any of Claims 1 to 11, **characterized in that** X is selected from the group consisting of a chemical bond, CH₂, O, S, carbonyl, NH, CH(C₁-C₆)-alkyl, N(C₁-C₆)-alkyl, OCH₂ and SCH₂, where in the two last-mentioned groups the carbon atom is attached to the aromatic moiety and the heteroatom O or S is attached to the partially hydrogenated moiety of the amine.

13. Compounds of the general formula (I) according to any of Claims 1 to 12, **characterized in that** the running number n is 0 or 1.

14. Compounds of the general formula (I) according to any of Claims 1 to 13, **characterized in that**
R¹ is selected from the group consisting of hydrogen, amino, ((C₁-C₃)-alkyl) amino, ((C₃-C₆)-cycloalkyl) amino and di-((C₁-C₃)-alkyl)amino;
R² is selected from the group consisting of Cl, Br, cyano, C(O)OH, C(O)NH₂; (C₁-C₆)-alkyl, (C₁-C₆)-haloalkyl, (C₁-C₆)-alkylcarbonyl, (C₁-C₆)-haloalkylcarbonyl; (C₁-C₆)-alkoxycarbonyl, (C₁-C₆)-haloalkoxycarbonyl; (C₂-C₆)-alkenyl, (C₂-C₆)-haloalkenyl: (C₂-C₆)-alkynyl, (C₂-C₆)-haloalkynyl; phenyl which may be substituted by F, Cl, Br, methyl and/or trifluoromethyl; (C₁-C₆)-alkoxy-(C₁-C₆)-alkyl and hydroxy-(C₁-C₆)-alkyl; ((C₁-C₂)-alkyl) aminocarbonyl, di-((C₁-C₂)-alkyl)aminocarbonyl, N-((C₁-C₂)-alkylsulfonyl)aminocarbonyl, N-((C₁-C₂)-haloalkylsulfonyl)aminocarbonyl; (C₃-C₆)-cycloalkylcarbonyl, (C₃-C₆)-cycloalkoxycarbonyl; (C₁-C₃)-alkylsulfonyl, (C₁-C₃)-alkylsulfinyl, (C₁-C₃)-haloalkylsulfonyl, (C₁-C₆)-haloalkylsulfinyl, (C₄-C₁₄)-arylsulfonyl and (C₆-C₁₄)-arylsulfinyl;
R³ is hydrogen;
R⁴ and R⁵ are each independently of one another selected from the group consisting of hydrogen, (C₁-C₆)-alkyl and (C₁-C₆)-alkoxy; or
R⁴ and R⁵ together with the carbon to which they are attached form a saturated three- to six-membered ring which may contain an oxygen atom;
R⁶ and R⁷ are independently of one another selected from the group consisting of hydrogen, methyl and phenyl;
R⁸ is selected from the group consisting of hydrogen, halogen, (C₁-C₃)-alkyl and (C₆-C₈)-aryl;
R⁹ is selected from the group consisting of hydrogen, chlorine, fluorine and (C₁-C₃)-alkoxy;
R¹⁰ is selected from the group consisting of hydrogen, fluorine, chlorine, bromine, iodine, cyano, methoxy (OCH₃), (C₁-C₃)-alkyl, (C₁-C₃)-haloalkyl, (C₁-C₃)-alkoxy, (C₂-C₄)-alkenyl, (C₂-C₄)-alkynyl, (C₁-C₃)-alkyl-(C₂-C₄)-alkynyl, hydroxy-(C₁-C₃)-alkyl-(C₂-C₄)-alkynyl, (C₁-C₃)-alkoxy-(C₂-C₄)-alkynyl and phenyl-(C₂-C₄) -alkynyl;
R¹¹ is selected from the group consisting of hydrogen and (C₁-C₄)-alkyl;
X is selected from the group consisting of a chemical bond, CH₂, O, S, CHCH₃, NCH₃, OCH₂ and SCH₂, where in the two last-mentioned groups the carbon atom is attached to the aromatic moiety and the heteroatom O or S is attached to the partially hydrogenated moiety of the amine; and
n is 0 or 1.

15. Compounds of the general formula (I) 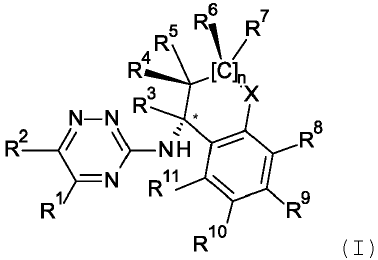 according to any of Claims 1 to 14, **characterized in that** the chiral carbon atom marked by (*) has an (R) configuration.

16. Compounds of the general formula (I) 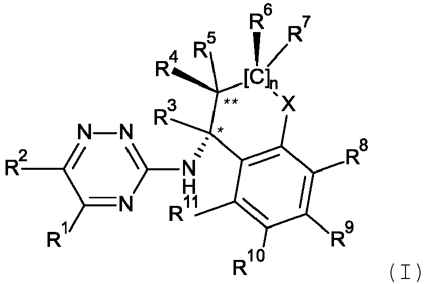 according to any of Claims 1 to 14, **characterized in that** the chiral carbon atom marked by (*) has an (R) configuration and the chiral carbon atom markd by (**) has an (S) configuration.

17. Process for preparing compounds of the general formula (I) or agrochemically acceptable salts thereof: 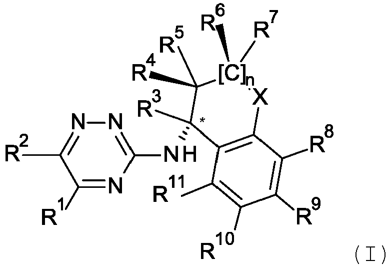 in which the R¹ to R¹¹ and X radicals and n are as defined in any of Claims 1 to 16, and where
- a compound of the general formula (II) 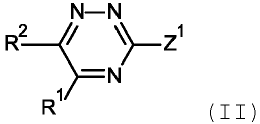 where R¹, R² are defined according to any of Claims 1 to 16 and Z¹ represents an exchangeable radical or a leaving group selected from the group consisting of fluorine, chlorine, bromine, iodine, (C₁-C₄)-alkylsulfanyl, (C₁-C₄)-alkylsulfinyl, (C₁-C₄)-alkylsulfonyl, unsubstituted phenyl-(C₁-C₄)-alkylsulfonyl or phenyl-(C₁-C₄)-alkylsulfonyl which is mono- or polysubstituted by fluorine, chlorine, bromine or (C₁-C₄)-alkyl or (C₁-C₄)-alkoxy and represents (C₁-C₄)-alkylphenylsulfonyl;
- is reacted with an amine of the general formula (III) or an acid addition salt of the amine of the general formula (III) 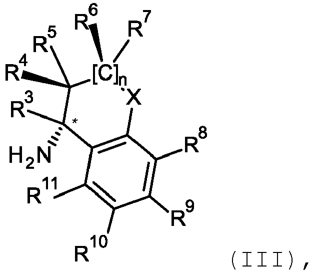 where the R³ to R¹¹ and X radicals and n are as defined in Claims 1 to 16.

18. Process for preparing compounds of the general formula (I): 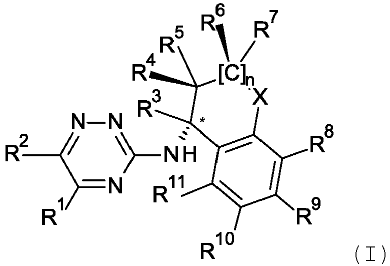 in which the R¹ to R¹¹ and X radicals and n are as defined in any of Claims 1 to 16, and where
- a compound of the general formula (II-a) 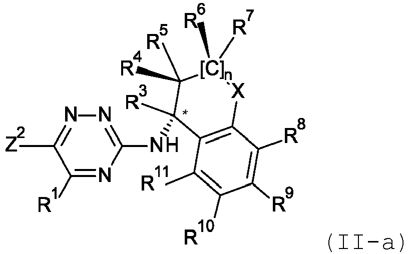 where Z² is selected from the group consisting of halogen, cyano, (C₁-C₆)-alkoxycarbonyl, (C₁-C₆)-alkylcarbonyl and (C₁-C₆)-alkylthio is prepared by the process according to Claim 17 and subsequently
- the Z² radical is converted by known processes into an R² radical, where R² is defined according to any of Claims 1 to 16.

19. Process for preparing compounds of the general formula (I) or agrochemically acceptable salts thereof: 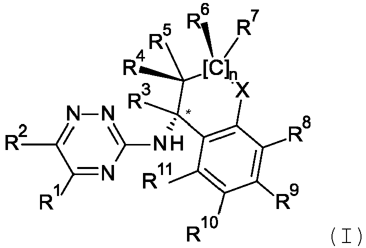 in which R¹ represents amino and the R² to R¹¹ and X radicals and n are as defined in any of Claims 1 to 16, and where
- a guanidine compound of the general formula (IV) or an acid addition salt thereof 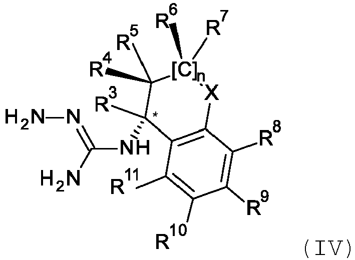
- is condensed with acylcyanides of the formula (V) 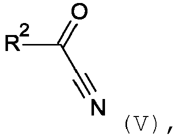 where the R² radical is as defined in Claims 1 to 16.

20. Herbicidal composition or plant growth-regulating composition, **characterized in that** it comprises one or more compounds of the general formula (I) or salts thereof according to any of Claims 1 to 16.

21. Method of controlling harmful plants or of regulating the growth of plants, **characterized in that** an effective amount of one or more compounds of the general formula (I) or salts thereof according to any of Claims 1 to 16 is applied to plants, plant parts, plant seeds or an area under cultivation.

22. Use of compounds of the general formula (I) or salts thereof according to any of Claims 1 to 16 as herbicides or as plant growth regulators.

23. Use according to Claim 23, **characterized in that** the compounds of the general formula (I) or salts thereof are used to control harmful plants or to regulate the growth of plants in crops of useful plants or ornamental plants.

24. Use according to Claim 22 or 23, **characterized in that** the crop plants are transgenic crop plants.

## Revendications

1. Composés de formule générale (I) 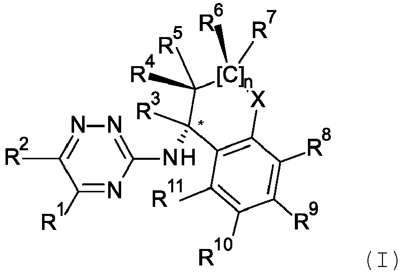 et leurs sels acceptables sur le plan agrochimique, dans laquelle
R¹ signifie hydrogène, amino, hydrazino, hydroxyamino, ((C₁-C₆)-alkyl)-amino, ((C₃-C₆)-cycloalkyl)-amino, di-((C₁-C₆)-alkyl)-amino, (C₁-C₆)-alkylcarbonylamino, bis-((C₁-C₆)-alkyl)-carbonylamino ;
R² est choisi dans le groupe constitué par hydrogène, halogène, hydroxy, nitro, amino, cyano, C(O)OH, C(O)NH₂ ; (C₁-C₆)-alkyle, (C₁-C₆)-halogénoalkyle, (C₁-C₆)-alkylcarbonyle, (C₁-C₆)-halogénoalkylcarbonyle, (C₁-C₆)-alkylcarbonyloxy, (C₁-C₆)-halogénoalkylcarbonyloxy, (C₁-C₆)-alkylcarbonyl-(C₁-C₄)-alkyle ; (C₁-C₆)-alcoxy, (C₁-C₆)-halogénoalcoxy, (C₁-C₆)-alcoxycarbonyle, (C₁-C₆)-halogénoalcoxycarbonyle, (C₁-C₆)-alcoxycarbonyl-(C₁-C₆)-alkyle, (C₁-C₆)-halogénoalcoxycarbonyl-(C₁-C₆)-alkyle, (C₁-C₆)-alcoxycarbonyl- (C₁-C₆)-halogénoalkyle, (C₁-C₆)-halogénoalcoxycarbonyl-(C₁-C₆)-halogénoalkyle ; (C₂-C₆)-alcényle, (C₂-C₆)-halogénoalcényle, (C₂-C₆)-alcénylcarbonyle, (C₂-C₆)-halogénoalcénylcarbonyle, (C₂-C₆)-alcényloxy, (C₂-C₆)-halogénoalcényloxy, (C₂-C₆)-alcényloxycarbonyle, (C₂-C₆)-halogénoalcényloxycarbonyle ; (C₂-C₆)-alcynyle, (C₂-C₆)-halogénoalcynyle, (C₂-C₆)-alcynylcarbonyle, (C₂-C₆)-halogénoalcynylcarbonyle, (C₂-C₆)-alcynyloxy, (C₂-C₆)-halogénoalcynyloxy, (C₂-C₆)-alcynyloxycarbonyle, (C₂-C₆)-halogénoalcynyloxycarbonyle ; tri-(C₁-C₆)-alkylsilyl-(C₂-C₆)-alcynyle, di-(C₁-C₆)-alkylsilyl-(C₂-C₆)-alcynyle, (C₁-C₆)-alkylsilyl-(C₂-C₆)-alcynyle ; phénylsilyl-(C₂-C₆)-alcynyle ; (C₆-C₁₄)-aryle, (C₆-C₁₄)-aryloxy, (C₆-C₁₄)-arylcarbonyle et (C₆-C₁₄)-aryloxycarbonyle, qui peuvent être substitués à chaque fois sur la partie aryle par halogène, (C₁-C₆)-alkyle et/ou (C₁-C₆)-halogénoalkyle ; (C₆-C₁₄)-aryl-(C₁-C₆)-alkyle, (C₆-C₁₄)-aryl-(C₁-C₆)-alcoxy, (C₆-C₁₄)-aryl-(C₁-C₆)-alkyl-carbonyle, (C₆-C₁₄)-aryl-(C₁-C₆)-alkyl-carbonyloxy, (C₆-C₁₄)-aryl-(C₁-C₆)-alcoxycarbonyle, (C₆-C₁₄)-aryl-(C₁-C₆)-alcoxycarbonyloxy ; Aminocarbonyl-(C₁-C₆)-alkyle, di-(C₁-C₆)-alkylaminocarbonyl-(C₁-C₆)-alkyle ; N-((C₁-C₆)-halogénoalcanoyl)-amino-carbonyle, ((C₆-C₁₄)-aryl)amino-carbonyle, di-((C₆-C₁₄)-aryl)-amino-carbonyle, ((C₁-C₆)-alkyl)amino-carbonyle, di-((C₁-C₆)-alkyl)-amino-carbonyle, N-((C₁-C₆)-alkylsulfonyl)-amino-carbonyle, N-((C₁-C₆)-halogénoalkylsulfonyl)-amino-carbonyle, N-((C₆-C₁₄)-arylsulfonyl)-amino-carbonyle ; (C₁-C₆)-alcoxy-(C₁-C₆)-alkyle, (C₁-C₆)-alcoxy-(C₁-C₆)-alcoxy, (C₁-C₆)-alcoxycarbonyl-(C₁-C₆)-alcoxy ; (C₃-C₈)-cycloalkyle, qui peut éventuellement être substitué au niveau du radical cycloalkyle par (C₁-C₆)-alkyle et/ou halogène ; (C₃-C₈)-cycloalcoxy, (C₃-C₈)-cycloalkyl-(C₁-C₆)-alkyle, (C₃-C₈)-cycloalkyl-(C₁-C₆)-halogénoalkyle, (C₃-C₈)-cycloalkyl-(C₁-C₆)-alcoxy, (C₃-C₈)-cycloalkyl-(C₁-C₆)-halogénoalcoxy, (C₃-C₈)-cycloalkylcarbonyle, (C₃-C₈)-cycloalcoxycarbonyle, (C₃-C₈)-cycloalkyl-(C₁-C₆)-alkylcarbonyle, (C₃-C₈)-cycloalkyl-(C₁-C₆)-halogénoalkylcarbonyle, (C₃-C₈)-cycloalkyl-(C₁-C₆)-alcoxycarbonyle, (C₃-C₈)-cycloalkyl-(C₁-C₆)-halogénoalcoxycarbonyle, (C₃-C₈)-cycloalkylcarbonyloxy, (C₃-C₈)-cycloalcoxycarbonyloxy, (C₃-C₈)-cycloalkyl-(C₁-C₆)-alkylcarbonyloxy, (C₃-C₈)-cycloalkyl-(C₁-C₆)-halogénoalkylcarbonyloxy, (C₃-C₈)-cycloalkyl-(C₁-C₆)-alcoxycarbonyloxy, (C₃-C₈)-cycloalkyl-(C₁-C₆)-halogénoalcoxycarbonyloxy ; (C₃-C₈)-cycloalcényle, (C₃-C₈)-cycloalcényloxy, (C₃-C₈)-cycloalcényl-(C₁-C₆)-alkyle, (C₃-C₈)-cycloalcényl-(C₁-C₆)-halogénoalkyle, (C₃-C₈)-cycloalcényl-(C₁-C₆)-alcoxy, (C₃-C₈)-cycloalcényl-(C₁-C₆)-halogénoalcoxy, (C₃-C₈)-cycloalcénylcarbonyle, (C₃-C₈)-cycloalcényloxycarbonyle, (C₃-C₈)-cycloalcényl-(C₁-C₆)-alkylcarbonyle, (C₃-C₈)-cycloalcényl-(C₁-C₆)-halogénoalkylcarbonyle, (C₃-C₈)-cycloalcényl-(C₁-C₆)-alcoxycarbonyle, (C₃-C₈)-cycloalcényl-(C₁-C₆)-halogénoalcoxycarbonyle, (C₃-C₈)-cycloalcénylcarbonyloxy, (C₃-C₈)-cycloalcényloxycarbonyloxy, (C₃-C₈)-cycloalcényl-(C₁-C₆)-alkylcarbonyloxy, (C₃-C₈)-cycloalcényl-(C₁-C₆)-halogénoalkylcarbonyloxy, (C₃-C₈)-cycloalcényl-(C₁-C₆)-alcoxycarbonyloxy, (C₃-C₈)-cycloalcényl-(C₁-C₆)-halogénoalcoxycarbonyloxy ; hydroxy-(C₁-C₆)-alkyle, hydroxy-(C₁-C₆)-alcoxy, cyano-(C₁-C₆)-alcoxy, cyano-(C₁-C₆)-alkyle ; et (C₁-C₆)-alkylsulfonyle, (C₁-C₆)-alkylthio, (C₁-C₆)-alkylsulfinyle, (C₁-C₆)-halogénoalkylsulfonyle, (C₁-C₆)-halogénoalkylthio, (C₁-C₆)-halogénoalkylsulfinyle, (C₁-C₆)-alkylsulfonyl-(C₁-C₆)-alkyle, (C₁-C₆)-alkylthio-(C₁-C₆)-alkyle, (C₁-C₆)-alkylsulfinyl-(C₁-C₆)-alkyle, (C₁-C₆)-halogénoalkylsulfonyl-(C₁-C₆)-alkyle, (C₁-C₆)-halogénoalkylthio-(C₁-C₆)-alkyle, (C₁-C₆)-halogénoalkylsulfinyl-(C₁-C₆)-alkyle, (C₁-C₆)-alkylsulfonyl-(C₁-C₆)-halogénoalkyle, (C₁-C₆)-alkylthio-(C₁-C₆)-halogénoalkyle, (C₁-C₆)-alkylsulfinyl-(C₁-C₆)-halogénoalkyle, (C₁-C₆)-halogénoalkylsulfonyl-(C₁-C₆)-halogénoalkyle, (C₁-C₆)-halogénoalkylthio-(C₁-C₆)-halogénoalkyle, (C₁-C₆)-halogénoalkylsulfinyl-(C₁-C₆)-halogénoalkyle, (C₁-C₆)-alkylsulfonyloxy, (C₁-C₆)-halogénoalkylsulfonyloxy, (C₁-C₆)-alkylthiocarbonyle, (C₁-C₆)-halogénoalkylthiocarbonyle, (C₁-C₆)-alkylthiocarbonyloxy, (C₁-C₆)-halogénoalkylthiocarbonyloxy, (C₁-C₆)-alkylthio- (C₁-C₆)-alkyle, (C₁-C₆)alkylthio-(C₁-C₆)-alcoxy, (C₁-C₆)-alkylthio-(C₁-C₆)-alkylcarbonyle, (C₁-C₆)-alkylthio(C₁-C₆)-alkylcarbonyloxy ; (C₄-C₁₄)-arylsulfonyle, (C₆-C₁₄)-arylthio, (C₆-C₁₄)-arylsulfinyle, (C₃-C₈)-cycloalkylthio, (C₃-C₈)-alcénylthio, (C₃-C₈)-cycloalcénylthio et (C₃-C₆)-alcynylthio, ou
R¹ et R² peuvent être liés l'un à l'autre par l'intermédiaire d'une liaison, de sorte qu'un carbocycle ou hétérocycle partiellement hydrogéné à 5 à 7 chaînons comportant au moins un hétéroatome choisi parmi N, O, S et p est formé, qui est éventuellement substitué par un ou plusieurs substituants choisis dans le groupe constitué par hydroxy, =O, =N-O-H, =N-O-(C₁-C₆)-alkyle, =N-O-benzyle, =N-O-phényle, phényle, phényle substitué par un ou plusieurs atomes d'halogènes identiques ou différents, (C₁-C₆)-alkyle, (C₃-C₆)-cycloalkyle et (C₁-C₆)-halogénoalkyle ;
R³ est choisi dans le groupe constitué par hydrogène, (C₁-C₆)-alkyle, (C₁-C₆)-alcoxy, (C₁-C₆)-alcoxy-(C₁-C₆)-alkyle, (C₁-C₆)-alcoxycarbonyle et aminocarbonyle ;
R⁴ et R⁵ sont choisis, à chaque fois indépendamment l'un de l'autre, dans le groupe constitué par hydrogène, (C₁-C₆)-alkyle, (C₁-C₆)-halogénoalkyle, hydroxy, (C₁-C₆)-alcoxy et (C₁-C₆)-halogénoalcoxy ; ou
R⁴ et R⁵, conjointement avec l'atome de carbone auquel ils sont liés, forment un cycle saturé à trois à sept chaînons, qui peut contenir un ou plusieurs hétéroatomes choisis parmi oxygène, soufre et azote ;
R⁶ et R⁷ sont choisis, à chaque fois indépendamment l'un de l'autre, dans le groupe constitué par hydrogène, (C₁-C₆)-alkyle, (C₁-C₆)-halogénoalkyle, (C₁-C₆)-alcoxy, (C₁-C₆)-halogénoalcoxy, (C₆-C₁₄)-aryle, (C₆-C₁₄)-aryloxy, (C₆-C₁₄)-arylcarbonyle et (C₆-C₁₄)-aryloxycarbonyle ; ou les radicaux R⁶ et R⁷ forment ensemble un groupe (C₁-C₇)-alkylène, qui peut contenir un ou plusieurs atomes d'oxygène et/ou de soufre, le groupe (C₁-C₇)-alkylène pouvant être substitué une ou plusieurs fois par halogène et les substituants halogènes respectifs pouvant être identiques ou différents ;
n est 0, 1 ou 2 ;
R⁸, R⁹, R¹⁰ et R¹¹ sont choisis, à chaque fois indépendamment les uns des autres, dans le groupe constitué par hydrogène, halogène, cyano, C(O)OH, C(O)NH₂, (C₁-C₆)-alkyle, (C₁-C₆)-alkylcarbonyle, (C₁-C₆)-alkyloxycarbonyle, (C₁-C₆)-alkylaminocarbonyle, (C₁-C₆)-di-alkylaminocarbonyle, (C₁-C₆)-halogénoalkyle, (C₁-C₆)-alcoxy, (C₁-C₆)-halogénoalcoxy, (C₂-C₆)-alcényle, (C₂-C₆)-alcynyle, (C₂-C₆)-halogénoalcynyle, (C₂-C₆)-alcynylcarbonyle, (C₂-C₆)-halogénoalcynylcarbonyle, (C₂-C₆)-alcynyloxy, (C₂-C₆)-halogénoalcynyloxy, (C₂-C₆)-alcynyloxycarbonyle, (C₂-C₆)-halogénoalcynyloxycarbonyle et nitro, les radicaux R⁹ et R¹⁰ pouvant être reliés par un groupe -O-CH₂-O- pour former un cycle ;
X représente une liaison, CH₂, O, S, carbonyle, NH, CR¹²R¹³, NR¹⁴, CH₂O ou CH₂S, dans lesquels dans les deux groupes mentionnés en dernier, l'atome de carbone est lié à la partie aromatique et l'hétéroatome O ou S est lié à la partie partiellement hydrogénée de l'amine ;
R¹² et R¹³ sont choisis, à chaque fois indépendamment l'un de l'autre, dans le groupe constitué par hydrogène, (C₁-C₆)-alkyle et (C₁-C₆)-halogénoalkyle ; et
R¹⁴ est choisi dans le groupe constitué par hydrogène, (C₁-C₆)-alkyle et (C₁-C₆)-halogénoalkyle.

2. Composés de formule générale (I) selon la revendication 1, **caractérisés en ce que** R¹ est choisi dans le groupe constitué par hydrogène, amino, ((C₁-C₆)-alkyl)amino, ((C₃-C₆)-cycloalkyl)amino, et di-((C₁-C₆)-alkyl)amino.

3. Composés de formule générale (I) selon la revendication 1 ou 2, **caractérisés en ce que** R² est choisi dans le groupe constitué par hydrogène, halogène, hydroxy, nitro, amino, cyano, C(O)OH, C(O)NH₂ ;
(C₁-C₆)-alkyle, (C₁-C₆)-halogénoalkyle ;
(C₁-C₆)-alkylcarbonyle, (C₁-C₆)-halogénoalkylcarbonyle, (C₁-C₆)-alcoxycarbonyle, (C₁-C₆)-halogénoalcoxycarbonyle ;
(C₂-C₆)-alcényle, (C₂-C₆)-halogénoalcényle ;
(C₂-C₆)-alcynyle, (C₂-C₆)-halogénoalcynyle ;
(C₆-C₁₄)-aryle, qui peut être substitué sur la partie aryle par halogène, (C₁-C₆) -alkyle et/ou (C₁-C₆)-halogénoalkyle ;
(C₁-C₆)-alcoxy-(C₁-C₆)-alkyle, hydroxy-(C₁-C₆)-alkyle ;
((C₁-C₆)-alkyl) amino-carbonyle, di-((C₁-C₆)-alkyl)aminocarbonyle, N-((C₁-C₆)-alkylsulfonyl)amino-carbonyle, N-((C₁-C₆)-halogénoalkylsulfonyl)amino-carbonyle, (C₃-C₈)-cycloalkylcarbonyle, (C₃-C₈)-cycloalcoxycarbonyle ; (C₁-C₆)-alkylsulfonyle, (C₁-C₆)-alkylthio, (C₁-C₆)-alkylsulfinyle, (C₁-C₆)-halogénoalkylsulfonyle, (C₁-C₆)-halogénoalkylsulfinyle, (C₄-C₁₄)-arylsulfonyle et (C₆-C₁₄)-arylsulfinyle.

4. Composés de formule générale (I) selon l'une quelconque des revendications 1 à 3, **caractérisés en ce que** R³ est hydrogène.

5. Composés de formule générale (I) selon l'une quelconque des revendications 1 à 4, **caractérisés en ce que** R⁴ et R⁵ sont choisis, à chaque fois indépendamment l'un de l'autre, dans le groupe constitué par hydrogène, hydroxy, (C₁-C₆)-alkyle, (C₁-C₆)-alkylphényle et (C₁-C₆)-alcoxy.

6. Composés de formule générale (I) selon l'une quelconque des revendications 1 à 5, **caractérisés en ce que** R⁴ et R⁵, conjointement avec l'atome de carbone auquel ils sont liés, forment un cycle saturé à trois à sept chaînons qui peut contenir un atome d'oxygène.

7. Composés de formule générale (I) selon l'une quelconque des revendications 1 à 6, **caractérisés en ce que** R⁶ et R⁷, indépendamment l'un de l'autre, sont choisis dans le groupe constitué par hydrogène, (C₁-C₆)-alkyle et (C₆-C₁₄)-aryle.

8. Composés de formule générale (I) selon l'une quelconque des revendications 1 à 7, **caractérisés en ce que** R⁸ est choisi dans le groupe constitué par hydrogène, halogène, (C₁-C₆)-alkyle, (C₁-C₆)-alcoxy et (C₆-C₁₄)-aryle.

9. Composés de formule générale (I) selon l'une quelconque des revendications 1 à 8, **caractérisés en ce que** R⁹ est choisi dans le groupe constitué par hydrogène, halogène, (C₁-C₆)-alkyle et (C₁-C₆)-alcoxy.

10. Composés de formule générale (I) selon l'une quelconque des revendications 1 à 9, **caractérisés en ce que** R¹⁰ est choisi dans le groupe constitué par hydrogène, halogène, cyano, aminocarbonyle, hydroxycarbonyle, (C₁-C₆)-alkyle, (C₁-C₆)-halogénoalkyle, (C₁-C₆)-alcoxy, (C₂-C₆)-alcényle, (C₂-C₆)-alcynyle, (C₁-C₆)-alkyl-(C₂-C₆)-alcynyle, hydroxy-(C₁-C₆)-alkyl-(C₂-C₆)-alcynyle, (C₁-C₆)-alcoxy-(C₂-C₆)-alcynyle et aryl-(C₂-C₆)-alcynyle.

11. Composés de formule générale (I) selon l'une quelconque des revendications 1 à 10, **caractérisés en ce que** R¹¹ est choisi dans le groupe constitué par hydrogène et (C₁-C₆)-alkyle.

12. Composés de formule générale (I) selon l'une quelconque des revendications 1 à 11, **caractérisés en ce que** X est choisi dans le groupe constitué par une liaison chimique, CH₂, O, S, carbonyle, NH, CH(C₁-C₆)alkyle, N(C₁-C₆)alkyle, OCH₂, et SCH₂, dans lesquels dans les deux groupes mentionnés en dernier, l'atome de carbone est lié à la partie aromatique et l'hétéroatome O ou S est lié à la partie partiellement hydrogénée de l'amine.

13. Composés de formule générale (I) selon l'une quelconque des revendications 1 à 12, **caractérisés en ce que** l'indice n est 0 ou 1.

14. Composés de formule générale (I) selon l'une quelconque des revendications 1 à 13, **caractérisés en ce que**
R¹ est choisi dans le groupe constitué par hydrogène, amino, ((C₁-C₃)-alkyl)amino, ((C₃-C₆)-cycloalkyl)amino et di((C₁-C₃)-alkyl)amino ;
R² est choisi dans le groupe constitué par Cl, Br, cyano, C(O)OH, C(O)NH₂ ; (C₁-C₆)-alkyle, (C₁-C₆)-halogénoalkyle, (C₁-C₆)-alkylcarbonyle, (C₁-C₆)-halogénoalkylcarbonyle ; (C₁-C₆)-alcoxycarbonyle, (C₁-C₆)-halogénoalcoxycarbonyle ; (C₂-C₆)-alcényle, (C₂-C₆)-halogénoalcényle ; (C₂-C₆)-alcynyle, (C₂-C₆)-halogénoalcynyle ; phényle, qui peut être substitué par F, Cl, Br, méthyle et/ou trifluorométhyle ; (C₁-C₆)-alcoxy-(C₁-C₆)-alkyle et hydroxy-(C₁-C₆)-alkyle ; ((C₁-C₂)-alkyl-amino-carbonyle, di-((C₁-C₂)-alkyl)amino-carbonyle, N-((C₁-C₂)-alkylsulfonyl)amino-carbonyle, N-((C₁-C₂)-halogénoalkylsulfonyl)amino-carbonyle ; (C₃-C₆)-cycloalkylcarbonyle, (C₃-C₆)-cycloalcoxycarbonyle ; (C₁-C₃)-alkylsulfonyle, (C₁-C₃)-alkylsulfinyle, (C₁-C₃)-halogénoalkylsulfonyle, (C₁-C₆)-halogénoalkylsulfinyle, (C₄-C₁₄)-arylsulfonyle et (C₆-C₁₄)-arylsulfinyle ;
R³ est hydrogène ;
R⁴ et R⁵ sont choisis, à chaque fois indépendamment l'un de l'autre, dans le groupe constitué par hydrogène, (C₁-C₆)-alkyle et (C₁-C₆)-alcoxy ; ou
R⁴ et R⁵, conjointement avec le carbone auquel ils sont liés, forment un cycle saturé à trois à six chaînons qui peut contenir un atome d'oxygène ;
R⁶ et R⁷ sont choisis, indépendamment l'un de l'autre, dans le groupe constitué par hydrogène, méthyle et phényle ;
R⁸ est choisi dans le groupe constitué par hydrogène, halogène, (C₁-C₃)-alkyle et (C₆-C₈)-aryle ;
R⁹ est choisi dans le groupe constitué par hydrogène, chlore, fluor et (C₁-C₃)-alcoxy ;
R¹⁰ est choisi dans le groupe constitué par hydrogène, fluor, chlore, brome, iode, cyano, méthoxy (OCH₃), (C₁-C₃)-alkyle, (C₁-C₃)-halogénoalkyle, (C₁-C₃)-alcoxy, (C₂-C₄)-alcényle, (C₂-C₄)-alcynyle, (C₁-C₃)-alkyl-(C₂-C₄)-alcynyle, hydroxy-(C₁-C₃)-alkyl-(C₂-C₄)-alcynyle, (C₁-C₃)-alcoxy-(C₂-C₄)-alcynyle et phényl-(C₂-C₄)-alcynyle ;
R¹¹ est choisi dans le groupe constitué par hydrogène et (C₁-C₄)-alkyle ;
X est choisi dans le groupe constitué par une liaison chimique, CH₂, O, S, CHCH₃, NCH₃, OCH₂, et SCH₂, dans lesquels dans les deux groupes mentionnés en dernier, l'atome de carbone est lié à la partie aromatique et l'hétéroatome O ou S est lié à la partie partiellement hydrogénée de l'amine; et
n est 0 ou 1.

15. Composés de formule générale (I) 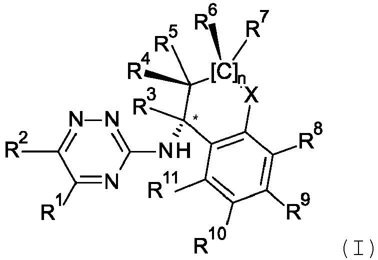 selon l'une quelconque des revendications 1 à 14, **caractérisés en ce que** l'atome de carbone chiral comportant la marque (*) présente une configuration (R).

16. Composés de formule générale (I) 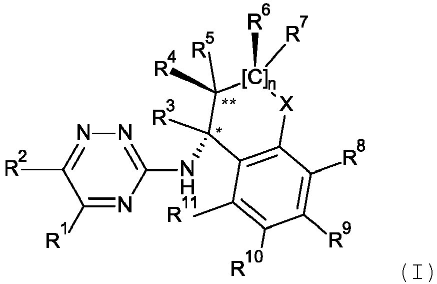 selon l'une quelconque des revendications 1 à 14, **caractérisés en ce que** l'atome de carbone chiral comportant la marque (*) présente une configuration (R) et l'atome de carbone chiral comportant la marque (**) présente une configuration (S).

17. Procédé pour la préparation de composés de formule générale (I) ou de leurs sels acceptables sur le plan agrochimique : 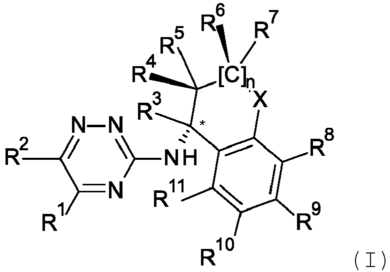 dans laquelle les radicaux R¹ à R¹¹ et X ainsi que n sont définis comme dans l'une quelconque des revendications 1 à 16, et
- un composé de formule générale (II) 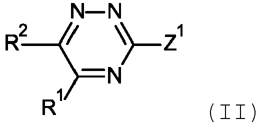 R¹, R² étant définis selon l'une quelconque des revendications 1 à 16 et Z¹ représentant un radical échangeable ou un groupe partant, choisi dans le groupe constitué par fluor, chlore, brome, iode, un (C₁-C₄)-alkylsulfanyle, un (C₁-C₄)-alkylsulfinyle, un (C₁-C₄)-alkylsulfonyle, un phényl-(C₁-C₄)-alkylsulfonyle non substitué ou substitué une fois par (C₁-C₄)-alcoxy ou une ou plusieurs fois par fluor, chlore, brome ou (C₁-C₄)-alkyle, et un (C₁-C₄)-alkylphényl-sulfonyle ;
- étant transformé avec une amine de formule générale (III) ou avec un sel d'addition d'acide de l'amine de formule générale (III) 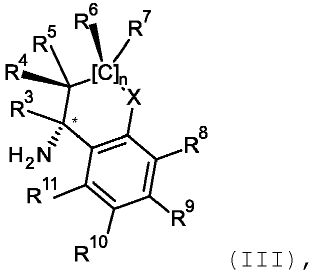 les radicaux R³ à R¹¹ et X ainsi que n étant définis selon les revendications 1 à 16.

18. Procédé pour la préparation de composés de formule générale (I) : 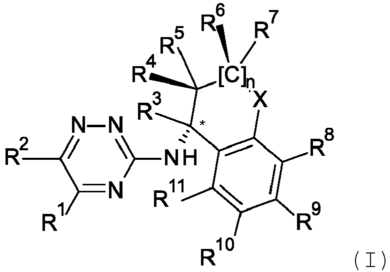 dans laquelle les radicaux R¹ à R¹¹ et X ainsi que n sont définis comme dans l'une quelconque des revendications 1 à 16, et
- un composé de formule générale (II-a), 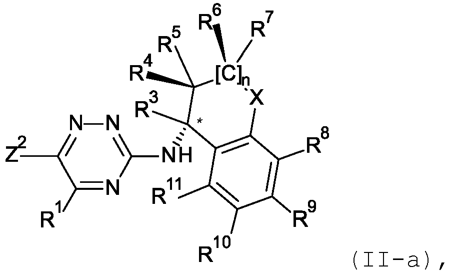 Z² étant choisi dans le groupe constitué par halogène, cyano, (C₁-C₆)-alcoxycarbonyle, (C₁-C₆) -alkylcarbonyle et (C₁-C₆)-alkylthio, étant préparé selon le procédé selon la revendication 17, et ensuite
- le radical Z² étant transformé en un radical R² selon un procédé connu, R² étant défini selon l'une quelconque des revendications 1 à 16.

19. Procédé pour la préparation de composés de formule générale (I) ou de leurs sels acceptables sur le plan agrochimique : 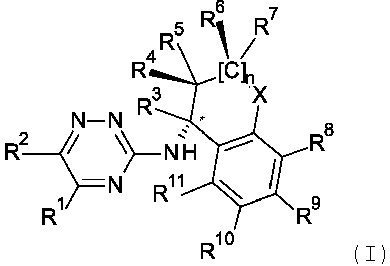 dans laquelle R¹ signifie amino et les radicaux R² à R¹¹ et X ainsi que n sont définis comme dans l'une quelconque des revendications 1 à 16, et
- un composé de type guanidine de formule générale (IV) ou un sel d'addition d'acide correspondant 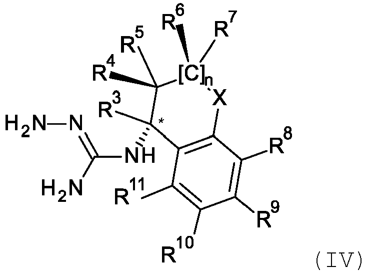
- étant condensé avec des cyanures d'acyle de formule (V) 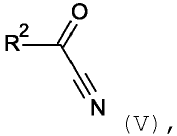 le radical R² étant défini comme dans les revendications 1 à 16.

20. Agent herbicide ou agent de régulation de la croissance végétale, **caractérisé en ce qu'**il contient un ou plusieurs composés de formule générale (I) ou leurs sels selon l'une quelconque des revendications 1 à 16.

21. Procédé pour la lutte contre des végétaux nuisibles ou pour la régulation de la croissance de végétaux, **caractérisé en ce que** l'on applique une quantité efficace d'un ou plusieurs composés de formule générale (I) ou leurs sels selon l'une quelconque des revendications 1 à 16 sur des végétaux, des parties de végétaux, des semences de végétaux ou sur une surface cultivée.

22. Utilisation de composés de formule générale (I) ou de leurs sels selon l'une quelconque des revendications 1 à 16 en tant qu'herbicides ou en tant que régulateurs de la croissance végétale.

23. Utilisation selon la revendication 22, **caractérisée en ce que** les composés de formule générale (I) ou leurs sels sont utilisés pour la lutte contre des végétaux nuisibles ou pour la régulation de la croissance de végétaux dans des cultures de végétaux utiles ou de plantes d'ornement.

24. Utilisation selon la revendication 22 ou 23, **caractérisée en ce que** les végétaux de culture sont des végétaux de culture transgéniques.
